# EUROPEAN PATENT APPLICATION

(11) **EP 3 492 103 A1**
(43) Date of publication of application: **05.06.2019**
(21) Application number: 18020142.8
(22) Date of filing: 11.04.2018
(51) Int. Cl.: A61K 41/00

(54) **MAGNETOSOMES FOR USE IN AN ACOUSTIC WAVE MEDICAL TREATMENT**

(30) Priority: 30.11.2017 EP 17020556
(71) Applicant: Nanobacterie, 75116 Paris (FR)
(72) Inventor: ALPHANDERY, Edouard, 75116 Paris (FR)

(57) **Abstract**

This invention relates to magnetosomes for use in an acoustic wave medical treatment of a body part of an individual, wherein the magnetosomes are administered to a body part of an individual and the body part is exposed to at least one acoustic wave.

## Description

### Field of the invention

The invention relates to the use of magnetosomes in an acoustic wave medical treatment.

### Technical background

For the destruction of tumors such as prostate tumors, high intensity ultrasound is currently used. This technique suffers from a number of drawbacks such as a large number of ultrasound spots necessary to heat the whole tumors and the use of high powers that are potentially dangerous and difficult to obtain cheaply. For this reason, it appears necessary to develop acoustic sensitizers, such as the magnetosomes presented in this invention, which enable the use of ultrasound of lower intensity than that of HIFU.

### Description of the invention

In this invention, an acoustic wave can be defined as: i), a mechanical wave, which preferentially induces a mechanical disturbance of a medium or a body part through which it travels such as compression and/or expansion of the medium, or ii), a wave that induces or is associated with the movement or vibration of a substance, atom, ion, magnetosome with a non-zero mass or non-zero weight. The acoustic wave is usually not an electromagnetic wave. In some cases, it can however produce or generate an electromagnetic wave, for example if moving/vibrating substances associated to the acoustic wave possess a non-zero charge. The word *"acoustic wave"* can designate acoustic wave energy, acoustic wave power, acoustic wave intensity, or acoustic wave frequency. In some cases, acoustic wave intensity can have a similar meaning as acoustic wave power or acoustic wave energy. In other cases, acoustic wave power can have a similar meaning as acoustic wave energy. The acoustic wave can be absorbed by, reflected by, or transmitted through magnetosomes or a body part. The acoustic wave can have a frequency, energy, power, or intensity, which can be designated as the acoustic wave frequency, energy, power, or intensity, respectively. The acoustic wave can designate an assembly of more than 1, 10, 10², 10³, 10⁵, 10¹⁰, or 10²⁰ acoustic wave(s). The acoustic wave energy can represent the acoustic wave power multiplied by the time of application of the acoustic wave. It can be expressed in a power unit such as Watt multiplied by a time unit such as second. The energy density of the acoustic wave can represent the energy of the acoustic wave per unit length such as cm, per unit surface area such as cm², or per unit volume such as cm³. The acoustic wave power can be proportional to the acoustic wave energy per unit time. It can be expressed in a power unit such as Watt. The acoustic wave power density can represent the acoustic wave power per unit length such as cm, per unit surface such as cm², or per unit volume such as cm³. The acoustic wave intensity can be proportional to the acoustic wave power per unit surface area such as cm². It can be expressed in a power unit, such as Watt, divided by a surface area unit such as cm². In some cases, the unit length, unit surface area, and unit volume can represent the length, surface area, and volume of the nanoparticle(s) or magnetosome(s), respectively.

In this invention, an acoustic wave can be defined as an infrasound, a sound, an ultrasound, or a hypersound. An infrasound can preferentially be defined as an acoustic wave of low frequency, preferentially of frequency lower than 2, 20, or 200 Hz. A sound can preferentially be defined as a sound of frequency larger than 2, 20, or 200 Hz and/or of frequency lower than 2, 20, or 200 kHz. An ultrasound can preferentially be defined as a sound of frequency larger than 2, 20, or 200 kHz and/or of frequency lower than 0.1, 1, or 10 GHz. A hypersound can preferentially be defined as a sound of frequency larger than 10⁻³, 10⁻², 10⁻¹, 1, or 10 GHz.

This invention relates to magnetosomes for use in an acoustic wave medical treatment of a body part of an individual, wherein the magnetosomes are administered to a body part of an individual and the body part is exposed to at least one acoustic wave.

In an embodiment of the invention, a compound is bound or attached to the magnetosomes, preferentially before the application of the acoustic wave on the magnetosomes.

In an embodiment of the invention, the acoustic wave is a wave, which is associated with, or linked with, or which induces, or produces, or results in, or is responsible for, or creates the movement, or vibration, or oscillation of a substance, where the substance has preferentially: i), a mass that is larger than 10⁹, 10⁷, 10⁵, 10³, 10², 10, 1, 10⁻², 10⁻⁵, 10⁻⁷, 10⁻⁹ , 10⁻²⁰, or 10⁻⁵⁰ grams or grams per cm³ of body part, or ii), a positive or negative charge, or iii), a size that is larger than 10⁻³, 10⁻¹, 1, 10, 10², 10³, 10⁵, or 10⁷ Å.

In another embodiment of the invention, the acoustic wave is a wave, which is associated with, or linked with, or which induces, or produces, or results in, or is responsible for, or create the movement, or vibration, or oscillation of a substance, where the substance has preferentially: i), a mass that is lower than 10⁹, 10⁷, 10⁵, 10³, 10², 10, 1, 10⁻², 10⁻⁵, 10⁻⁷, 10⁻⁹, 10⁻²⁰, or 10⁻⁵⁰ grams or grams per cm³ of body part, ii), a neutral charge, or iii), a size that is lower than 10⁻³, 10⁻¹, 1, 10, 10², 10³, 10⁵, or 10⁷ Å.

In one embodiment of the invention, the movement, or vibration, or oscillation of the substance is periodic or is repeated periodically. In this case, the movement, or vibration, or oscillation of the substance can preferentially be repeated more than 2, 5, 10, 10³, 10⁶, or 10⁹ times, where this repetition preferentially means that at least one of the properties associated with the movement, vibration, or oscillation of the substance can be repeated, where this property can be the speed or speed variation with time or space of the substance, the displacement or displacement variation with time or space of the substance, the acceleration or acceleration variation with time or space of the substance.

In one embodiment of the invention, the substance is the magnetosome.

In one embodiment of the invention, the acoustic wave is a wave, which is associated with, or linked with, or which induces, or produces, or results in, or is responsible for, or creates the movement, or vibration, or oscillation of the compound, preferentially after the dissociation of the compound from the magnetosome.

In another embodiment of the invention, another radiation than an acoustic wave, *i.e.* an electromagnetic radiation, a magnetic field, an alternating magnetic field, a laser, an ionizing radiation, X-rays, or gamma rays, can be applied on the nanoparticles to induce the dissociation of the compound from the nanoparticles. In this case, the acoustic wave is associated with, or linked with, or induces, or produces, or results in, or is responsible for, or creates the movement, or vibration, or oscillation of the compound, preferentially after the dissociation of the compound from the magnetosome.

The invention also relates to magnetosomes for use in an acoustic wave medical treatment of a body part of an individual, wherein the magnetosomes are administered to a body part of an individual and the acoustic wave is applied to the magnetosomes and/or body part.

The invention also relates to a method for treating a body part of an individual, comprising administering an effective amount of magnetosomes to the body part and applying an effective acoustic wave to the body part.

In this invention, the acoustic wave can be the acoustic wave generated or produced by an equipment generating or producing the acoustic wave, preferentially before, during, or after the acoustic wave has reached or covered or targeted the body part or magnetosomes. The equipment is different from the magnetosomes and is preferentially fabricated by a human.

In this invention, the acoustic wave can in some cases be the acoustic wave generated by the magnetosomes. In this case, the acoustic wave may be due to a change in the organization of the magnetosomes, preferentially between before and after magnetosome administration in the body part or individual. For example, magnetosomes may be organized in chains before their administration to the individual and then be progressively degraded, for example by lysosomes, following their administration to the individual, leading to a variation, preferentially an increase, in the distances between the magnetosomes and therefore to a change in the interactions between the magnetosomes, which could produce an acoustic wave. In some cases, the intensity, strength, or power, of the acoustic wave can't be detected, because it is too small or undetectable with the acoustic wave detectors that are available. In this case, the existence of the acoustic wave can be revealed by the changes in movement, oscillation, size, organization, composition, or charge, of the substances that produce the acoustic wave, such as the magnetosomes.

In some cases, the acoustic wave can be due to the movement of more than 2, 5, 10, 10⁵, 10¹⁰, or 10²⁰ magnetosomes or to a change with time, preferentially within less than 10¹⁰, 10⁵, 10³, 10, 5, 2, 1, 10⁻², or 10⁻⁵ minutes, of the magnetosome concentration, preferentially by a factor of more than 1.1, 2, 5, 10, 10⁵, 10¹⁰, or 10²⁰, where these changes in magnetosome movement or concentration preferentially occur in the body part.

In one embodiment of the invention, the acoustic wave is or is associated with or is similar to a gravitational wave or a wave that is due to the movement of more than 1, 2, 5, 10, 10⁵, or 10¹⁰ substances with a non-zero mass.

In this invention, the body part or magnetosomes exposed to the acoustic wave can mean that the body part or magnetosomes receive or absorb the energy or power of the acoustic wave, or receive or absorb at least 10⁻⁹, 10⁻⁷, 10⁻⁵, 10⁻³, 10⁻¹, 1, 5, 10, 25, 50, 75, or 80% of the energy or power of the acoustic wave.

In one embodiment of this invention, the body part is divided between a portion of the body part comprising the magnetosomes and a portion of the body part not comprising the magnetosomes. In some cases, the portion of the body part comprising the magnetosomes can absorb more than 10⁻⁹, 10⁻⁷, 10⁻⁵, 10⁻³, 10⁻¹ , 1, 5, 10, 25, 50, 75, or 80% of the energy of the acoustic wave and the portion of the body part not comprising the magnetosomes can preferentially absorb less than 10⁻⁹, 10⁻⁷, 10⁻⁵, 10⁻³, 10⁻¹ , 1, 5, 10, 25, 50, 75, or 80% of the energy of the acoustic wave. In some other cases, the portion of the body part comprising the magnetosomes can absorb less than 10⁻⁹, 10⁻⁷, 10⁻⁵, 10⁻³, 10⁻¹, 1, 5, 10, 25, 50, 75, or 80% of the energy of the acoustic wave and the portion of the body part not comprising the magnetosomes can preferentially absorb more than 10⁻⁹, 10⁻⁷, 10⁻⁵, 10⁻³, 10⁻¹ , 1, 5, 10, 25, 50, 75, or 80% of the energy of the acoustic wave. The percentages mentioned in this embodiment and in the previous embodiment can represent the energy of the acoustic wave applied on the magnetosomes divided by the energy of the acoustic wave applied on the body part or the energy of the acoustic wave applied on the magnetosomes or body part divided by the energy of the acoustic wave produced by the equipment generating the acoustic wave.

In some cases, the body part can designate the portion of the body part or the portion of the body part comprising the magnetosome or magnetosome region.

In some other cases, the body part can designate the portion of the body part or the portion of the body part not comprising the magnetosome or magnetosome region.

In still some other cases, the body part can designate both the portion of the body part comprising the magnetosome or magnetosome region and the portion of the body part not comprising the magnetosome or magnetosome region.

In this invention, the body part or magnetosomes exposed to the acoustic wave can mean that the acoustic wave covers, targets, is present in, is applied in or on, or is located in at least 10⁻⁹, 10⁻⁷, 10⁻⁵, 10⁻³, 10⁻¹, 1, 5, 10, 25, 50, 75, or 80% of the body part or magnetosome(s). This percentage can represent the number or volume of magnetosomes or body part exposed to the acoustic wave divided by the total number or volume of magnetosomes or body part. In some cases, the acoustic wave can also cover, target, be present, be applied in or on, or be located outside of the body part or magnetosomes, preferentially when these acoustic waves are of low enough power or energy not to induce toxicity.

Furthermore, in some cases the body part or magnetosomes can be exposed to the acoustic wave when an acoustic wave is applied on the body part or magnetosomes or when the body part or magnetosomes are subjected to the application of the acoustic wave or when the body part or magnetosomes undergo the acoustic wave or when the body part or magnetosomes undergo the effects of the acoustic wave or when the body part or magnetosomes undergo the disturbance created by the acoustic wave or when the body part or magnetosomes undergo the disturbance of the acoustic wave.

In this invention, magnetosome(s) can be in some cases designated by the word "*nanoparticle(s).*"

In one embodiment of the invention, magnetosome(s) or the magnetosome(s) represent(s) an assembly or suspension of more than 1, 10, 10², 10³, 10⁵, 10¹⁰, 10²⁰ , or 10⁵⁰ magnetosome(s). In an embodiment of the invention, a compound is bound or attached to the magnetosomes, preferentially before the application of the acoustic wave on the magnetosomes.

In an embodiment of the invention, the compound is a therapeutic, immunogenic, metabolic, luminescent, fluorescent, radioactive, diagnostic, biologic, or chemical compound. In some cases, the compound(s) can be or represent an assembly of more than 1, 10, 10², 10³, 10⁵, 10⁷, 10¹⁰, 10²⁰, or 10⁵⁰ compounds. It is preferentially linked or bound to the magnetosomes. In some cases, the compound can dissociate from the magnetosomes under the application of the acoustic wave. In some cases, more than 10⁻⁵, 10⁻³, 10⁻¹, 1, 5, 10, 25, 50, 75, 85, or 90% of compounds can be dissociated from the magnetosomes, where this percentage can represent the ratio between the quantity of compounds dissociated from the magnetosomes following or under the application of the acoustic wave and the quantity of compounds linked or bound to the magnetosomes before or without the application of the acoustic wave. In some cases, the number of compounds linked or bound to one magnetosome can be larger than 1, 2, 5, 10, 10³, 10⁵, or 10¹⁰. In some other cases, the number of compounds linked or bound to one magnetosome is lower than 2, 5, 10, 10³, 10⁵, or 10¹⁰. In still some other cases, the percentage of compounds dissociated can increase by a factor of at least 1.01, 1.1, 2, 5, 7, 10, 10², or 10⁵ between before and after the application of the acoustic wave on the magnetosomes.

In one embodiment of the invention, the compound is part of the magnetosome. In this case, it can be free iron or free oxygen, preferentially in the ionic form, that preferentially dissociates or leaks or diffuses away from the magnetosome, preferentially under or following the application of the acoustic wave on the magnetosome or body part, preferentially following dissolution of the magnetosome, preferentially following administration of the magnetosome in/to the body part.

In another embodiment of the invention, the ratio between the mass, number, or weight, of the compounds, preferentially linked to a single magnetosome, and the mas, number, or weight of a single magnetosome is lower than 10²⁰, 10⁹, 10⁵, 10², 2, 1, 10⁻², 10⁻⁵, 10⁻⁹, or 10⁻²⁰.

In another embodiment of the invention, the ratio between the mass, number, or weight, of the compound, preferentially linked to a single magnetosome, and the mas, number, or weight of a single magnetosome is larger than 10²⁰, 10⁹, 10⁵, 10², 1, 10⁻², 10⁻⁵, 10⁻⁹, or 10⁻²⁰.

In one embodiment of the invention, a suitable range of values for the number of compounds preferentially linked to a single magnetosome is between 1 and 178, where this minimum value of 1 corresponds to the minimum number of compounds that can be linked to a single magnetosomes and the maximum value of 178 corresponds to the number of RhB molecules that was linked to a single magnetosome in patent WO2017/068252 incorporated in reference, and which could dissociate at least in part from a single magnetosome. In some cases, this maximum value of 178 can be increased, preferentially by a factor of more than 5, 10, 10³, 10⁷, 10¹⁰, or 10²⁰, by: i), decreasing the size, or mass of the compound linked to the magnetosome, preferentially by a factor of more than 1.1, 2, 5, 10, 10³, 10⁷, 10¹⁰, or 10²⁰, ii), changing the type of bounds between the compounds and the magnetosome, or iii), changing the method for attaching or binding the compound to the magnetosomes. An assembly or suspension of magnetosomes comprising more than 1, 10, 10², 10³, 10⁵, 10¹⁰, 10²⁰, or 10⁵⁰ magnetosome(s) can be used to produce a temperature increase or the dissociation of a compound from the magnetosomes.

In another embodiment of the invention, magnetosome(s) or the magnetosome(s) represent(s) an assembly or suspension of less than 2, 10, 10², 10³, 10⁵, 10¹⁰, 10²⁰, or 10⁵⁰ magnetosome(s). An assembly or suspension of magnetosomes comprising less than 2, 10, 10², 10³, 10⁵, 10¹⁰, 10²⁰, or 10⁵⁰ magnetosome(s) can be used to prevent toxicity due to the application of the acoustic wave on magnetosomes.

In another embodiment of the invention, the magnetosome region represents the volume comprising an assembly of more than 1, 10, 10², 10³, 10⁵, 10¹⁰, 10²⁰, or 10⁵⁰ magnetosome(s). In some cases, a volume comprising a large number of magnetosomes can enable to induce a temperature increase or to dissociate the compound from the magnetosomes.

In still another embodiment of the invention, the magnetosome region represents the volume comprising an assembly of less than 2, 10, 10², 10³, 10⁵, 10¹⁰, 10²⁰, or 10⁵⁰ magnetosome(s). In some cases, a volume comprising a low number of magnetosomes can prevent toxicity induced by the application of the acoustic wave on magnetosomes.

In some cases, the magnetosome(s) or magnetosome(s) can represent the magnetosome region or magnetosome assembly.

In this invention, the magnetosome region can also be defined as the space, surface area, or volume, where magnetosomes are located, preferentially comprising more than 1, 2, 5, 10, 10², 10³, 10⁵, 10¹⁰, 10¹⁵, 10²⁰, or 10⁵⁰ magnetosome(s).

In this invention, the magnetosome region can also be defined as the space, surface area, or volume, where magnetosomes are located, preferentially comprising less than 1, 2, 5, 10, 10², 10³, 10⁵, 10¹⁰, 10¹⁵, 10²⁰, or 10⁵⁰ magnetosome(s).

In one embodiment of the invention, the magnetosome is defined as a particle with a size in one dimension, which is larger than 10⁻¹, 1, 2, 5, 10, 20, 50, 70, 100, 200, or 500 nm. A magnetosome with a large size can have a larger coercivity and/or a larger remanent magnetization and/or can more strongly or more efficiently absorb the energy or power of the acoustic wave than a magnetosome with a small size. In some cases, the amount of energy or power absorbed by a magnetosome can be increased by a factor of 1.001, 1.01, 1.1, 1.2, 1.5, 2, 5, 10, 10³, 10⁵, or 10⁷ by increasing the size of the magnetosome by a factor of 1.001, 1.01, 1.1, 1.2, 1.5, 2, 5, 10, 10³, 10⁵, or 10⁷.

In another embodiment of the invention, the magnetosome is defined as a particle with a size in one dimension, which is lower than 10⁴, 10³, 10², 10, 1, or 10⁻¹ nm. A magnetosome with a small size can more easily be administered, for example intravenously, or can enable the avoidance of some toxicity effects, such as embolism.

In still another embodiment of the invention, the magnetosome size lies between 10⁻² and 10⁴ nm, between 10⁻¹ and 10³ nm, or between 1 and 10² nm. This can be the case when the magnetosome or magnetosome assembly possesses a well-defined, preferentially narrow, distribution in sizes.

In still another embodiment of the invention, the magnetosome size distribution is lower than 1000, 100, 75, 50, 25, 10, 5, 2, or 1 nm. A narrow magnetosome size distribution may be desired to prevent aggregation, or to favor an organization in chains of the magnetosomes.

In still another embodiment of the invention, the magnetosome size distribution is larger than 1000, 100, 75, 50, 25, 10, 5, 2, or 1 nm. A large magnetosome size distribution may in some cases enable magnetosomes to be eliminated more rapidly.

In another embodiment of the invention, the magnetosome has a surface charge, which is larger than -200, -100, -50, -10, -5, 0.1, 1, 2, 5, 10, 50, or 100 mV, preferentially at a pH lower than 0.1, 1, 2, 3, 4, 5, 6, 7, 8, 9, 10, 11, 12, 13, or 14.

Preferentially, a magnetosome can have a large surface charge at low pH when its mineral central part is surrounded by a coating that enables to reach such charge without being destroyed.

In another embodiment of the invention, the magnetosome has a surface charge, which is lower than -200, -100, -50, -10, -5, 0.1, 1, 2, 5, 10, 50, or 100 mV, preferentially at a pH larger than 0.1, 1, 2, 3, 4, 5, 6, 7, 8, 9, 10, 11, 12, 13, or 14. A magnetosome can have a low surface charge at high pH when its mineral central part is surrounded by a coating that enables to reach such charge without being destroyed.

In still another embodiment of the invention, the magnetosome has a surface charge comprised between +200 and -200 mV, +100 and -100 mV, +50 and -50 mV, +40 et-40mV, +20 and -20, +10 and -10 mV, or between +5 and -5 mV, preferentially at a pH lower than 0.1, 1, 2, 3, 4, 5, 6, 7, 8, 9, 10, 11, 12, 13, or 14.

In still another embodiment of the invention, the magnetosome has a surface charge comprised between +200 and -200 mV, +100 and -100 mV, +50 and -50 mV, +40 et-40mV, +20 and -20, +10 and -10 mV, or between +5 and -5 mV, preferentially at a pH larger than 0.1, 1, 2, 3, 4, 5, 6, 7, 8, 9, 10, 11, 12, 13, or 14.

In another embodiment of the invention, the magnetosome has a weight or a mass, preferentially expressed in unit such as gram (g), kilogram (kg), or milligram (mg). A gram of magnetosome can be a gram of metal such as iron comprised in the magnetosome. The mass or weight of the magnetosome can correspond to the mass or weight of one magnetosome or to the mass or weight of an assembly of magnetosomes.

In an embodiment of the invention, the mass of the magnetosome is larger than 10⁻²⁰, 10⁻¹⁰, 10⁻⁵, 10⁻², 1, 10, 10³, 10⁹, 10²⁰ gram. In some cases, a large magnetosome mass may be desired to increase the quantity of acoustic wave energy absorbed by the magnetosome.

In an embodiment, the mass of the magnetosome is lower than 10⁻²⁰, 10⁻¹⁰, 10⁻⁵, 10⁻², 1, 10, 10³, 10⁹, 10²⁰ gram. In some cases, a low magnetosome mass may be desired to prevent or minimize magnetosome toxicity.

In another embodiment of the invention, the magnetosome or magnetosome suspension or magnetosome assembly has a concentration, preferentially expressed in unit such as gram (g), kilogram (kg), or milligram (mg) of magnetosomes, iron, iron oxide, maghemite, magnetite, per unit volume such as liter (1), milliliter (ml), cm³, or m³, or per unit surface area or per unit length. The magnetosome, magnetosome suspension, or magnetosome assembly, has preferentially a concentration before or after administration in the body part, wherein the volume, surface area, or length, is preferentially the volume, surface area, or length of the magnetosome suspension or assembly before or after administration in the body part, or the volume, surface area, or length of the body part.

In another embodiment of the invention, the magnetosome is surrounded by an organic or inorganic coating, wherein the coating preferentially enables to bind two or more magnetosome(s) together preferentially in a chain and/or prevents magnetosome aggregation and/or enables uniform magnetosome distribution.

In another embodiment of the invention, the magnetosomes are arranged in chains, wherein the chains are characterized by a length that is shorter than 2.10⁵, 2.10³, or 2.10² nm or by a number of magnetosomes in each chain that is preferentially lower than 2, 5, 10, 10², or 10³. In some cases, short chains of magnetosomes may be desired, for example to favor magnetosome internalization in cells.

In another embodiment of the invention, the magnetosomes are arranged in chains, wherein the chains are characterized by a length that is longer than 2. 10⁵, 2.10³, or 2.10² nm or by a number of magnetosomes in each chain that is preferentially larger than 2, 5, 10, 10², or 10³. In some cases, long chains of magnetosomes may be desired to increase the quantity of heat or compounds dissociated from the magnetosomes under the application of an acoustic wave. In still another embodiment of the invention, the magnetosomes are arranged in chains when they are bound or linked to each other or when the crystallographic directions of two adjacent magnetosomes in the chain are aligned, wherein such alignment is preferentially characterized by an angle between two crystallographic directions belonging to two adjacent magnetosomes in the chains of less than 90, 80, 70, 60, 50, 20, 10, 3, or 2° (degree).

The invention also relates to magnetosomes for use according to the invention, wherein the magnetosomes are crystallized, metallic, or magnetic.

In an embodiment of the invention, the magnetosomes are crystallized. In this case, they preferentially possess more than 1, 2, 10, 10², 10³, 10⁶, or 10⁹ crystallographic plane(s) or regular atomic arrangement(s), preferentially observable by electron microscopy.

In one embodiment of the invention, the magnetosomes are metallic. In this case, they contain at least 1, 10, 10³, 10⁵, or 10⁹ metallic atom(s) or contain at least 1, 10, 50, 75, or 90% of metallic atoms, where this percentage can be the ratio between the number or mass of metallic atoms in the magnetosome divided by the total number or mass of all atoms in the magnetosome. The magnetosomes can also contain at least 1, 10, 10³, 10⁵, or 10⁹ oxygen atom(s), or contain at least 1, 10, 50, 75, or 90% of oxygen atoms, where this percentage can be the ratio between the number or mass of oxygen atoms in the magnetosome divided by the total number or mass of all atoms in the magnetosome.

In another embodiment of the invention, the magnetosome contains less than 1, 10, 10³, 10⁵, or 10⁹ metallic atom(s) or contains less than 1, 10, 50, 75, or 90% of metallic atoms, where this percentage can be the ratio between the number or mass of metallic atoms in the magnetosome divided by the total number or mass of all atoms in the magnetosome. It can also contain less than 1, 10, 10³, 10⁵, or 10⁹ oxygen atom(s), or contain less than 1, 10, 50, 75, or 90% of oxygen atoms, where this percentage can be the ratio between the number or mass of oxygen atoms in the magnetosome divided by the total number or mass of all atoms in the magnetosome.

In one embodiment of the invention, the magnetosome is magnetic. In this case, the magnetosome can have a diamagnetic, superparamagnetic, paramagnetic, ferromagnetic, or ferrimagnetic behavior or property, preferentially at a temperature, which is lower than 0.5, 1, 10, 20, 50, 100, 200, 350, 500, 10³, or 10⁵ K.

In another embodiment of the invention, the magnetosome is magnetic when its diamagnetic, superparamagnetic, paramagnetic, ferromagnetic, or ferrimagnetic behavior or property is observed at a temperature, which is larger than 0.5, 1, 10, 20, 50, 100, 200, 350, 500, 10³, or 10⁵ K.

In another embodiment of the invention, the magnetosomes are assimilated to or are comprised in a ferrofluid, a chemical or biological ferrofluid, wherein chemical and biological ferrofluids are fluids containing iron, preferentially forming nanoparticles, which are fabricated through a chemical and biological synthesis, respectively. A chemical synthesis can be defined as a synthesis involving a majority of steps, or more than 1, 2, 5, or 10 steps, or more than 1, 2, 5, 25, 50, 75, or 90% of steps, which involve chemical reactions occurring without the involvement of living organisms, or parts of living organisms such as DNA, RNA, proteins, enzymes, lipids. A biological synthesis can be defined as a synthesis involving a majority of steps, or more than 1, 2, 5, or 10 steps, or more than 1, 2, 5, 25, 50, 75, or 90% of steps, which involve chemical reactions occurring with the involvement of at least 1, 2, 10, 10³, 10⁶, or 10⁹ living organisms, or parts of living organisms such as DNA, RNA, proteins, enzymes, lipids. The ferrofluid can comprise the magnetosomes and an excipient, a solvent, a matrix, a gel, which preferentially enables the administration of the magnetosomes to the individual or body part.

In one embodiment of the invention, the magnetosomes are nanoparticles characterized by at least one of the following properties: i), the presence of a core, preferentially magnetic, preferentially mineral, preferentially composed of iron oxide, most preferentially maghemite or magnetite, or an intermediate composition between maghemite and magnetite, ii), the presence of a coating that surrounds the magnetic core and preferentially prevents magnetosome aggregation, preferentially enabling magnetosome administration in an organism or in the body part or stabilizing the magnetosome core, where coating thickness may preferably lie between 0.1 nm and 10 µm, between 0.1 nm and 1 µm, between 0.1 nm and 100 nm, between 0.1 nm and 10 nm, or between 1 nm and 5 nm, iii), magnetic properties leading to diamagnetic, paramagnetic, superparamagnetic, ferromagnetic, or ferrimagnetic behavior, iv), a coercivity larger than 0.01, 0.1, 1, 10, 100, 10³, 10⁴, 10⁵, 10⁹, or 10²⁰ Oe, v), a ratio between remanent and saturating magnetization larger than 0.01, 0.1, 0.2, 0.3, 0.4, 0.5, 0.75, 0.9, or 0.99, vi), a saturating magnetization larger than 0.1, 1, 5, 10, or 50 emu/g, vii), magnetic properties such as coercivity, remanent and saturating magnetization, preferentially measured or observed at a temperature larger than 0.1 K, 1 K, 10 K, 20 K, 50 K, 100 K, 200 K, 300 K, 350 K, or 3000 K, viii), a crystallinity, *i.e.* magnetosomes preferentially possessing at least 1, 2, 5, 10, or 100 crystalline plane(s), preferentially observable or measured by electron microscopy, ix), the presence of a single domain, x), a size that is larger than 0.1, 0.5, 1.5, 10, 15, 20, 25, 30, 50, 60, 70, 80, 100, 120, 150, or 200 nm, xi), a size lying between 0.1 nm and 10 µm, between 0.1 nm and 1 µm, between 0.1 nm and 100 nm, between 1 nm and 100 nm, or between 5 nm and 80 nm, xii), a non-pyrogenicity or apyrogenicity, which preferentially means that magnetosomes possess an endotoxin concentration lower than 10000, 1000, 100, 50, 10, 5, 2, or 1 EU (endotoxin unit) per mg of magnetosome or per mg of iron comprised in magnetosomes, or which means that magnetosomes do not trigger fever or an increase in whole body temperature of more than 6.6, 5, 3, 2, or 1 °C preferentially following their administration to a living organism or body part, xiii), a synthesis by a synthetizing living organism, preferentially by magnetotactic bacteria, leading to the production of magnetosomes, preferentially extracted from magnetotactic bacteria, preferentially only or mostly comprising the mineral magnetic core of the magnetosomes, xiv), the presence of less than 50, 25, 15, 10, 5, 2, or 1% of organic or carbon material originating from the synthetizing living organism, xv), the presence of more than 99, 95, 80, 70, 60, 50, or 25% of mineral material originating from the synthetizing living organism, or xvi), a specific absorption rate (SAR) that is larger than 1, 10, 1000, or 10⁴ Watt per gram of magnetosome, preferentially measured under the application of an alternating magnetic field of strength preferentially larger than 0.1, 1, 10, or 100 mT, and/or frequency larger than 1, 10, 100, or 1000 KHz, alternatively preferentially measured under the application of the acoustic wave.

In another embodiment of the invention, the magnetosomes are nanoparticles characterized by at least one of the following properties: i), a coercivity lower than 0.01, 0.1, 1, 10, 100, 10³, 10⁴, 10⁵, 10⁹, or 10²⁰ Oe, ii), a ratio between remanent and saturating magnetization lower than 0.01, 0.1, 0.2, 0.3, 0.4, 0.5, 0.75, 0.9, or 0.99, iii), a saturating magnetization lower than 0.1, 1, 5, 10, 50, 200, 1000, or 5000 emu/g, iv), magnetic properties preferentially measured or observed at a temperature lower than 0.1 K, 1 K, 10 K, 20 K, 50 K, 100 K, 200 K, 300 K, 350 K, or 3000 K, viii), a size that is lower than 0.1, 0.5, 1.5, 10, 15, 20, 25, 30, 50, 60, 70, 80, 100, 120, 150, or 200 nm, ix), the presence of more than 50, 25, 15, 10, 5, 2, or 1% of organic or carbon material originating from the synthetizing living organism, x), the presence of less than 99, 95, 80, 70, 60, 50, or 25% of mineral material originating from the synthetizing living organism, or xi), a specific absorption rate (SAR) that is lower than 1, 10, 1000, or 10⁴ Watt per gram of magnetosome, preferentially measured under the application of an alternating magnetic field of strength preferentially lower than 0.1, 1, 10, or 100, 200, 500, 10³, or 10⁵ mT, and/or of frequency preferentially lower than 1, 10, 100, 10³, 10⁵, or 10⁹ KHz, alternatively preferentially measured under the application of the acoustic wave.

In one embodiment of the invention, magnetosomes are arranged in chains comprising more than 1, 2, 3, 4, 5, 6, 7, 8, 9, 10, 15, 20, 25, 30, 35, or 40 magnetosomes. Magnetosomes can be arranged in chains inside magnetotactic bacteria or outside magnetotactic bacteria, preferentially after their extraction or isolation from magnetotactic bacteria.

In one embodiment of the invention, magnetosomes are not arranged in chains.

In one embodiment of the invention, magnetosomes are purified to remove more than 10, 50, or 90% of endotoxins and/or other biological material such as proteins and lipids originating from magnetotactic bacteria. Such purification can use detergent such as NaOH or KOH. Purified magnetosomes can be recoated with a synthetic coating such as a substance comprising a function selected in the group comprising carboxylic acids, phosphoric acids, sulfonic acids, esters, amides, ketones, alcohols, phenols, thiols, amines, ether, sulfides, acid anhydrides, acyl halides, amidines, amides, nitriles, hydroperoxides, imines, aldehydes, or peroxides. The coating can be made of carboxy-methyl-dextran, citric acid, phosphatidylcholine (DOPC), or oleic acid. In some cases, the coating can enable the dispersion of the magnetosomes in a matrix or solvent such as water. Purified magnetosomes (recoated or not) are preferentially non-pyrogenic. They comprise less than 10⁸, 10⁵, 10³, or 10 EU (endotoxin unit) per mm³ or mL of magnetosomes, of magnetosome suspension, or of body part. Purified magnetosomes (recoated or not) can be re-suspended in a liquid or redispersed in a matrix to lead to a homogenous dispersion or to a high stability.

In one embodiment of the invention, a magnetosome suspension is stable, which preferentially means that it is stable at a concentration larger than 1, 5, 10, 50, 100, 200, 500, or 1000 mg of magnetosome per mL of solvent, *i.e.* the optical density of this suspension, preferentially measured at 480 nm or another fixed wavelength, does not decrease by more than 1, 5, 10, 50, 75, or 90 %, preferentially within 1, 5, 10, 10³, 10⁷, or 10²⁰ seconds following homogenization or mixing of this suspension.

In one embodiment of the invention, magnetosomes are synthesized by a living organism, which consists or comprises at least 1, 2, 5, 10, 10³, 10⁶, or 10⁹ eukaryotic or prokaryotic cell(s).

In one embodiment of the invention, magnetosomes are synthesized by a living organism when at least 1, 2, 5, 10 or 100 step(s) of their production, such as crystallization of iron oxide, stabilization of the iron oxide mineral, organization of the magnetosome minerals, for example in chains or aggregates, involves or is due to a living organism.

In another embodiment of the invention, magnetosomes are not synthesized by a living organism when less than 1, 2, 5, 10 or 100 step(s) of their production, such as crystallization of iron oxide, stabilization of the iron oxide mineral, organization of the magnetosome minerals, for example in chains or aggregates, involves or is due to a living organism.

In one embodiment of the invention, magnetosomes are synthesized by magnetotactic bacteria such as such as *Magnetospirillum magneticum* strain AMB-1, magnetotactic coccus strain MC-1, three facultative anaerobic vibrios strains MV-1, MV-2 and MV-4, the *Magnetospirillum magnetotacticum* strain MS-1, the *Magnetospirillum gryphiswaldense* strain MSR-1, a facultative anerobic magnetotactic spirillum, *Magnetospirillum magneticum* strain MGT-1, and an obligate anaerobe, *Desulfovibrio magneticus* RS-1.

In one embodiment of the invention, a magnetotactic bacterium is defined as a bacterium able to synthesize magnetosomes, wherein these magnetosomes are preferentially characterized by at least one of the following properties: i), they are produced intracellularly, ii), they are magnetic, iii), they comprise a mineral, iv), their core is preferentially composed of a metallic oxide such as iron oxide, v), their core is surrounded by biological material such as lipids, proteins, endotoxins, which can preferentially be removed, v), they are arranged in chains, vi), they produce heat under the application of an alternating magnetic field.

In one embodiment of the invention, magnetosomes comprise the mineral part synthesized by magnetotactic bacteria, *i.e.* preferentially the crystallized iron oxide produced by these bacteria. In this case, magnetosomes or magnetosome mineral parts preferentially do not comprise proteins, lipids, endotoxins, or biological materials comprising carbon or more than 0.1, 1, 10, 30, 50, 75% of carbon, which is/are produced by these bacteria.

In one embodiment of the invention, magnetosomes are assimilated to chemical nanoparticles, *i.e.* to nanoparticles that have not been synthesized by the synthetizing living organism, most preferentially by a magnetotactic bacterium, but possess at least 1, 2, 3, 4, 5, 6, 7, 8, 9, or 10 common property(ies) with the magnetosomes, where these common properties are preferentially a ferrimagnetic behavior, a large size, and/or a chain arrangement.

In one embodiment of the invention, an acoustic wave medical treatment is a medical treatment also designated as treatment, which uses acoustic wave energy, intensity, or power, preferentially applied on magnetosomes or body part, to trigger a medical, pharmaceutical, immunological, metabolic, diagnostic, medical device, drug, biological, or cosmetic effect. In some cases, a medical treatment can be the treatment of an illness such as an infectious disease, a cancer, or a therapeutic treatment. It can be the treatment of a disease due to the malfunction of an organ or body part. It can be due to the malfunction of a body part of an individual. In some cases, a medical treatment can be a diagnostic of a disease or a cosmetic treatment.

In one embodiment of the invention, the medical treatment is the treatment of an anemia, preferentially an anemia of a substance comprised in the body part or compound, preferentially an anemia in iron. In some cases, the anemia can be defined as a concentration in a substance comprised in an individual, which is more than 1.001, 1.01, 1.1, 2, 5, 10, 10², 10⁵, 10¹⁰, or 10²⁰ times lower in the individual suffering from anemia than in a healthy individual.

In some cases, anemia of a substance comprised in the body part is defined as a concentration of a substance comprised in the magnetosome or compound, such as iron or oxide, which is lower, preferentially 1.001, 1.01, 1.1, 2, 5, 10, 10², 10⁵, 10¹⁰, or 10²⁰ times lower, in the body part before or without magnetosome administration than after or with magnetosome administration.

In an embodiment of the invention, the body part comprises more than 1, 2, 5, 10, or 100 similar or different organism(s), apparatus, organ(s), tissue(s), cell(s), or biomolecule(s). The body part can be all or part of the head, neck, shoulder, arm, leg, knee, foot, hand, ankle, elbow, trunk, inferior members, or superior members.

In one embodiment of the invention, the body part is or comprises water, an excipient, a solution, a suspension, at least one chemical element, organic material, or gel, which can be synthetic or produced by a living organism.

In an embodiment of the invention, the organ or body part belongs to the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, integumentary system, reproductive organ (internal or external), sensory organ, or endocrine glands. The organ or body part can be human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, or skin. The body part or organ can belong to the circulatory system.

In an embodiment of the invention, the magnetosomes according to the invention are drugs, medical devices, cosmetic products, biological products, products used for research purposes, or products used to determine the properties of biological samples.

In one embodiment of the invention, an acoustic wave medical treatment is a treatment, which induces the death, destruction, denaturation, or inactivation of at least 1, 10, 10³, 10⁶, or 10⁹ biological material(s), such as cell(s), preferentially pathological cell(s), RNA, DNA, protein(s), lipid(s), or enzyme(s). The death of cell(s) can occur through apoptosis or necrosis, but is preferentially occurring through apoptosis.

Preferably, the body part of an individual also designated as body part represents part of an individual, where the individual is preferentially a human, an animal, or an organism comprising at least one prokaryotic or eukaryotic cell, or an assembly of at least one prokaryotic or eukaryotic cell.

In one embodiment of the invention, the body part is alive (or not), is any tissue, water, medium, substance, cell, organelle, organ protein, lipid, DNA, RNA, biological material, preferentially localized in a specific region of an individual, preferentially originating or extracted from such region.

In one embodiment of the invention, the magnetosomes are administered to or in the body part, when they are directly administered to the body part or when they are administered close to the body part, preferentially less than 1, 10⁻¹, 10⁻², 10⁻³, 10⁻⁴, 10⁻⁵, 10⁻⁶, or 10⁻⁹ m away from the body part. In this case, the magnetosomes may not need to be transported or diffuse from the region where they are administered to the body part.

In another embodiment of the invention, the magnetosomes are administered to or in the body part, when they are administered far from the body part, preferentially more than 1, 10⁻¹, 10⁻², 10⁻³, 10⁻⁴, 10⁻⁵, 10⁻⁶, or 10⁻⁹ m away from the body part. In this case, the magnetosomes may be transported or diffuse from the region where they are administered to the body part.

In another embodiment of the invention, the magnetosomes are administered to or in the body part when they are injected in, or mixed with, or introduced in, or inserted in the body part.

In another embodiment of the invention, the magnetosomes are administered to or in the body part when they occupy more than 10⁻⁹, 10⁻⁷, 10⁻⁵, 10⁻³, 1, 10, 25, 50, or 75 % of the body part, where this percentage can be the ratio between the volume of the region occupied by the magnetosomes in the body part or magnetosome region and the volume of the body part. This occupation can correspond to that measured 10⁻⁵, 10⁻³, 10⁻¹, 1, 10, 10³, or 10⁵ minutes following magnetosome administration.

In another embodiment of the invention, the magnetosomes are administered to or in the body part following at least one of the following administration routes: local, enteral, gastrointestinal, parenteral, topical, oral, inhalation, intramuscular, subcutaneous, intra-tumor, in an organ, in a vein, in arteries, in blood, or in tissue.

In one embodiment of the invention, the body part is a pathological site. The pathological site can be defined as an unhealthy site, or a site that is in a different condition from a site of a healthy individual, or the site of an unhealthy individual. It can comprise pathological cells, such as tumor cells, bacteria, eukaryotic or prokaryotic cells, viruses or other pathological material. It can also comprise healthy cells, which are not arranged or working as they usual do in a healthy individual. For example, it can comprise a larger or lower number of healthy cells, or it can comprise healthy cells that do not function as they usually do in a healthy individual.

In another embodiment of the invention, the body part, healthy or pathological site, or magnetosome region, has a size, length, surface area, or volume, which is larger than 1, 10⁻¹, 10⁻², 10⁻³, 10⁻⁴, 10⁻⁵, 10⁻⁶, 10⁻⁷, 10⁻⁸, or 10⁻⁹ m or 10⁻⁹ m² or 10⁻⁹ m³.

In another embodiment of the invention, the body part, healthy or pathological site, or magnetosome region, has a size, length, surface area, or volume, which is lower than 1, 10⁻¹, 10⁻², 10⁻³, 10⁻⁴, 10⁻⁵, 10⁻⁶, 10⁻⁷, 10⁻⁸, or 10⁻⁹ m or 10⁻⁹ m² or 10⁻⁹ m³.

In another embodiment of the invention, the pathological site is defined as a site or region that comprises pathological cell(s), where a pathological cell can be defined as a cell that does not work as a healthy cell, for example a cell that divides more quickly than a healthy cell. In some cases, the number of pathological cells can be lower than 1, 10, 10³, 10⁵, 10⁷, 10⁹, or 10²⁰. In some other cases, the number of pathological cells is larger than 1, 10, 10³, 10⁵, 10⁷, 10⁹, or 10²⁰.

In another embodiment of the invention, the healthy site is defined as a site or region that comprises healthy cell(s), where a healthy cell can be defined as a cell that belongs to a healthy individual or to the body part of a healthy individual. In some cases, the number of healthy cells can be larger than 1, 10, 10³, 10⁵, 10⁷, 10⁹, or 10²⁰. In some other cases, the number of healthy cells is smaller than 1, 10, 10³, 10⁵, 10⁷, 10⁹, or 10²⁰.

In one embodiment of the invention, the body part comprises the pathological site, the healthy site, or the magnetosome region.

In some cases, the body part can comprise: i), more than 10⁻⁹, 10⁻⁷, 10⁻⁵, 10⁻³, 10⁻¹, 1, 10, 10³, 10⁵, 10⁷, or 10⁹ mg of magnetosomes, preferentially per mm³ or per cm³ of body part, or ii), more than 10⁻⁹, 10⁻⁷, 10⁻⁵, 10⁻³, 10⁻¹, 1, 10, 10³, 10⁵, 10⁷, or 10⁹ pathological or healthy cells, preferentially per mm³ or per cm³ of body part.

In some other cases, the body part can comprise: i), less than 10⁻⁹, 10⁻⁷, 10⁻⁵, 10⁻³, 10⁻¹, 1, 10, 10³, 10⁵, 10⁷, or 10⁹ mg of magnetosomes, preferentially per mm³ or per cm³ of body part, or ii), less than 10⁻⁹, 10⁻⁷, 10⁻⁵, 10⁻³, 10⁻¹, 1, 10, 10³, 10⁵, 10⁷, or 10⁹ pathological or healthy cells, preferentially per mm³ or per cm³ of body part.

In one embodiment of the invention, a suitable range of magnetosome concentration comprised in the body part is between 1 ng of magnetosome, preferentially in iron, per mm³ of body part and 1 gram of magnetosome, preferentially in iron, per mm³ of body part. The minimum value of this range (1 ng/mm³) was estimated by calculating the lowest concentration of a magnetosome suspension that can be administered in the body part, *i.e.* the lowest magnetosome concentration that can typically be detected. In some cases, it is possible that this minimum value is decreased, for example by a factor larger than 10, 10³, 10⁶, or 10⁹, if more sensitive detection methods are used or developed to detect the presence of the magnetosomes or if part of the magnetosomes have diffused away from the body part following their administration in the body part. The maximum value of this range (1 g/mm³) was estimated by calculating the largest magnetosome concentration that yields a stable suspension. It is possible that this maximum value is larger, for example by a factor of 10, 10³, 10⁶, or 10⁹, for example if magnetosomes are inserted in a matrix or solvent that is solid, semi-solid, or more viscous than water, or if magnetosomes have concentrated in the body part following their administration in the body part yielding a larger concentration in the body part than in the magnetosome suspension used for injection.

In one embodiment of the invention, the body part is associated with: i), the body part exposed to the acoustic wave(s), or ii), the body part receiving the acoustic wave energy or power, or iii), the body part absorbing the acoustic wave energy or power.

In another embodiment of the invention, the body part is associated with: i), the body part not exposed to the acoustic wave(s), or ii), the body part not receiving the acoustic wave energy or power, or iii), the body part not absorbing the acoustic wave energy or power.

The invention also relates to magnetosomes for use according to the invention, wherein the medical treatment is the treatment of a disorder or malfunction of the body part, of an infectious disease, an auto-immune disease, a neuropathology, a cancer, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, or a personality disorder.

In one embodiment, the disorder or malfunction of the body part is associated with the malfunction of cells, which divide more rapidly or enter in an apoptotic or necrotic state for example, or with the malfunction of the immune system or immune cell(s).

The invention also relates to magnetosomes for use according to the invention, wherein the magnetosomes possess a specific absorption rate larger than 10⁻⁹ Watt per gram of magnetosome.

In one embodiment of the invention, the magnetosomes possess a specific absorption rate (SAR), which is larger than 10⁻⁹, 10⁻⁶, 10⁻³, 1, 10³, 10⁶, or 10⁹ Watt per gram of magnetosomes (W/g_{M}), per cm³ of magnetosomes (W/cm³_{M}), per gram of body part (W/g_{bp}), or per cm³ of body part (W/cm³_{bp}).

In one embodiment of the invention, the magnetosomes possess a specific absorption rate (SAR), which is lower than 10⁻⁹, 10⁻⁶, 10⁻³, 1, 10³, 10⁶, or 10⁹ Watt as measured per gram of magnetosomes (W/g_{M}), per cm³ of magnetosomes (W/cm³_{M}), per gram of body part (W/g_{bp}), or per cm³ of body part (W/cm³_{bp}).

In one embodiment of the invention, a suitable range of magnetosome SAR, preferentially measured under the application of the acoustic wave, is between 1.2 Watt per gram of magnetosome in iron and 424 Watt per gram of magnetosome in iron. In some cases, the minimum value of this range (1.2 W/g_{M}) can be lower, for example by a factor of more than 1.5, 2, 5, 10, 10³, 10⁵, 10⁷, or 10⁹, preferentially when the viscosity of the body part is increased or when the thermal diffusion of the body part is decreased or when magnetosome diffusion away from the body part is increased or when the power of the acoustic wave is decreased or when the heat produced by the acoustic wave in the absence of the magnetosomes is increased. In some other cases, the maximum value of this range (424 W/g_{M}) can be larger, for example by a factor of more than 1.5, 2, 5, 10, 10³, 10⁵, 10⁷, or 10⁹, preferentially when the viscosity of the body part is decreased or when the thermal diffusion of the body part is increased or when magnetosome concentration in the body part is increased or when the power of the acoustic wave is increased or when the heat produced by the acoustic wave in the absence of the magnetosomes is decreased.

In one embodiment of the invention, the SAR is estimated by exposing magnetosomes to the acoustic wave. In some cases, it can be equal or proportional to the specific heat capacity of the medium surrounding the magnetosomes times the initial slope of the temperature variation with time resulting from the application of the acoustic wave divided by the magnetosome concentration.

In another embodiment of the invention, the SAR does not correspond to or is not associated with the SAR estimated by exposing the magnetosomes to an alternating magnetic field or to another source of excitation than an acoustic wave.

In one embodiment of the invention, large SAR values may be obtained in some specific conditions, for example using acoustic waves of high power or frequency or using high magnetosome concentration or combining the application of the acoustic wave with the application of another source of energy.

In another embodiment of the invention, the lowest values of the SAR can be justified by the fact that SAR values may decrease with decreasing magnetosome concentration or magnetosome number, preferentially by a factor of more than 1.1, 2, 5, 10, 10², 10³, 10⁵, or 10¹⁰, when the magnetosome concentration or magnetosome number decreases by a factor of more than 1.1, 2, 5, 10, 10², 10³, 10⁵, or 10¹⁰. For example, a SAR of 1 W per gram of magnetosome for 10⁷ magnetosome can lead to a SAR value of 10⁻⁷ W per gram of magnetosome for a single magnetosome. This behavior may be explained by a collective effect in which the SAR values of the individual magnetosomes would add to yield a SAR for the assembly of the magnetosomes, which is the sum of or is proportional to the SAR value of a single magnetosome.

In still another embodiment of the invention, the small magnetosomes with a size preferentially lower than 500, 200, 100, 50, 20, 10, or 1 nm, do not possess a SAR, or possess a SAR that is lower than 10⁵, 10³, 10, 1, 10⁻¹, 10⁻³, 10⁻⁶, or 10⁻⁹ Watt per gram of magnetosome.

In still another embodiment of the invention, the large magnetosomes with a size preferentially larger than 500, 200, 100, 50, 20, 10, or 1 nm, possess a SAR preferentially larger than 10⁵, 10³, 10, 1, 10⁻¹, 10⁻³, 10⁻⁶, or 10⁻⁹ Watt per gram of magnetosome.

The invention also relates to magnetosomes for use according to the invention, wherein the magnetosomes possess a specific absorption rate, which increases with increasing power of the acoustic wave applied on the magnetosomes, preferentially at a rate that increases with decreasing magnetosome concentrations. In some cases, the magnetosome specific absorption rate can increase by a factor of more than 1.01, 1.1, 2, 5, 10, 10², 10³, 10⁵, or 10¹⁰ when the power of the acoustic wave applied on magnetosomes increases by a factor of more than 1.01, 1.1, 2, 5, 10, 10², 10³, 10⁵, or 10¹⁰, preferentially at a rate that increases by a factor of more than 1.01, 1.1, 2, 5, 10, 10, 10³, 10⁵, or 10¹⁰ when the magnetosome concentration decreases by a factor of more than 1.01, 1.1, 2, 5, 10, 10², 10³, 10⁵, or 10¹⁰. To meet this feature, the magnetosome concentration should preferentially be comprised between 10⁻⁹ and 10⁹, 10⁻⁵ and 10⁵, 10⁻² and 10², or between 10⁻¹ and 10 mg of magnetosomes per mL or per cm³ of body part.

In an embodiment of the invention, the rate of SAR increase with increasing intensity of the acoustic wave corresponds to the percentage of SAR increase, *i.e*. (SAR_{I2}-SAR_{I1})/SAR_{I1}, where SAR_{I1} and SAR_{I2} are the SAR measured at two different intensities of the acoustic wave I₁ and I₂ where I₂>I₁, preferentially divided by I₂/I₁.

In an embodiment of the invention, a suitable range of rate of SAR increase with increasing acoustic wave power is between 15% and 440%. The minimum and maximum values of this range were estimated using the values of SARᵣₑₐₗ(M) given in tables 2 and 1, respectively. In some cases, the minimum value of this range can be decreased, preferentially by a factor of more than 2, 5, 10, 10³, or 10⁵, by decreasing the intensity of the acoustic wave or by changing the maximum concentration or body part. In some other cases, the maximum value of this range can be increased, preferentially by a factor of more than 2, 5, 10, 10³, or 10⁵, by increasing the intensity of the acoustic wave or by changing the maximum concentration or body part.

In one embodiment of the invention, the magnetosomes possess a specific absorption rate, which increases when the magnetosome concentration decreases. In some cases, the magnetosome specific absorption rate can increase by a factor of more than 1.1, 2, 5, 10, 10², 10³, 10⁵, or 10¹⁰ when the magnetosome concentration decreases by a factor of 1.1, 2, 5, 10, 10², 10³, 10⁵, or 10¹⁰. To meet this feature, the magnetosome concentration should preferentially be comprised between 10⁻⁹ and 10⁹, 10⁻⁵ and 10⁵, 10⁻² and 10², or between 10⁻¹ and 10 mg of magnetosomes per mL, or the magnetosome concentration should be lower than 10⁻⁹, 10⁻⁷, 10⁻⁵, 10⁻², 10⁻¹, 1, 10, or 100 mg of magnetosomes per mL. This behavior is interesting since it the opposite to that observed when the SAR is measured by applying an alternating magnetic field.

In still another embodiment of the invention, the SAR of the magnetosomes does not vary or decrease by more than 10⁵, 500, 90, 70, 50, 25, 10, 5, or 2 % between different magnetosome concentrations, where this percentage can represent C₂-C₁/C₁, where C₁ and C₂ are two different magnetosome concentrations. In some cases, the situation can occur when the magnetosome concentration is between 10⁻⁶ and 10⁶, 10⁻⁵ and 10⁵, 10⁻³ and 10³, or between 10⁻² and 10² mg per mL or mg per cm³ of body part. In some other cases, this situation occurs when the magnetosome concentration is lower than 1000, 100, 10, 1, 0.1, or 0.01 mg per mL or mg per cm³ of body part. In still some other cases, the situation occurs when the magnetosome concentration is larger than 1000, 100, 10, 1, 0.1, or 0.01 mg per mL or mg per cm³ of body part.

In one embodiment of the invention, the specific absorption rate (SAR) of the magnetosomes, designated as SARᵣₑₐₗ, is the specific absorption rate of the magnetosomes comprised, mixed or inserted in the body part. It can be expressed in a power unit such as Watt divided by a mass unit such as gram or in a power unit divided by a length, surface area, or volume unit such as cm, cm², or cm³. SAR₍ᵣₑₐₗ₎ is preferentially measured under the application of a radiation that produces a temperature increase preferentially in the presence of the magnetosomes. This radiation can be an acoustic wave, preferentially of power or power density larger than 10⁻⁹, 10⁻⁵, 10⁻³, 10⁻¹, 1, 10, or 10³ W/cm, W/cm², or W/cm³, preferentially of frequency larger than 10⁻⁶, 10⁻³, 10⁻¹, 1, 10, 10³, or 10⁶ MHz, an alternating magnetic field, preferentially of frequency larger than 10⁻⁹, 10⁻⁶, 10⁻³, 1, 10³, 10⁶, or 10⁹ kHz, preferentially of strength larger than 10⁻⁹, 10⁻⁶, 10⁻³, 10⁻¹, 1, 10, 10³, or 10⁶ mT, or a laser, preferentially of power or power density larger than 10⁻⁹, 10⁻⁵, 10⁻³, 10⁻¹, 1, 10, or 10³ W/cm, W/cm², or W/cm³. SAR₍ᵣₑₐₗ₎ is preferentially measured in adiabatic conditions or in conditions in which heat exchanges are minimized, preferentially between: i), the portion of the body part comprising the magnetosomes and the portion of the body part not comprising the magnetosomes or the region outside the body part comprising the magnetosomes, or ii), the container or tube containing the magnetosomes and the exterior of this container or tube. Heat exchanges are minimized when they produce a temperature decrease of less than 75, 60, 50, 25, 10, 5, 2, 1, or 0.1 °C. In some cases, SAR₍ᵣₑₐₗ₎ can be proportional to the difference between the initial slope of the temperature variation with time of the body part, medium, water, tissue comprising the magnetosomes, (ΔT/δt)_{(M)}, minus the initial slope of the temperature variation with time of body part, medium, water, tissue, not comprising the magnetosomes, (ΔT/δt)_{(WM)}, where (ΔT/δt)_{(M)} and (ΔT/δt)_{(WM)} are preferentially estimated in °C/sec. SAR₍ᵣₑₐₗ₎ is preferentially estimated using to the formula: SAR₍ᵣₑₐₗ₎=αᵣₑₐₗ[(ΔT/δt)_{(M)}-(ΔT/δt)_{(WM)}], where αᵣₑₐₗ is a proportionality coefficient. In some cases, SARᵣₑₐₗ=[(ΔT/δt)_{(M)}-(ΔT/δt)_{(WM})].Cᵥ/C_{mag}, where Cᵥ is the specific heat capacity, preferentially of the body part, tissue, water, medium comprising the magnetosomes, and C_{mag} is the magnetosome concentration or quantity or number of magnetosomes preferentially comprised in the body part.

In one embodiment of the invention, SAR_{(M)} is the specific absorption rate of the magnetosomes estimated without subtracting (ΔT/δt)_{(WM)} to (ΔT/δt)_{(M)}. In some cases, it can be proportional to the initial slope of the temperature variation with time of the magnetosomes comprised in the body part, (ΔT/δt)_{(M)}, preferentially estimated in °C/sec, preferentially leading to the formula: SAR_{(M)}=α_{M}.(ΔT/δt)_{(M)}, where α_{M} is a proportionality coefficient. In some other cases, SAR_{M}=(ΔT/δt)_{(M)}.Cᵥ/C_{mag}, where Cᵥ is the specific heat capacity, preferentially of the body part, tissue, water, medium comprising the magnetosomes, and C_{mag} is the magnetosome concentration or quantity or number of magnetosomes preferentially comprised in the body part.

In another embodiment of the invention, the SAR measured by applying the acoustic wave on the magnetosomes, designated as SAR_{AW}, is different from the SAR measured by applying an alternating magnetic field, designated as SAR_{AMF}. In some cases SAR_{AW} differs from SAR_{AMF} by at least 10⁻⁹, 10⁻⁷, 10⁻⁵, 10⁻³, 10⁻¹, 1, 10, 10³, 10⁵, 10⁷, or 10⁹ %, where this percentage can be equal to (SAR_{AW}-SAR_{AMF})/SAR_{AW}, where this percentage is preferentially measured for a given magnetosome concentration. In some cases, the magnetosome concentration is comprised between 10⁻⁹ and 10⁹, 10⁻⁷ and 10⁷, 10⁻⁵ and 10⁵, 10⁻³ and 10³, or between 10⁻¹ and 10 mg of magnetosomes per mL or between 10⁻¹ and 10 mg of magnetosomes per cm³ of body part. In some other cases, the magnetosome concentration is lower than 10⁹, 10⁷, 10⁵, 10³, 10, 1, 10⁻³, 10⁻⁵, 10⁻⁷, or 10⁻⁹ mg of magnetosomes per mL or mg of magnetosomes per cm³ of body part. In still some other cases, the magnetosome concentration is larger than 10⁹, 10⁷, 10⁵, 10³, 10, 1, 10⁻³, 10⁻⁵, 10⁻⁷, or 10⁻⁹ mg of magnetosomes per mL or 10⁻⁹ mg of magnetosomes per cm³ of body part.

In this invention, a mL can represent a mL of a liquid, solid, or gas, preferentially surrounding the magnetosomes. In some cases, it can be equivalent to a cm³., preferentially of body part.

In one embodiment of the invention, the SAR is not measured in the presence of a magnetic field, preferentially an alternating magnetic field, preferentially applied on magnetosomes. In some cases, the SAR is preferentially not due to hysteresis losses, or to Brownian motion, or to Neel relaxation, or to a movement of the magnetosomes under the application of the acoustic wave, or to the inversion of the magnetic moment of the magnetosomes under the application of the acoustic wave, or to a coupling of the magnetosome magnetic moment with the acoustic wave.

In another embodiment of the invention, SAR_{AW} is not due or not only due or not mainly due to Brownian motion, Neel relaxation or hysteresis losses. In some cases, SAR_{AW} is due, preferentially partly or mainly, to the absorption of the acoustic wave by the magnetosome(s) or body part comprising the magnetosomes. In some cases, SAR_{AW} is due, preferentially partly or mainly, to the absorption of the acoustic wave by the body part without or not comprising the magnetosomes, preferentially followed by heat diffusion between the body part without or not comprising the magnetosomes and the body part with or comprising the magnetosomes.

In one embodiment of the invention, the initial slope of the temperature variation with time of the magnetosomes, (ΔT/δt)ᵣₑₐₗ, is the initial slope of the temperature variation with time of the magnetosomes comprised, mixed or inserted in a body part, designated as (ΔT/δt)_{(M)}, minus the initial slope of the temperature variation with time of the body part without the magnetosomes, designated as (ΔT/δt)_{(WM)}, (ΔT/δt)ᵣₑₐₗ = (ΔT/δt)_{(M)} - (ΔT/δt)_{(WM)}. These initial slopes can be the slopes of the temperature variation with time measured: i), during the first 10⁻⁹, 10⁻⁶, 10⁻³, 10⁻¹, 1, 10, 10³, or 10⁵ minute(s) of the application of the acoustic wave, ii), when the temperature varies linearly with time, iii), before the saturating temperature has been reached, iv), during an initial time of heating or heating step, which represents less than 1, 5, 10, 25, 50, 75, 80, 90, 95, or 99% of the total duration of heating or heating step.

In another embodiment of the invention, the values of SAR_{(M)} and/or SARᵣₑₐₗ is(are) larger than 10⁻⁹, 10⁻⁶, 10⁻³, 1, 10³, 10⁶, or 10⁹ Watt as measured per gram of magnetosomes (W/g_{mag}), per cm³ of magnetosomes (W/cm³_{mag}), per gram of body part (W/g_{bp}), or per cm³ of body part (W/cm³_{bp}).

In another embodiment of the invention, the values of SAR_{(M)} and/or SARᵣₑₐₗ is(are) lower than 10⁻⁹, 10⁻⁶, 10⁻³, 1, 10³, 10⁶, or 10⁹ Watt as measured per gram of magnetosomes (W/g_{mag}), per cm³ of magnetosomes (W/cm³_{mag}), per gram of body part (W/g_{bp}), or per cm³ of body part (W/cm³_{bp}).

In another embodiment of the invention, a suitable range of values for SAR_{(M)} is between 37 W/g and 3124 W/g. The minimum value of this range (37 W/g) was estimated by applying an acoustic wave of 0.5 W/cm² to 45 µg of magnetosomes per cm³ of tissue. In some cases, this minimum value can be decreased by a factor of more than 1.5, 2, 5, 10, 50, 10², 10⁵, 10⁷, 10⁹, or 10²⁰ by decreasing the intensity, power, or frequency of the acoustic wave by a factor of more than 1.5, 2, 5, 10, 50, 10², 10⁵, 10⁷, 10⁹, or 10²⁰. The maximum value of this range (3124 W/g) was estimated by applying an acoustic wave of 1.5 W/cm² to a water solution comprising 100 µg of magnetosomes mixed in 100 µl of water. In some cases, this maximum value can be increased by a factor of more than 1.5, 2, 5, 10, 50, 10², 10⁵, 10⁷, 10⁹, or 10²⁰ by increasing the intensity, power, or frequency of the acoustic wave by a factor of more than 1.5, 2, 5, 10, 50, 10², 10⁵, 10⁷, 10⁹, or 10²⁰ or by using a body part that absorbs less the acoustic wave.

In another embodiment of the invention, a suitable range of values for SAR₍ᵣₑₐₗ₎ is between 5 W/g and 427 W/g. The minimum value of this range (5 W/g) was estimated by applying an acoustic wave of 1 W/cm² to 45 µg of magnetosomes per cm³ of tissue. In some cases, this minimum value can be decreased by a factor of more than 1.5, 2, 5, 10, 50, 10², 10⁵, 10⁷, 10⁹, or 10²⁰ by decreasing the intensity, power, or frequency of the acoustic wave by a factor of more than 1.5, 2, 5, 10, 50, 10², 10⁵, 10⁷, 10⁹, or 10²⁰. The maximum value of this range (427 W/g) was estimated by applying an acoustic wave of 1 W/cm² to a water solution comprising 100 µg of magnetosomes mixed in 100 µl of water. In some cases, this maximum value can be increased by a factor of more than 1.5, 2, 5, 10, 50, 10², 10⁵, 10⁷, 10⁹, or 10²⁰ by increasing the intensity, power, or frequency of the acoustic wave by a factor of more than 1.5, 2, 5, 10, 50, 10², 10⁵, 10⁷, 10⁹, or 10²⁰ or by using a body part that absorbs less the acoustic wave.

In still another embodiment of the invention, the value(s) of α_{M} and/or α_{WM} is(are) smaller than 10⁻⁹, 10⁻⁶, 10⁻³, 1, 10³, 10⁶, or 10⁹ (sec/°C).(W/g_{mag}) or (sec/°C).(W/g_{bp}) or (sec/°C).(W/cm³_{bp}) or (sec/°C).(W/cm³_{mag}).

In still another embodiment of the invention, the value(s) of α_{M} and/or α_{WM} is(are) larger than 10⁻⁹, 10⁻⁶, 10⁻³, 1, 10³, 10⁶, or 10⁹ (sec/°C).(W/g_{mag}) or (sec/°C).(W/g_{bp}) or (sec/°C).(W/cm³_{bp}) or (sec/°C).(W/cm³_{mag}).

The invention also relates to magnetosomes for use in an acoustic wave medical treatment of a body part of an individual, wherein the magnetosomes are administered to the body part and produce a slope of the initial variation of temperature with time, which is larger than 10⁻⁹ °C per second per gram of magnetosome.

In one embodiment of the invention, the values of (ΔT/δt)_{(M)}, (ΔT/δt)_{(WM)} and/or (ΔT/δt)₍ᵣₑₐₗ₎ are larger than 10⁻⁹, 10⁻⁶, 10⁻³, 10⁻¹, 1, 10³, 10⁶, or 10⁹ °C/sec, or °C/sec as measured per gram of magnetosome, per gram of body part, per cm³ of magnetosome, per cm² of magnetosome, per cm of magnetosome, per cm³ of body part, per cm² of body part, or per cm of body part In another embodiment of the invention, the values of (ΔT/δt)_{(M)}, (ΔT/δt)_{(WM)} and/or (ΔT/δt)₍ᵣₑₐₗ₎ are lower than 10⁻⁹, 10⁻⁶, 10⁻³, 10⁻¹, 1, 10³, 10⁶, or 10⁹ °C/sec, or °C/sec as measured per gram of magnetosome, per gram of body part, per cm³ of magnetosome, per cm² of magnetosome, per cm of magnetosome, per cm³ of body part, per cm² of body part, or per cm of body part.

In one embodiment of the invention, a suitable range of values for (ΔT/δt)_{(M)} is between 0.09 °C/sec and 0.7 °C/sec. The minimum value of this range (0.09 °C/sec) was estimated by exposing 45 µg of magnetosomes per cm³ of tissue to an acoustic wave of 0.5 W/cm². In some cases, this minimum value can be decreased by a factor of more than 1.5, 2, 5, 10, 50, 10², 10⁵, 10⁷, 10⁹, or 10²⁰, preferentially by decreasing the magnetosome concentration by a factor of more than 1.5, 2, 5, 10, 50, 10², 10⁵, 10⁷, 10⁹, or 10²⁰ or by decreasing the intensity, power, or frequency of the acoustic wave by a factor of more than 1.5, 2, 5, 10, 50, 10², 10⁵, 10⁷, 10⁹, or 10²⁰. The maximum value of this range (0.7 °C/sec) was estimated by exposing 100 µg of magnetosomes mixed in 100 µL of water to an acoustic wave of 1.5 W/cm². In some cases, this maximum value can be increased by a factor of more than 1.5, 2, 5, 10, 50, 10², 10⁵, 10⁷, 10⁹, or 10²⁰, preferentially by increasing the magnetosome concentration by a factor of more than 1.5, 2, 5, 10, 50, 10², 10⁵, 10⁷, 10⁹, or 10²⁰ or by increasing the intensity, power, or frequency of the acoustic wave by a factor of more than 1.5, 2, 5, 10, 50, 10², 10⁵, 10⁷, 10⁹, or 10²⁰.

In another embodiment of the invention, a suitable range of values for (ΔT/δt)_{(WM)} is between 0.063 °C/sec and 0.645 °C/sec. The minimum value of this range (0.063 °C/sec) was estimated by applying an acoustic wave of 0.5 W/cm² to a piece of tissue. In some cases, this minimum value can be decreased by a factor of more than 1.5, 2, 5, 10, 50, 10², 10⁵, 10⁷, 10⁹, or 10²⁰ by decreasing the intensity, power, or frequency of the acoustic wave by a factor of more than 1.5, 2, 5, 10, 50, 10², 10⁵, 10⁷, 10⁹, or 10²⁰. The maximum value of this range (0.645 °C/sec) was estimated by applying an acoustic wave of 1.5 W/cm² to a water solution. In some cases, this maximum value can be increased by a factor of more than 1.5, 2, 5, 10, 50, 10², 10⁵, 10⁷, 10⁹, or 10²⁰ by increasing the intensity, power, or frequency of the acoustic wave by a factor of more than 1.5, 2, 5, 10, 50, 10², 10⁵, 10⁷, 10⁹, or 10²⁰ or by using a body part that absorbs less the acoustic wave.

In one embodiment of the invention, the thermal conductivity or density of the body part, the velocity of the acoustic wave, attenuation of the acoustic wave, absorption of the acoustic wave, elasticity of the acoustic wave, or acoustic impedance of the acoustic wave, is at least 1.001, 1.01, 1.1, 1.2, 1.5, 2, 5, 10, 50, 10², 10³, or 10⁵ larger in the portion of the body part comprising the magnetosomes than in the portion of the body part without the magnetosomes. In one embodiment of the invention, the thermal conductivity or density of the body part, the velocity of the acoustic wave, attenuation of the acoustic wave, absorption of the acoustic wave, elasticity of the acoustic wave, or acoustic impedance of the acoustic wave, is at least 1.001, 1.01, 1.1, 1.2, 1.5, 2, 5, 10, 50, 10², 10³, or 10⁵ lower in the portion of the body part comprising the magnetosomes than in the portion of the body part without the magnetosomes. In one embodiment of the invention, the portion of the body part comprising the magnetosomes is the magnetosome region or portion of the body part in which the magnetosome, magnetosome assembly, or magnetosome suspension is or has been administered.

In another embodiment of the invention, the portion of the body part without the magnetosomes is the portion of the body part in which the magnetosome, magnetosome assembly, or magnetosome suspension is not or has not been administered, for example the portion of the body part before magnetosome administration or outside of the magnetosome region.

In an embodiment of the invention, the thermal conductivity of the body part is at least 10²⁰, 10¹⁰, 10⁵, 10³, 10², 50, 10, 5, 2, 1, 10⁻¹, 10⁻², 10⁻³, 10⁻⁵, 10⁻¹⁰, or 10⁻²⁰ W/m.K (Watt/meter.kelvin) larger in the portion of the body part comprising the magnetosomes than in the portion of the body part without the magnetosomes.

In an embodiment of the invention, the thermal conductivity of the body part is at least 10²⁰, 10¹⁰, 10⁵, 10³, 10², 50, 10, 5, 2, 1, 10⁻¹, 10⁻², 10⁻³, 10⁻⁵, 10⁻¹⁰, or 10⁻²⁰ W/m.K (Watt/meter.kelvin) lower in the portion of the body part comprising the magnetosomes than in the portion of the body part without the magnetosomes.

In an embodiment of the invention, the density of the body part is at least 10⁻⁹, 10⁻⁶, 10⁻³, 10⁻¹, 1, 2, 5, 10, 10³, or 10⁶ g/cm³ larger in the portion of the body part comprising the magnetosomes than in the portion of the body part without the magnetosomes.

In an embodiment of the invention, the density of the body part is at least 10⁻⁹, 10⁻⁶, 10⁻³, 10⁻¹, 1, 2, 5, 10, 10³, or 10⁶ g/cm³ lower in the portion of the body part comprising the magnetosomes than in the portion of the body part without the magnetosomes.

In an embodiment of the invention, the velocity of the acoustic wave is at least 10⁻⁵, 10⁻³, 10⁻¹, 1, 10, 100, 1000, 1500, 2000, 3000, 5000, 10⁴, or 10⁶ m/s larger in the portion of the body part comprising the magnetosomes than in the portion of the body part without the magnetosomes. In an embodiment of the invention, the velocity of the acoustic wave is at least 10⁻⁵, 10⁻³, 10⁻¹, 1, 10, 100, 1000, 1500, 2000, 3000, 5000, 10⁴, or 10⁶ m/s lower in the portion of the body part comprising the magnetosomes than in the portion of the body part without the magnetosomes. In an embodiment of the invention, the attenuation of the acoustic wave is at least 10⁻⁵, 10⁻³, 10⁻¹, 1, 10, 100, 10³ , or 10⁵ dB/cm larger in the portion of the body part comprising the magnetosomes than in the portion of the body part without the magnetosomes.

In an embodiment of the invention, the attenuation of the acoustic wave is at least 10⁻⁵, 10⁻³, 10⁻¹, 1, 10, 100, 10³, or 10⁵ dB/cm lower in the portion of the body part comprising the magnetosomes than in the portion of the body part without the magnetosomes.

In an embodiment of the invention, the acoustic impedance of the acoustic wave is at least 10⁻³, 10⁻², 10⁻¹, 0.5, 1, 1.5, 2, 5, 10, 10², 10⁴, 10⁶, 10⁹, or 10²⁰ MRayl or Kg.m⁻²s⁻¹ larger in the portion of the body part comprising the magnetosomes than in the portion of the body part without the magnetosomes.

In an embodiment of the invention, the acoustic impedance of the acoustic wave is at least 10⁻³, 10⁻², 10⁻¹, 0.5, 1, 1.5, 2, 5, 10, 10², 10⁴, 10⁶, 10⁹, or 10²⁰ MRayl or Kg.m⁻²s⁻¹ lower in the portion of the body part comprising the magnetosomes than in the portion of the body part without the magnetosomes.

In another embodiment of the invention, the increase, decrease, or variation of thermal conductivity or density of the body part, the velocity of the acoustic wave, attenuation of the acoustic wave, absorption of the acoustic wave, elasticity of the acoustic wave, or acoustic impedance of the acoustic wave, between the portion of the body part without the magnetosomes and the portion of the body part with the magnetosomes, is due to at least one of the following properties: i), a magnetosome concentration in the body part that is larger than 10⁻⁹, 10⁻⁷, 10⁻⁵, 10⁻³, 10⁻¹, 1, 10, 10³, 10⁵, 10⁷, or 10⁹ mg per mm³ or per cm³ of body part, ii), a magnetosome size that is larger than 10⁻⁶, 10⁻³, 10⁻¹, 1, 20, 50, 10³, or 10⁶ nm, iii), a magnetosome arrangement in chains, or iv), magnetosomes forming aggregates.

In another embodiment of the invention, the increase, decrease, or variation of thermal conductivity or density of the body part, the velocity of the acoustic wave, attenuation of the acoustic wave, absorption of the acoustic wave, elasticity of the acoustic wave, or acoustic impedance of the acoustic wave, between the body part without the magnetosomes and the body part with the magnetosomes, is due to at least one of the following properties: i), a magnetosomes concentration in the body part that is lower than 10⁻⁹, 10⁻⁷, 10⁻⁵, 10⁻³, 10⁻¹, 1, 10, 10³, 10⁵, 10⁷, or 10⁹ mg per mm³ or per cm³ of body part, ii), a magnetosomes size that is lower than 10⁻⁶, 10⁻³, 10⁻¹, 1, 20, 50, 10³, or 10⁶ nm.

The invention also relates to magnetosomes for use according to the invention, wherein the acoustic wave intensity is lower than 1000 W/cm².

In an embodiment of the invention, the acoustic wave has a low energy. In this case, the energy or energy density of the acoustic wave is preferentially lower than 10⁹, 10⁶, 10³, 100, 10, 1, 10⁻¹, 10⁻², or 10⁻⁵ W.sec, W.sec/cm, W.sec/cm², or W.sec/cm³. The acoustic wave can have a low energy when the power of the acoustic wave is low and/or when the time of application of the acoustic is short, preferentially shorter than 24, 12, 6, 3, 2, or 1 hour, or shorter than 50, 30, 15, 10, 5, 2, or 1 minute(s), or shorter than 50, 30, 20, 10, 5, 2, 1, 10⁻¹, 10⁻³, 10⁻⁶, or 10⁻⁹ second(s).

In one embodiment of the invention, the acoustic wave has a low power. In this case, the power of the acoustic wave is preferentially lower than 10⁹, 10⁶, 10³, 100, 10, 1, 10⁻¹, 10⁻², or 10⁻⁵ W.

In one embodiment of the invention, the acoustic wave has a low power density. In this case, the power density of the acoustic wave is preferentially lower than 10⁹, 10⁶, 10³, 100, 10, 1, 10⁻¹, 10⁻², or 10⁻⁵ W/cm, W/cm², or W/cm³.

The invention also relates to the use of acoustic wave of low intensity. In this case, the acoustic wave intensity is preferentially lower than 100, 10, or 1 W/cm².

In some cases, the acoustic wave intensity can be lower than 10⁹, 10⁵, 100, 10, 1, 10⁻¹, 10⁻², 10⁻³, 10⁻⁵, or 10⁻⁷ W/cm².

In some other cases, the acoustic wave intensity can be larger than 10⁹, 10⁵, 100, 10, 1, 10⁻¹, 10⁻², 10⁻³, 10⁻⁵, or 10⁻⁷ W/cm².

In still some other cases, the acoustic wave intensity can be between 10⁻⁵ and 10⁵, or between 10⁻³ and 10³, or between 10⁻¹ and 10 W/cm².

The invention also relates to magnetosomes for use according to the invention, wherein the acoustic wave frequency is lower than 100 MHz. In some cases, the acoustic wave frequency can be lower than 10⁵, 10³, 10², 10, 1, 10⁻¹, 10⁻², 10⁻³, 10⁻⁴, 10⁻⁵, 10⁻⁶, 10⁻⁷, 10⁻⁸, 10⁻¹⁰, or 10⁻¹⁵ MHz. In one embodiment of the invention, the acoustic wave frequency is larger than 10⁵, 10³, 10², 10, 1, 10⁻¹, 10⁻², 10⁻³, 10⁻⁴, 10⁻⁵, 10⁻⁶, 10⁻⁷, 10⁻⁸, 10⁻¹⁰, or 10⁻¹⁵ MHz.

In still another embodiment of the invention, the acoustic wave frequency is between 10⁻¹⁵ and 10¹⁵, 10⁻¹³ and 10¹³, 10⁻¹¹ and 10¹¹, 10⁻⁹ and 10⁹, 10⁻⁷ and 10⁷, 10⁻⁵ and 10⁵, 10⁻³ and 10³, or between 10⁻¹ and 10 MHz.

The invention also relates to magnetosomes for use according to the invention, wherein the acoustic wave is an ultrasound.

In some cases, the acoustic wave can be an infrasound, a sound, or a hypersound.

In one embodiment of the invention, the acoustic wave can induce a movement or vibration of the magnetosomes. This is preferentially the case when the mass of the magnetosomes is lower than 10²⁰, 10¹⁰, 10⁵, 10³, 10², 1, 10⁻³, or 10⁻⁵ µg per magnetosome. In this case, the acoustic wave can induce either a larger or lower movement or vibration for the magnetosomes than for the other substances, atoms, ions, which are not arranged or assembled in magnetosomes, and preferentially surround the magnetosomes.

In another embodiment of the invention, the acoustic wave can be absorbed by the magnetosomes. In this case, the acoustic wave energy is preferentially absorbed more importantly by the magnetosomes than by the other substances, atoms, ions, which are not arranged or assembled in magnetosomes or do not consist in magnetosomes, and preferentially surround the magnetosomes or preferentially belong to the body part. In some cases, the acoustic wave energy is absorbed more importantly by the magnetosomes than by the other substances when it is absorbed at least 1.1, 2, 5, 10, 25, 50, or 100 times more by the magnetosomes than by the other substances or when it results in a temperature increase that is at least 10⁻⁹, 10⁻⁶, 10⁻³, 1, 2, 5, 10, or 20 °C larger for the magnetosomes or in the magnetosome region than for the other substances or regions outside the magnetosome region.

In one embodiment of the invention, the acoustic wave intensity, power, energy, or frequency is the acoustic wave intensity or frequency generated by an apparatus generating acoustic waves, or by a transducer. It can be the acoustic wave intensity, power, energy, or frequency measured just after the acoustic wave has left the apparatus generating the acoustic wave or transducer. It can also be the acoustic wave intensity, power, energy, or frequency measured after the acoustic wave has left the apparatus generating the acoustic wave or transducer and travelled through another medium (liquid, gas, solid), such as the body part.

The invention also relates to magnetosomes for use according to the invention, wherein the magnetosomes are administered to the body part and the body part is sequentially exposed to the acoustic wave.

The invention also relates to magnetosomes for use according to the invention, wherein the acoustic wave is sequentially applied on the magnetosomes.

In one embodiment of the invention, the acoustic wave is applied sequentially. A sequence can correspond to or be the application of the acoustic wave during a time t₁ followed by the non-application of the acoustic wave during a time t₂. A sequence can also correspond to or be the application of an acoustic wave during a time t₁ followed by the application of another acoustic wave during a time t₃, wherein the intensity, power, energy, or frequency of the acoustic wave applied during the time t₃ is lower than the intensity, power, energy, or frequency of the acoustic wave applied during the time t₁.

In one embodiment of the invention, the time t₁ is the duration of a heating step.

In another embodiment of the invention, the time t₁ is the duration of the dissociation step, where the dissociation step is the step during which the compound dissociates from the magnetosomes. In some cases, the compound dissociates from the magnetosomes when more than 0.1, 1, 10, 25, 50, 75, or 90% of compounds are dissociated from the magnetosomes, where this percentage can correspond to the number of compounds not linked or not bound to the magnetosomes, preferentially following application of the acoustic wave, divided by the number of compounds linked or bound to the magnetosomes, preferentially before or without the application of the acoustic wave.

In another embodiment of the invention, the time t₂ or t₃ is the duration of a cooling step.

In another embodiment of the invention, the time t₂ or t₃ is the duration of the non-dissociation step, where the non-dissociation step is the step during which the compound does not dissociate from the magnetosomes. In some cases, the compound does not dissociate from the magnetosomes when less than 0.1, 1, 10, 25, 50, 75, or 90% of compounds are dissociated from the magnetosomes, where this percentage can correspond to the number of compounds not linked or not bound to the magnetosomes, preferentially following application of the acoustic wave, divided by the number of compounds linked or bound to the magnetosomes, preferentially before or without the application of the acoustic wave.

In some cases, the values of t₂ can be the same as the values of t₃.

In one embodiment of the invention, the time t₁, t₂, or t₃, is shorter than 10⁻³, 10⁻², 10⁻¹, 1, 10, 10², or 10³ minute(s). In some cases, t₁, t₂, or t₃, is shorter than 10⁻⁹, 10⁻⁷, 10⁻⁵, 10⁻³, 10⁻¹, 1, 10, 10³, 10⁵, 10⁷, or 10⁹ seconds.

In still another embodiment of the invention, the time t₁, t₂, or t₃, is longer than 10⁻³, 10⁻², 10⁻¹, 1, 10, 10², or 10³ minute(s). In some cases, t₁, t₂, or t₃, is longer than 10⁻⁹, 10⁻⁷, 10⁻⁵, 10⁻³, 10⁻¹, 1, 10, 10³, 10⁵, 10⁷, or 10⁹ seconds.

In one embodiment of the invention, the time t₁, t₂, or t₃, is shorter than the time of a pulse, preferentially by a factor of at least 1.1, 1.5, 2, 5, 10, 10², 10³, 10⁵, 10⁷, 10⁹, 10¹², 10¹⁵, or 10²⁰.

In still another embodiment of the invention, the time t₁, t₂, or t₃, is longer than the time of a pulse, preferentially by a factor of at least 1.1, 1.5, 2, 5, 10, 10², 10³, 10⁵, 10⁷, 10⁹, 10¹², 10¹⁵, or 10²⁰.

In still another embodiment of the invention, the pulse is defined as the application of the acoustic wave, preferentially the acoustic wave power, energy, or intensity, on the nanoparticle or body part during a time shorter than 10⁶, 10³, 1, 10⁻³, 10⁻⁶, or 10⁻⁹ seconds.

In one embodiment of the invention, the ratio t₁/t₂ or t₂/t₃ is smaller than 10⁻⁹, 10⁻⁶, 10⁻³, 10⁻¹, 1, 10, 10³, 10⁶, or 10⁹.

In still another embodiment of the invention, the ratio t₁/t₂ or t₂/t₃ is larger than 10⁻⁹, 10⁻⁶, 10⁻³, 10⁻¹, 1, 10, 10³, 10⁶, or 10⁹.

In one embodiment of the invention, the acoustic wave intensity, energy, power, or frequency applied during t₃ is at least 1.1, 1.3, 1.5, 2, 5, 10, 10², 10³, 10⁵, 10⁷, or 10⁹ lower than the acoustic wave intensity, energy, power, or frequency applied during t₁.

In an embodiment of the invention, a suitable range of values is between 0.2 and 0.43 minutes for t₁, and between 0.2 and 0.36 minutes for t₂. The minimum and maximum values of these ranges were estimated by exposing sequentially 500 µg of magnetosomes mixed in 100 µl of water to an acoustic wave of 1.5 W/cm² and frequency 3 MHz, as described in example 1(c), and by estimating the smallest and largest values of t₁ and t₂ values between two different sequences.

In another embodiment of this invention, the minimum of the range of t₁ or t₂ values can be decreased, for example by a factor of more than 1.5, 2, 5, 10, 10³, 10⁶, or 10⁹. This may be achieved by increasing the quantity of magnetosomes in the body part, for example by a factor of more than 1.5, 2, 5, 10, 10³, 10⁶, or 10⁹, by reducing magnetosome diffusion away from the body part or magnetosome degradation, between different sequences. This may also be achieved by increasing the power, intensity, or frequency of the acoustic wave, for example by a factor of more than 1.5, 2, 5, 10, 10³, 10⁶, or 10⁹. This may also be achieved by decreasing the absorption of the body part not comprising the magnetosomes, and preferentially by decreasing the temperature variation resulting from this absorption. For t₁ and t₂, this can be achieved by using an equipment, substance, preferentially different from the compound or magnetosomes, that heats the body part (to seek an effect on t₁) or cools down the body part (to seek the effect on t₂).

In another embodiment of this invention, the maximum of the range of t₁ or t₂ values can be increased, for example by a factor of more than 1.5, 2, 5, 10, 10³, 10⁶, or 10⁹. This may be achieved by decreasing the quantity of magnetosomes in the body part, for example by a factor of more than 1.5, 2, 5, 10, 10³, 10⁶, or 10⁹, by increasing magnetosome diffusion away from the body part or by increasing magnetosome degradation, between different sequences. This may also be achieved by decreasing the power, intensity, or frequency of the acoustic wave, for example by a factor of more than 1.5, 2, 5, 10, 10³, 10⁶, or 10⁹. This may also be achieved by increasing the absorption of the body part not comprising the magnetosomes, and preferentially by increasing the temperature variation resulting from this absorption. For t₁ and t₂, this can be achieved by using an equipment, substance, preferentially different from the compound or magnetosomes, that heats the body part (to seek an effect on t₁) or cools down the body part (to seek the effect on t₂).

In one embodiment of the invention, the acoustic wave intensity applied during t₁ or t₃ is lower than 10⁹, 10⁶, 10³, 100, 10, 1, 10⁻¹, 10⁻², or 10⁻⁵ W/cm².

In one embodiment of the invention, the acoustic wave power applied during t₁ or t₃ is lower than 10⁹, 10⁶, 10³, 100, 10, 1, 10⁻¹, 10⁻², or 10⁻⁵ W.

In one embodiment of the invention, the acoustic wave power density applied during t₁ or t₃ is lower than 10⁹, 10⁶, 10³, 100, 10, 1, 10⁻¹, 10⁻², or 10⁻⁵ W/cm, W/cm², or W/cm³.

In one embodiment of the invention, the acoustic wave energy or energy density applied during t₁ or t₃ is lower than 10⁹, 10⁶, 10³, 100, 10, 1, 10⁻¹, 10⁻², or 10⁻⁵ W.sec, W.sec/cm, W.sec/cm², or W.sec/cm³.

In one embodiment of the invention, the acoustic wave frequency applied during t₁ or t₃ is lower than 10³, 10, 1, 10⁻³, 10⁻⁶, 10⁻⁹, or 10⁻²⁰ GHz.

In one embodiment of the invention, a heating or dissociation step, preferentially of duration t₁, is repeated more than 1, 2, 3, 4, 5, 6, 7, 8, 9, 10, 250, 500, 10², 10³, 10⁵, 10⁷, or 10⁹ time(s). In one embodiment of the invention, a cooling or non-dissociation step, preferentially of duration t₂ or t₃, is repeated more than 1, 2, 3, 4, 5, 6, 7, 8, 9, 10, 250, 500, 10², 10³, 10⁵, 10⁷, or 10⁹ time(s).

In one embodiment of the invention, a sequence, preferentially of duration t₁+t₂ or t₁+t₃, is repeated more than 1, 2, 3, 4, 5, 6, 7, 8, 9, 10, 250, 500, 10², 10³, 10⁵, 10⁷, or 10⁹ time(s).

In some cases, the treatment can comprise more than 1, 2, 3, 4, 5, 6, 7, 8, 9, 10, 250, 500, 10², 10³, 10⁵, 10⁷, or 10⁹ sequence(s).

In some other cases, the treatment can comprise less than 1, 2, 3, 4, 5, 6, 7, 8, 9, 10, 250, 500, 10², 10³, 10⁵, 10⁷, or 10⁹ sequence(s).

In another embodiment of the invention, the magnetosomes remain in the body part during the treatment, preferentially during more than 1, 2, 5, 10, 20, 50, 100, 10³, or 10⁴ sequence(s), preferentially during more than 1, 2, 5, 10, 50, 100, or 10³ day(s).

In another embodiment of the invention, the magnetosomes remain in the body part during the treatment, preferentially during less than 1, 2, 5, 10, 20, 50, 100, 10³, or 10⁴ sequence(s), preferentially during less than 1, 2, 5, 10, 50, 100, or 10³ day(s).

In some cases, the magnetosomes remain in the body part during the treatment without decreasing in size by more than 1, 10, 20, 50, 100, 500, 10³, or 10⁴% compared with the size of the magnetosomes before administration in the body part or before the beginning of the treatment.

In one embodiment of the invention, the times t₁, t₂, or t₃ do not vary by more than 99.9, 99, 90, 75, 50, 25, 10, or 10⁻¹% between two sequences. In some cases, this percentage of variation can be estimated as the absolute value of (t₁-t₁')/t₁, (t₂-t₂')/t₂, or as (t₃-t₃')/t₃, where t₁ and t₁', t₂ and t₂', or t₃ and t₃', are the durations of two different heating/dissociating or cooling/non-dissociating steps belonging to two different sequences.

In one embodiment of the invention, a suitable range of percentages of (t₁-t₁')/t₁ is between 5% and 43% and a suitable range of percentages of (t₂-t₂')/t₂ is between 5% and 44%. The minimum and maximum values of these ranges were estimated by exposing sequentially 500 µg of magnetosomes mixed in 100 µl of water to an acoustic wave of 1.5 W/cm² and frequency 3 MHz, as described in example 1(c), and by estimating the smallest and largest variations in t₁ and t₂ values between two different sequences.

In one embodiment of the invention, the durations (t₁ + t₂) or (t₁ + t₃) do not vary by more than 99.9, 99, 90, 75, 50, 25, 10, or 10⁻¹% between two different sequences. In some cases, this percentage of variation can be estimated as the absolute value of [(t₁+t₂)-(t₁+t₂)']/(t₁+t₂), or [(t₁+t₃)-(t₁+t₃)']/(t₁+t₃), where (t₁+t₂) and (t₁+t₂)', or (t₁+t₃) and (t₁+t₃)', are the duration of two different sequences.

In one embodiment of the invention, a suitable range of percentages of [(t₁+t₂)-(t₁+t₂)']/(t₁+t₂) is between 2% and 35%. The minimum and maximum values of these ranges were estimated by exposing sequentially 500 µg of magnetosomes mixed in 100 µl of water to an acoustic wave of 1.5 W/cm² and frequency 3 MHz, as described in example 1(c), and by estimating the smallest and largest variations in (t₁+t₂) values between two different sequences.

In another embodiment of this invention, the minimum of the range given by the values (t₁-t₁')/t₁, (t₂-t₂')/t₂, [(t₁+t₂)-(t₁+t₂)']/(t₁+t₂), can be decreased, for example by a factor of more than 1.5, 2, 5, 10, 10³, 10⁶, or 10⁹. This may be achieved by increasing the quantity of magnetosomes in the body part, for example by a factor of more than 1.5, 2, 5, 10, 10³, 10⁶, or 10⁹, by decreasing magnetosome diffusion away from the body part or magnetosome degradation, between different sequences. This may also be achieved by increasing the power, intensity, or frequency of the acoustic wave, for example by a factor of more than 1.5, 2, 5, 10, 10³, 10⁶, or 10⁹. This may also be achieved by decreasing the absorption of the body part not comprising the magnetosomes, and preferentially by decreasing the temperature increase resulting from this absorption.

In another embodiment of this invention, the maximum of the range given by the values of (t₁-t₁')/t₁, (t₂-t₂')/t₂, [(t₁+t₂)-(t₁+t₂)']/(t₁+t₂), can be increased, for example up to 50, 70, 80, 90, 95, 99, or 99.9%. This may be achieved by decreasing the quantity of magnetosomes, for example by a factor of more than 1.5, 2, 5, 10, 10³, 10⁶, or 10⁹, by decreasing magnetosome diffusion away from the body part or magnetosome degradation, between different sequences. This may also be achieved by decreasing the power, intensity, or frequency of the acoustic wave, for example by a factor of more than 1.5, 2, 5, 10, 10³, 10⁶, or 10⁹. This may also be achieved by increasing the absorption of the body part not comprising the magnetosomes, and preferentially by increasing the temperature increase resulting from this absorption. In one embodiment of the invention, the time t₁, t₂, or t₃ is chosen to reach a desired parameter, which preferentially makes the treatment efficient. For example, it is chosen to reach: i), a desired temperature, ii), a desired percentage of dissociated compound, iii), a desired level of cavitation, iv), a desired level or concentration of free radicals or radical oxygen species (ROS), v), a desired level of apoptosis or desired number of apoptotic cells. In this case, the desired temperature, or desired level of cavitation, or desired level or concentration of free radicals or ROS, or desired level of apoptosis or desired number of apoptotic cells, are preferentially higher or larger during the time t₁ than during t₂ or t₃ by a factor, which is preferentially at least equal to 1, 1.2, 1.5, 2, 5, 10, 10², 10³, 10⁵, 10⁹, or 10²⁰.

In some cases, ROS or bubbles produced by cavitation can be associated with the compound. In some cases, the temperature can be the temperature measured at the macroscopic scale, *i.e.* the temperature preferentially measured at a scale larger than the size of 1, 10, 10³, 10⁵, or 10⁹ magnetosome(s) or at scale that is larger than the size of 1, 10, 10³, 10⁵, or 10⁹ magnetosome(s).

In some other cases, the temperature can be the temperature measured at the nanoscopic scale, *i.e.* the temperature preferentially measured at a scale that comprises less than 2, 5, 10, 10³, 10⁵, or 10⁹ magnetosomes or at a scale smaller than the size of 1, 2, 5, 10, 10³, 10⁵, or 10⁹ magnetosome(s).

In another embodiment of the invention, the temperature reached by applying the acoustic wave on the magnetosomes is preferentially larger than the physiological temperature by at least 10⁻⁵, 10⁻³, 10⁻¹, 1, 2, 3, 4, 5, 7, 10, 15, 20, 25, 30, 50, 100, 200, 500, 1000, or 5000 °C. It is preferentially the same as the desired temperature or differs from the desired temperature by less than 10⁻⁵, 10⁻³, 10⁻¹, 1, 2, 3, 4, 5, 7, 10, 15, 20, 25, 30, 50, 100, 200, 500, 1000, or 5000 °C, where the desired temperature is preferentially the temperature that one wants to reach during the treatment.

In another embodiment of the invention, the level of cavitation reached by applying the acoustic wave on the magnetosomes corresponds to a number of bubbles larger than 1, 10, 10³, or 10⁹, or to a size of bubbles larger than 0.1, 1, 10, 50, 100, 10³, or 10⁵ nm, or to a speed of bubbles larger than 0.1, 1, 10, 50, 100, 10³, or 10⁵ nm per second. It is preferentially the same as the desired level of cavitation or differs from the desired level of cavitation by less than 90, 70, 50, 10, 5, 2, or 1%, where the desired level of cavitation preferentially leads to the desired number, size, or speed of bubbles, or to the desired temperature resulting from cavitation, that one wishes to reach during treatment.

In some cases, the desired number of bubbles can be larger than 1, 2, 5, 10, 10³, 10⁶, 10⁹, or 10²⁰ bubble(s) per magnetosome or per assembly of magnetosomes. The desired size of bubbles can be larger than 10⁻³, 1, 10, 10², 10³, 10⁵, 10¹⁰, or 10²⁰ nm. The desired speed of cavitation can be larger than 10⁻³, 1, 10², 10³, 10⁵, 10⁷, or 10⁹ m/sec. The desired temperature resulting from cavitation can be 10⁻⁵, 10⁻³, 10⁻¹, 1, 2, 3, 4, 5, 7, 10, 15, 20, 25, 30, 10², 10³, or 10⁵ °C above physiological temperature. The desired level of free radicals or ROS preferentially leads to free radical or ROS concentration, which preferentially is, corresponds to, or leads to a H₂O₂ or free radical concentration, which is larger than 10²⁰, 10¹⁰, 10⁵, 10³, 10, 1, 10⁻³, or 10⁻⁵ nM of H₂O₂ or free radical per magnetosome or per assembly of magnetosomes or per cm³ or mm³ of body part. The desired level of apoptosis is preferentially the number of apoptotic cells, which is larger than 1, 5, 10, 10², 10³, 10⁶, 10⁹, or 10²⁰ or 10²⁰ cell(s) per mm³ or cm³ of body part. The desired level of apoptotic cells can also be more than 10⁻⁹, 10⁻⁵, 10⁻², 1, 5, 10, 10³, 10⁵, or 10⁹ % of the total number of pathological cells preferentially comprised in the body part.

In some other cases, the desired number of bubbles can be lower than 1, 2, 5, 10, 10³, 10⁶, 10⁹, or 10²⁰ bubble(s) per magnetosome or per assembly of magnetosomes. The desired size of bubbles can be lower than 10⁻³, 1, 10, 10², 10³, 10⁵, 10¹⁰, or 10²⁰ nm. The desired speed of cavitation can be lower than 10⁻³, 1, 10², 10³, 10⁵, 10⁷, or 10⁹ m/sec. The desired temperature resulting from cavitation can be 10⁻⁵, 10⁻³, 10⁻¹, 1, 2, 3, 4, 5, 7, 10, 15, 20, 25, 30, 10², 10³, or 10⁵ °C below physiological temperature. The desired level of free radicals or ROS preferentially leads to free radical or ROS concentration, which preferentially is, corresponds to, or leads to a H₂O₂ or free radical concentration, which is lower than 10²⁰, 10¹⁰, 10⁵, 10³, 10, 1, 10⁻³, or 10⁻⁵ nM of H₂O₂ or free radical per magnetosome or per assembly of magnetosomes or per cm³ or mm³ of body part. The desired level of apoptosis preferentially is the number of apoptotic cells, which is lower than 1, 5, 10, 10², 10³, 10⁶, 10⁹, or 10²⁰ or 10²⁰ cell(s) per mm³ or cm³ of body part. The desired level of apoptotic cells can also be less than 10⁻⁹, 10⁻⁵, 10⁻², 1, 5, 10, 10³, 10⁵, or 10⁹ % of the total number of pathological cells preferentially comprised in the body part.

In one embodiment of the invention, the desired temperature, percentage of dissociated compound, level of cavitation, level of free radicals or ROS, or level of apoptosis, is the temperature, percentage of dissociated compound, level of cavitation, level of free radicals of ROS, or level of apoptosis, reached during treatment, or reached during the time t₁, t₂, or t₃. It can also be a temperature, level of cavitation, level of free radicals or ROS, or level of apoptosis that does(do) not differ by more or less than 10⁻⁹, 10⁻⁵, 10⁻², 1, 5, 10, 10³, 10⁵, or 10⁹ % from the desired temperature, desired level of cavitation, desired level of free radicals or ROS, or desired level of apoptosis.

In one embodiment of the invention, cavitation leads to the formation or nucleation of bubbles originating from the magnetosomes. In this case, the size or number of bubbles can differ by a factor of more than 1.1, 1.2, 1.5, 2, 2.5, 5, 10, 10², 10³, or 10⁵, from the size or number of bubbles, which are not originating from the magnetosomes or are not produced by the magnetosomes or are produced in the absence of magnetosomes.

In one embodiment of the invention, cavitation leads to the formation or nucleation of bubbles originating from the magnetosomes. In this case, the size or number of bubbles can differ by a factor of less than 1.1, 1.2, 1.5, 2, 2.5, 5, 10, 10², 10³, or 10⁵, from the size or number of bubbles, which are not originating from the magnetosomes or are not produced by the magnetosomes or are produced in the absence of magnetosomes.

In one embodiment of the invention, heat, dissociation of the compound, or ROS produced by the acoustic wave, preferentially by the application of the acoustic waves on magnetosomes, is preferentially resulting from the implosion or destruction of cavitation bubbles, also preferentially designated as bubbles. In some cases, stable cavitation, which preferentially does not result in implosion or destruction of cavitation bubbles, can be distinguished from inertial cavitation, which preferentially results in implosion or destruction of cavitation bubbles.

In one embodiment of the invention, the acoustic wave, preferentially of low intensity, energy, or power, applies a pressure, preferentially on magnetosomes or on the body part, which is larger than 10⁻⁹, 10⁻⁵, 10⁻³, 10⁻¹, 1, 10, 10³, 10⁶, or 10⁹ MPa, preferentially per cm³ of body part.

In still another embodiment of the invention, the acoustic wave, preferentially of low intensity, energy, or power, applies a pressure, preferentially on magnetosomes or on the body part, which is lower than 10⁻⁹, 10⁻⁵, 10⁻³, 10⁻¹, 1, 10, 10³, 10⁶, or 10⁹ MPa, preferentially per cm³ of body part.

In one embodiment of the invention, magnetosomes are sonosensitizers, preferentially used in sonodynamic therapy.

In one embodiment of the invention, a sonosensitizer is defined as a substance that enhances the effect of acoustic waves, *i.e*. that increases the number of biological material(s), preferentially pathological cell(s), which is(are) dead, destroyed, denatured, or inactivated, preferentially by a factor of 1.2, 1.5, 2, 5, 10, 10³, or 10⁵ and/or that decreases the number of healthy cell(s), which is(are) dead, destroyed, denatured, or inactivated, preferentially by a factor of 1.2, 1.5, 2, 5, 10, 10³, or 10⁵, preferentially during medical treatment. A sonosensitizer can in some cases also be defined as a substance that leads to a temperature increase under the application of the acoustic wave, which is larger in the presence than in the absence of the sonosensitizers, *i.e.* a temperature increase that is preferentially 0.1, 1, 2, 5, 10, 20, or 50 °C larger in the presence than in the absence of the sonosensitizer, where the concentration of the sonosensitizer is preferentially larger than 10⁻⁹, 10⁻⁶, 10⁻³, 10⁻¹, 1, 10, 100, or 10³ mg per mL or 10³ mg per cm³ of body part. A sonosensitizer can in some other cases also be defined as a substance that leads to the dissociation of the compound under the application of the acoustic wave, which is larger in the presence than in the absence of the sonosensitizers, *i.e.* a percentage of dissociated compounds that is preferentially 0.1, 1, 2, 5, 10, 20, 50, 75, 80, or 90% larger in the presence than in the absence of the sonosensitizer, where the concentration of the sonosensitizer is preferentially larger than 10⁻⁹, 10⁻⁶, 10⁻³, 10⁻¹, 1, 10, 100, or 10³ mg per mL or 10³ mg per cm³ of body part.

In one embodiment of the invention, sonodynamic therapy is defined as a therapy, which triggers a therapeutic activity by: i), applying low intensity acoustic wave on the magnetosome or body part, or ii), by using the magnetosome as a sonosensitizer. In some cases, sonodynamic therapy is defined as a therapy, in which the temperature increase is lower than 10⁵, 10³, 500, 200, 100, 50, 25, 10, 5, 2, or 1 °C, and the percentage of dissociation of the compound is larger than 10⁻⁵, 10⁻², 10⁻¹, 1, 10, 10³, or 10⁷ %.

In one embodiment of the invention, the percentage of dissociation of compounds is defined as the ratio between the number or mass of compounds that have dissociated following the application of the acoustic wave divided by the number or mass compounds that are linked to the magnetosome before the application of the acoustic wave.

In some other cases, sonodynamic therapy is defined as a therapy, in which the temperature increase is larger than 10⁵, 10³, 500, 200, 100, 50, 25, 10, 5, 2, or 1 °C, and the percentage of dissociation of the compound is lower than 10⁻⁵, 10⁻², 10⁻¹, 1, 10, 10³, or 10⁷ %.

In some cases, the acoustic wave frequency, intensity, energy, or power, is sufficiently low to enable internalization of the magnetosomes in cells preferentially belonging to the body part. It can be sufficiently low to enable the internalization of more than 1, 10, 10³, 10⁶, 10⁹, or 10²⁰ magnetosome(s) per cell, or 10⁻⁶, 10⁻³, 10⁻¹, 1, 10, 10³, 10⁶, 10⁹, or 10²⁰ magnetosome(s) per cell or 10²⁰ magnetosome(s) per mm³ of body part or 10²⁰ mg of magnetosome(s) per mm³ of body part.

In some other cases, the acoustic wave frequency, intensity, energy, or power, is sufficiently large to enable internalization of the magnetosomes in cells preferentially belonging to the body part. It can be sufficiently large to enable the internalization of more than 1, 10, 10³, 10⁶, 10⁹, or 10²⁰ magnetosome(s) per cell, or 10⁻⁶, 10⁻³, 10⁻¹, 1, 10, 10³, 10⁶, 10⁹, or 10²⁰ magnetosome(s) on a basis per cell or per mm³ of body part, or 10⁻⁶, 10⁻³, 10⁻¹, 1, 10, 10³, 10⁶, 10⁹, or 10²⁰ mg of magnetosome(s) per mm³ of body part.

In one embodiment of the invention, the acoustic wave frequency, intensity, energy, or power, is sufficiently low to enable the destruction or growth inhibition of healthy or pathological cells preferentially belonging to the body part. It can be sufficiently low to enable the destruction or growth inhibition of more than 1, 10, 10³, 10⁶, 10⁹, or 10²⁰ cell(s).

In one embodiment of the invention, the acoustic wave frequency, intensity, energy, or power, is sufficiently large to enable the destruction or growth inhibition of healthy or pathological cells preferentially belonging to the body part. It can be sufficiently large to enable the destruction or growth inhibition of more than 1, 10, 10³, 10⁶, 10⁹, or 10²⁰ cell(s).

In some cases, the acoustic wave is applied on less than 10⁻⁶, 10⁻³, 10⁻¹, 1, 10, 10³, 10⁶, 10⁹, or 10²⁰ magnetosome(s) per cell or 10²⁰ magnetosomes per mm³ of body part or 10²⁰ mg of magnetosomes per mm³ of body part.

In some other cases, the acoustic wave is applied on more than 10⁻⁶, 10⁻³, 10⁻¹, 1, 10, 10³, 10⁶, 10⁹, or 10²⁰ magnetosome(s) on a basis per cell or per mm³ of body part, or 10⁻⁶, 10⁻³, 10⁻¹, 1, 10, 10³, 10⁶, 10⁹, or 10²⁰ mg of magnetosomes per mm³ of body part.

In one embodiment of the invention, the acoustic wave frequency, intensity, energy, or power, is sufficiently low to prevent internalization of the magnetosomes in healthy or pathological cells preferentially belonging to the body part. It can be sufficiently low to prevent the internalization of more than 10⁻⁶, 10⁻³, 10⁻¹, 1, 10, 10³, 10⁶, 10⁹, or 10²⁰ magnetosome(s) on a basis per cell or per mm³ of body part, or 10⁻⁶, 10⁻³, 10⁻¹, 1, 10, 10³, 10⁶, 10⁹, or 10²⁰ mg of magnetosomes per mm³ of body part.

In another embodiment of the invention, the acoustic wave frequency, intensity, energy, or power, is sufficiently large to prevent internalization of the magnetosomes in healthy or pathological cells preferentially belonging to the body part. It can be sufficiently large to prevent the internalization of more than 10⁻⁶, 10⁻³, 10⁻¹, 1, 10, 10³, 10⁶, 10⁹, or 10²⁰ magnetosome(s) on a basis per cell or per mm³ of body part, or 10⁻⁶, 10⁻³, 10⁻¹, 1, 10, 10³, 10⁶, 10⁹, or 10²⁰ mg of magnetosomes per mm³ of body part.

In one embodiment of the invention, the acoustic wave frequency, intensity, energy, or power, is sufficiently low to prevent the destruction or growth inhibition of healthy or pathological cells preferentially belonging to the body part. It can be sufficiently low to prevent the destruction or growth inhibition of more than 1, 10, 10³, 10⁶, 10⁹, or 10²⁰ cell(s).

In another embodiment of the invention, the acoustic wave frequency, intensity, energy, or power, is sufficiently large to prevent the destruction or growth inhibition of healthy or pathological cells preferentially belonging to the body part. It can be sufficiently large to prevent the destruction or growth inhibition of more than 1, 10, 10³, 10⁶, 10⁹, or 10²⁰ cell(s).The invention also relates to magnetosomes for use according to the invention, wherein the acoustic wave has a penetration depth in the body part, which is larger than 1 cm.

In some cases, the acoustic wave can have a penetration depth, which is larger than 0.001, 0.1, 1,5, 10, 20, 50, or 500 cm.

In some other cases, the acoustic wave has a penetration depth, which is lower than 0.001, 0.1, 1, 5, 10, 20, 50, or 500 cm.

The penetration depth is preferentially the penetration of the acoustic wave through or in the body part, or through or in the magnetosome region, or through or in 99, 75, 50, 25, 1, 0.1, 10⁻³, 10⁻⁹, or 10⁻²⁰ % of the body part, or through or in 99, 75, 50, 25, 1, 0.1, 10⁻³, 10⁻⁹, or 10⁻²⁰ % of the magnetosome region, or through or in the region separating the equipment generating the acoustic wave and the body part or magnetosome region.

The invention also relates to magnetosomes for use according to the invention, wherein the concentration of the administered magnetosomes is larger than 10⁻³ mg per mm³ of body part. In some cases, the magnetosome concentration is larger than 10⁻⁹, 10⁻⁷, 10⁻⁵, 10⁻³, 10⁻¹, 1, 10, 10³, 10⁵, 10⁷, or 10⁹ gram or milligram as measured per mm³ of body part, per mL of suspension, or per mm³ of matrix or body part (biological or not) comprising the magnetosomes.

The invention also relates to magnetosomes for use according to the invention, wherein the concentration of magnetosomes exposed to the acoustic wave is lower than 10 g of magnetosomes, preferentially in iron or iron oxide, per cm³ of body part.

In one embodiment of the invention, magnetosomes designate an assembly of more than 1, 10, 10², 10³, 10⁵, 10¹⁰, or 10⁵⁰ magnetosome(s), magnetosome(s) per cm³ , or magnetosome(s) per cm³ of body part. A large quantity of magnetosomes may be necessary to lead to an effect of the application of the acoustic wave on the magnetosomes, such as a temperature increase or the dissociation of the compound from the magnetosomes.

In another embodiment of the invention, magnetosomes designate an assembly of less than 2, 10, 10², 10³, 10⁵, 10¹⁰, or 10⁵⁰ magnetosomes, magnetosome(s) per cm³, or magnetosome(s) per cm³ of body part. A low quantity of magnetosomes may be necessary to prevent toxicity possibly arising at high magnetosome concentration.

In one embodiment of the invention, the magnetosome concentration is lower than 10⁵⁰, 10²⁰, 10¹⁰, 10⁵, 10², 10, 5, 2, or 1 magnetosome(s) on a basis per cm³ or per cm³ of body part.

In another embodiment of the invention, the magnetosome concentration is lower than 10⁵, 10³, 10, 1, 10⁻³, 10⁻⁶, or 10⁻⁹ g of magnetosome(s) on a basis per cm³ or per cm³ of body part. A low concentration of magnetosomes may be necessary to prevent toxicity possibly arising at high magnetosome concentration.

In one embodiment of the invention, the magnetosome concentration is larger than 10⁵⁰, 10²⁰, 10¹⁰, 10⁵, 10², 10, 5, 2, or 1 magnetosome(s) on a basis per cm³ or per cm³ of body part.

In another embodiment of the invention, the magnetosome concentration is larger than 10⁵, 10³, 10, 1, 10⁻³, 10⁻⁶, or 10⁻⁹ g of magnetosome(s) on a basis per cm³ or per cm³ of body part.

A large concentration of magnetosomes may be necessary to lead to an effect of the application of the acoustic wave on the magnetosomes, such as a temperature increase or the dissociation of the compound from the magnetosomes. In some cases, the magnetosome concentration is the concentration in at least one substance comprised in the magnetosome such as iron, oxide, iron oxide, or another metal than iron.

The invention also relates to magnetosomes for use according to the invention, wherein the application of the acoustic wave on the magnetosomes induces a temperature increase of the body part, which is larger than 0.01, 0.1, 1, 2, 5, 10, 30, 50, 100, or 1000 °C, where this temperature increase preferentially corresponds to ΔT_{real(M)} = ΔT_{(M)} - ΔT_{(WM)}, where ΔT_{(M)} and ΔT_{(WM)} are the temperature increases of the body part comprising the magnetosomes and of the body part not comprising the magnetosomes, respectively

In one embodiment of the invention, the acoustic wave frequency is thermal. In this case, the acoustic wave frequency is such that it does induce an increase in temperature or it induces an increase in temperature of more than 10³, 10², 10, 5, 2, 1, or 0.1 °C, wherein the increase in temperature is preferentially occurring or measured in the body part without the magnetosomes, or in the magnetosome region, or in the body part with the magnetosomes. The temperature increase can also occur in the body part without magnetosomes and in the magnetosome region or it can occur in the magnetosome region and not occur in the body part without the magnetosomes. In this case, the acoustic wave frequency is preferentially larger than 10⁻²⁰, 10⁻¹⁰, 10⁻⁵, 10⁻³, 10⁻¹, 1, 10, 10³, 10⁵, 10¹⁰, or 10²⁰ Hz.

The invention also relates to magnetosomes for use according to the invention, wherein the application of the acoustic wave on the magnetosomes induces a temperature increase of the body part, which is lower than 0.01, 0.1, 1, 2, 5, 10, 30, 50, 100, or 1000 °C, where this temperature increase preferentially corresponds to ΔT_{real(M)} = ΔT_{(M)} - ΔT_{(WM)}, where ΔT_{(M)} and ΔT_{(WM)} are the temperature increases of the body part comprising the magnetosomes and the body part not comprising the magnetosomes, respectively

In one embodiment of the invention, the acoustic wave frequency is non-thermal. In this case, the acoustic wave frequency is such that it doesn't induce an increase in temperature or it induces an increase in temperature of less than 10³, 10², 10, 5, 2, 1, or 0.1 °C, wherein the increase in temperature is preferentially occurring or measured in the body part not comprising the magnetosomes, or in the magnetosome region, or in the body part comprising the magnetosomes. The temperature increase can also occur in the magnetosome region and not occur in the body part not comprising the magnetosomes. In this case, the acoustic wave frequency is preferentially lower than 10⁻²⁰, 10⁻¹⁰, 10⁻⁵, 10⁻³, 10⁻¹, 1, 10, 10³, 10⁵, 10¹⁰, or 10²⁰ Hz.

In one embodiment of the invention, a suitable range of ΔT_{real(M)} is between 3 °C and 28 °C. The minimum value of this range (3 °C) was estimated by exposing 100 µg of magnetosomes mixed in 100 µl of water to an acoustic wave of 1.5 W/cm². In some cases, this minimum value can be decreased by a factor of more than 1.5, 2, 5, 10, 50, 10², 10⁵, 10⁷, 10⁹, or 10²⁰ by decreasing the intensity, power, or frequency of the acoustic wave by a factor of more than 1.5, 2, 5, 10, 50, 10², 10⁵, 10⁷, 10⁹, or 10²⁰ or by decreasing the magnetosome concentration in the body part by a factor of more than 1.5, 2, 5, 10, 50, 10², 10⁵, 10⁷, 10⁹, or 10²⁰. The maximum value of this range (28 °C) was estimated by exposing 45 µg of magnetosomes per cm³ of tissue to an acoustic wave of 1.5 W/cm². In some cases, this maximum value can be increased by a factor of more than 1.5, 2, 5, 10, 50, 10², 10⁵, 10⁷, 10⁹, or 10²⁰ by increasing the intensity, power, or frequency of the acoustic wave by a factor of more than 1.5, 2, 5, 10, 50, 10², 10⁵, 10⁷, 10⁹, or 10²⁰ or by increasing the magnetosome concentration in the body part by a factor of more than 1.5, 2, 5, 10, 50, 10², 10⁵, 10⁷, 10⁹, or 10²⁰.

In another embodiment of the invention, a suitable range of ΔT_{(M)} is between 18 °C and 56 °C. The minimum value of this range (18 °C) was estimated by exposing a suspension comprising 100 µg of magnetosomes in 100 µl of water to an acoustic wave of 0.5 W/cm². In some cases, this minimum value can be decreased by a factor of more than 1.5, 2, 5, 10, 50, 10², 10⁵, 10⁷, 10⁹, or 10²⁰ by decreasing the intensity, power, or frequency of the acoustic wave by a factor of more than 1.5, 2, 5, 10, 50, 10², 10⁵, 10⁷, 10⁹, or 10²⁰ or by decreasing the magnetosome concentration in the body part by a factor of more than 1.5, 2, 5, 10, 50, 10², 10⁵, 10⁷, 10⁹, or 10²⁰. The maximum value of this range (56 °C) was estimated by exposing 45 µg of magnetosomes per cm³ of tissue to an acoustic wave of 1.5 W/cm². In some cases, this maximum value can be increased by a factor of more than 1.5, 2, 5, 10, 50, 10², 10⁵, 10⁷, 10⁹, or 10²⁰ by increasing the intensity, power, or frequency of the acoustic wave by a factor of more than 1.5, 2, 5, 10, 50, 10², 10⁵, 10⁷, 10⁹, or 10²⁰ or by increasing the magnetosome concentration in the body part by a factor of more than 1.5, 2, 5, 10, 50, 10², 10⁵, 10⁷, 10⁹, or 10²⁰.

In still another embodiment of the invention, a suitable range of ΔT_{(WM)} is between 13 °C and 32 °C. The minimum value of this range (13 °C) was estimated by exposing a water solution to an acoustic wave of 0.5 W/cm². In some cases, this minimum value can be decreased by a factor of more than 1.5, 2, 5, 10, 50, 10², 10⁵, 10⁷, 10⁹, or 10²⁰ by decreasing the intensity, power, or frequency of the acoustic wave by a factor of more than 1.5, 2, 5, 10, 50, 10², 10⁵, 10⁷, 10⁹, or 10²⁰. The maximum of this range (32 °C) was estimated by exposing a tissue to an acoustic wave of 1 W/cm². In some cases, this maximum value can be increased by a factor of more than 1.5, 2, 5, 10, 50, 10², 10⁵, 10⁷, 10⁹, or 10²⁰ by increasing the intensity, power, or frequency of the acoustic wave by a factor of more than 1.5, 2, 5, 10, 50, 10², 10⁵, 10⁷, 10⁹. The invention also relates to magnetosomes for use according to the invention, wherein the compound according to the invention, preferentially to be administered to the body part, is attached to the magnetosomes.

Preferentially, the compound is attached to the magnetosomes in the absence of the application of the acoustic wave and dissociates from the magnetosomes under the application of the acoustic wave.

In some cases, the compound is attached to the magnetosomes when it is located at a distance of less than 10⁵, 10³, 100, or 10 nm from the magnetosomes, or when it is bound or linked to the magnetosomes, preferentially through Hydrogen, Van der Walls, London, covalent, metallic, or ionic bonds.

The invention also relates to magnetosomes for use according to the invention, wherein the application of the acoustic wave on the magnetosomes induces the dissociation of the compound from the magnetosomes.

In one embodiment of the invention, the compound is dissociated from the magnetosomes when it is located at a distance of more than 10⁵, 10³, 100, or 10 nm from the magnetosomes, or when it is not bound or not linked to the magnetosomes through Hydrogen, Van der Walls, London, covalent, metallic, or ionic bonds.

In another embodiment of the invention, the compound is dissociated from the magnetosomes when the magnetosomes can be magnetically separated from the compound. In this case, a magnet can preferentially be used to attract the magnetosomes, preferentially using a magnetic field whose intensity varies spatially, preferentially using a magnet with a lower strength than 10, 1, 10⁻¹, 10⁻³, or 10⁻⁹ T, where this strength is preferentially measured at the surface or near the magnet and decreases with increasing distance away from the magnet.

In another embodiment of the invention, the compound is dissociated from the magnetosomes when the percentage of compounds associated or linked to the magnetosomes or located at a distance of less than 10, 1 , 10⁻¹, 10⁻³, 10⁻⁶, or 10⁻⁹ cm from the magnetosomes, is lower than 99, 90, 75, 50, 30, 20, 10, 5, 2, or 1%, where this percentage can represent the ratio between the number or mass of compounds linked or associated to the magnetosomes before magnetic separation and the number or mass of compounds linked or associated to the magnetosomes after magnetic separation.

In another embodiment of the invention, the compound is not dissociated from the magnetosomes or non-dissociated when the magnetosomes can't be magnetically separated from the compound, preferentially using a magnet with a strength lower than 10, 1, 10⁻¹, 10⁻³, or 10⁻⁹ T.

In still another embodiment of the invention, the compound is not dissociated from the magnetosomes or non-dissociated when the percentage of compounds associated or linked to the magnetosome or located at distance of less than 10, 1 , 10⁻¹, 10⁻³, 10⁻⁶, or 10⁻⁹ cm from the magnetosomes, is larger than 99, 90, 75, 50, 30, 20, 10, 5, 2, or 1%, where this percentage can represent the ratio between the number or mass of compounds linked or associated to the magnetosomes before magnetic separation and the number or mass of compounds linked or associated to the magnetosomes after magnetic separation.

The invention also relates to magnetosomes for use according to the invention, wherein at least 0.01, 0.1, 1, 5, 10, 25, 50, 75, 80, or 90% of compounds are dissociated from the magnetosomes. This percentage can be equal to the ratio between the number or concentration of compounds dissociated from the magnetosomes divided by the total number or concentration of compounds not dissociated from the magnetosomes and/or attached to the magnetosomes.

In one embodiment of the invention, the intensity, energy, power, frequency of the acoustic wave decreases, preferentially by a factor of 1.1, 1.2, 1.5, 2, 3, 5, 10, 10², 10³, 10⁵, or 10¹⁰, with an increase in the penetration depth, preferentially by a factor of 1.1, 1.2, 1.5, 2, 3, 5, 10, 10², 10³, 10⁵, or 10¹⁰.

In one embodiment of the invention, the penetration depth of the acoustic wave is inversely proportional to the acoustic wave frequency. In some cases, the frequency of the acoustic wave can be decreased, preferentially by a factor of 1.1, 1.2, 1.5, 2, 3, 5, 10, 10², 10³, 10⁵, or 10¹⁰, preferentially in order to increase the penetration depth of the acoustic wave, preferentially by a factor of 1.1, 1.2, 1.5, 2, 3, 5, 10, 10², 10³, 10⁵, or 10¹⁰. Inversely, the frequency of the acoustic wave can be increased, preferentially by a factor of 1.1, 1.2, 1.5, 2, 3, 5, 10, 10², 10³, 10⁵, or 10¹⁰, preferentially in order to decrease the penetration depth of the acoustic wave, preferentially by a factor of 1.1, 1.2, 1.5, 2, 3, 5, 10, 10², 10³, 10⁵, or 10¹⁰.

In one embodiment of the invention, an acoustic wave volume or acoustic volume is defined as the volume, which is exposed to the acoustic wave or which receives the acoustic wave energy or which undergoes the effects of the acoustic wave.

The invention also relates to magnetosomes for use according to the invention, wherein the acoustic wave is unfocused. In this case, the acoustic wave can be applied over an acoustic wave volume preferentially comprised in the body part, which is larger than 0.001, 0.01, 0.1, 1, 10, 10², 10³, 10⁵, or 10¹⁰ cm³, or which is larger than the magnetosome region or body part by a factor of more than 1.1, 1.5, 2, 5, 10, 10³, or 10⁵. In this case, the acoustic wave can be applied on the body part, possibly including regions not comprising the magnetosomes.

In one embodiment of the invention, an unfocused acoustic wave covers more than 10⁻⁵, 10⁻³, 10⁻¹, 1, 10, 10³, or 10⁵% of the body part, using less than 10³, 10², 10, 5, 2, or 1 application(s) or 1 application spot(s). An application spot can be defined as the acoustic volume that can be covered or targeted during a single application of acoustic wave or during one sequence, preferentially during t₁ or t₃.

The invention also relates to magnetosomes for use according to the invention, wherein the acoustic wave is focused. In this case, the acoustic wave is preferentially applied over an acoustic wave volume preferentially comprised in the body part, which is smaller than 0.001, 0.1, 1, 10, 10², 10³, 10⁵, or 10¹⁰ cm³.

In one embodiment of the invention, a focused acoustic wave can cover less than 10⁻⁵, 10⁻³, 10⁻¹, 1, 10, 10³, or 10⁵% of the body part, using more than 10³, 10², 10, 5, 2, or 1 application(s) or 1 application spot(s).

The invention also relates to magnetosomes for use according to the invention, wherein the magnetosomes induce the destruction of a pathological site of the body part without the destruction of a healthy site surrounding the pathological site. In this case, magnetosomes can induce the destruction of more than 1, 10, 10³, 10⁶, 10⁹, or 10¹⁵ pathological cell(s) and/or less than 1, 10, 10³, 10⁶, 10⁹, or 10¹⁵ healthy cell(s).

In one embodiment of the invention, the destruction of the pathological site is associated with the death, destruction, denaturation, or inactivation of biological material(s). It can involve: i), immune, pharmaceutical, or metabolic mechanisms, ii), cavitation, iii), heat, or iv), generation of free radicals such as radical oxygen species.

In one embodiment of the invention, cavitation is associated with the production of bubbles. Cavitation can induce a mechanical stress on cells, preferentially cell membranes, and induce cell death. In the presence of the magnetosomes, the size and number of the bubbles can be changed. They can be increased or decreased by a factor larger or smaller than 1.5, 2, 10, 100, 10³, or 10⁹. In some cases, the size and number of the bubbles can tend to be close to that of the magnetosomes exposed to the acoustic wave and differ from the size and number of these magnetosomes by a factor of less than 10⁹, 10⁵, 10³, 10², or 10.

The invention also relates to magnetosomes for use according to the invention, wherein the application of acoustic waves on magnetosomes leads to a temperature increase, which is larger in the presence than in the absence of the magnetosomes, or which is larger during t₁ than during t₂ or t₃. The temperature increase occurring during the heating step can be characterized by at least one of the following properties: i), it has a magnitude of more than 10⁻⁵, 10⁻³, 10⁻¹, 1, 2, 5, or 10, 10³, 10⁵, or 10⁹ °C, preferentially above physiological temperature or above the temperature of the individual before or after the heating step, ii), it leads to an initial temperature increase, also designated as the initial slope of the temperature increase with time, ΔT/δt, which is larger than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec or 10⁵ °C per sec per gram of magnetosome or body part or 10⁵ °C per sec per cm³ of magnetosome or body part, iii), it is such that the maximum temperature reached during a heating step remains below 25, 30, 37, 39, 41, 45, 50, 100, 10³, 10⁵, 10⁹, or 10¹¹ °C, iv), it leads to temperature increase, which is more than 10⁻⁵, 10⁻³, 10⁻¹, 1, 2, 5, or 10, 10³, 10⁵, or 10⁹ °C above the temperature increase reached by application of the acoustic wave in the absence of the magnetosomes, or v), it leads to an initial temperature increase, ΔT/δt, which is more than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec or 10⁵ °C per sec per gram of magnetosome or body part or 10⁵ °C per sec per cm³ of magnetosome or body part above the initial temperature increase reached by application of the acoustic wave without the magnetosomes.

In one embodiment of the invention, the temperature increase occurring during the heating step is characterized by at least one of the following properties: i), it has a magnitude of less than 10⁻⁵, 10⁻³, 10⁻¹, 1, 2, 5, or 10, 10³, 10⁵, or 10⁹ °C, preferentially above physiological temperature or above the temperature of the individual before or after the heating step, ii), it leads to an initial temperature increase, also designated as the initial slope of the temperature increase with time, ΔT/δt, which is lower than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec, or °C/sec per gram of magnetosome or body part or °C/sec per cm³ of magnetosome or body part, iii), it is such that the maximum temperature reached during a heating step remains below 25, 30, 37, 39, 41, 45, 50, 100, 10³, 10⁵, 10⁹, or 10¹¹ °C, iv), it leads to temperature increase, which is less than 10⁻⁵, 10⁻³, 10⁻¹, 1, 2, 5, or 10, 10³, 10⁵, or 10⁹ °C above the temperature increase reached by application of the acoustic wave in the absence of the magnetosomes, or v), it leads to an initial temperature increase, ΔT/δt, which is less than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec, or °C/sec per gram of magnetosome or body part or °C/sec per cm³ of magnetosome or body part above the initial temperature increase reached by application of the acoustic wave without the magnetosomes.

In one embodiment of the invention, a suitable range for the magnitude of the temperature increase is between 0 °C and 36 °C as deduced from table 2. In some cases, the maximum value of this range (36 °C) can be increased, for example by more than 1, 5, 10, 20, 50, or 100 °C, preferentially when the temperature of the body part is increased, for example by using an equipment or a substance that increases the temperature of the body part or by increasing the intensity, power, or frequency of the acoustic wave or by increasing magnetosome concentration.

In another embodiment of the invention, the acoustic wave power, intensity, energy, frequency, or time of application, as well as the magnetosome concentration, are set at specific values chosen to reach a desired temperature during the heating step, which is preferentially larger than or equal to 0, 5, 10, 25, 30, 37, 39, 41, 45, 50, 100, 10³, or 10⁵ °C.

In still another embodiment of the invention, the acoustic wave power, intensity, energy, frequency, or time of application, as well as the magnetosome concentration, are set at specific values chosen to reach a desired temperature during the heating step, which is preferentially lower than 0, 5, 10, 25, 30, 37, 39, 41, 45, 50, 100, 10³, or 10⁵ °C.

In one embodiment of the invention, a suitable range of values for the desired temperature is between 41 °C and 100 °C, where 41 °C is preferentially the minimum value that can trigger a therapeutic effect such as an antitumor activity and 100 °C is the boiling temperature of water, which preferentially constitutes the majority of the body part. In some cases, the minimum value of this range (41 °C) can be decreased, for example by more than 1, 5, 10, 50, 100, 150, 200, or 250 °C, for example when the body part is cooled down. In some other cases, the maximum value of this range (100 °C) can be increased, for example by more than 1, 5, 10, 50, 100, 10³, or 10⁵ °C, for example when the body part does not mainly comprise water or when the body part is heated, or when the intensity, frequency of the acoustic wave is increased, or when the magnetosome concentration is increased.

In still another embodiment of the invention, the acoustic wave power, intensity, energy, frequency, or time of application, as well as the magnetosome concentration, are set at specific values chosen to reach a desired percentage of dissociated compounds during the dissociation step, which is preferentially larger than 0.1, 1, 5, 10, 25, 50, 75, or 90%.

In still another embodiment of the invention, the acoustic wave power, intensity, energy, frequency, or time of application, as well as the magnetosome concentration, are set at specific values chosen to reach a desired percentage of dissociated compounds during the dissociation step, which is preferentially lower than 0.1, 1, 5, 10, 25, 50, 75, or 90%. In some cases, to reach the desired temperature during the heating step or the desired level of dissociated compounds during the dissociation step, it is possible to increase, preferentially between time t₂ or t₃ and time t₁, the acoustic wave power, intensity, energy, frequency, or time of application, preferentially by a factor of more than 1.00001, 1.0001, 1.001, 1.01, 1.1, 1.2, 1.5, 2, 5, 10, 10³, 10⁵, 10¹⁰, 10²⁰, or 10⁴⁰.

In some other cases, to reach the desired temperature during the heating step or the desired percentage of dissociated compounds during the dissociation step, it is possible to increase, preferentially between time t₂ or t₃ and time t₁, the acoustic wave power, intensity, energy, frequency, or time of application, preferentially by a factor of less than 1.00001, 1.0001, 1.001, 1.01, 1.1, 1.2, 1.5, 2, 5, 10, 10³, 10⁵, 10¹⁰, 10²⁰, or 10⁴⁰.

In still some other cases, to reach the desired temperature during the heating step or the desired percentage of dissociated compounds during the dissociation step, it is also possible to increase the magnetosome concentration, preferentially between before and after magnetosome administration, preferentially between inside and outside of the body part. In some cases, the magnetosome concentration can be increased by a factor of at least 1.00001, 1.0001, 1.001, 1.01, 1.1, 1.2, 1.5, 2, 5, 10, 10³, 10⁵, 10¹⁰, 10²⁰, or 10⁴⁰. In some other cases, the magnetosome concentration can be increased by a factor of less than 1.00001, 1.0001, 1.001, 1.01, 1.1, 1.2, 1.5, 2, 5, 10, 10³, 10⁵, 10¹⁰, 10²⁰, or 10⁴⁰.

In one embodiment of the invention, the temperature increase or dissociation of the compound from the magnetosomes preferentially occurs in the magnetosome region, or occurs in more than 10⁹, 10⁵, 10³, 90, 70, 50, 20, 10, or 1% of the magnetosome region, and preferentially does not occur outside of this region.

The invention also relates to the non-application of an acoustic wave on magnetosomes or to the application of an acoustic wave on magnetosomes whose intensity, energy, power, or frequency is/are lower than that/those applied on magnetosomes during the heating or dissociation step. This leads to either enhanced cooling or to a lower percentage of compounds dissociated from the magnetosomes. In this case, the non-application of the acoustic wave on magnetosomes or the application of the acoustic wave on magnetosomes, which has a lower intensity, energy, power, or frequency than that applied during the heating or dissociation step, can in some cases lead to a temperature decrease, preferentially occurring during t₂ or t₃, which is larger in the presence than in the absence of magnetosomes or is larger during t₂ or t₃ than during t₁. In this case, the non-application of the acoustic wave on magnetosomes or the application of an acoustic wave on magnetosomes, which has a lower intensity, energy, power, or frequency than that applied during the heating or dissociation step, can in some other cases lead to a percentage of dissociation that is larger in the presence than in the absence of magnetosomes or is larger during t₁ than during t₂ or t₃.

In some cases, the temperature decrease occurring during the cooling or non-dissociation step is characterized by at least one of the following properties: i), it has a magnitude of more than 10⁻⁵, 10⁻³, 10⁻¹, 1, 2, 5, 10, 10³, 10⁵, or 10⁹ °C, preferentially above physiological temperature or above the temperature of the individual before or after the cooling step, ii), it leads to an initial temperature decrease, also designated as the initial slope of the temperature decrease with time, ΔT/δt, which is larger than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec, or °C/sec per gram of magnetosome or body part or °C/sec per cm³ of magnetosome or body part, iii), it is such that the minimum temperature reached during a cooling step remains above -273, -150, -100, -75, -50, -30, -10, 0, 5, 10, 25, 30, 37, 39, 41, 45, 50, 100, 10³, 10⁵, 10⁹, or 10¹¹ °C, iv), it leads to a temperature decrease, which is more than 10⁻⁵, 10⁻³, 10⁻¹, 1, 2, 5, or 10, 10³, 10⁵, or 10⁹ °C above the temperature decrease reached by the non-application of the acoustic wave in the absence of the magnetosomes, v), it leads to an initial temperature decrease, ΔT/δt, which is more than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec, or °C/sec per gram of magnetosome or body part or °C/sec per cm³ of magnetosome or body part, or which is above the initial temperature decrease reached by application of the acoustic wave without the magnetosomes.

In some other cases, the temperature decrease occurring during the cooling or non-dissociation step can be characterized by at least one of the following properties: i), it has a magnitude of less than 10⁻⁵, 10⁻³, 10⁻¹, 1, 2, 5, 10, 10³, 10⁵, or 10⁹ °C, preferentially above physiological temperature or above the temperature of the individual before or after the cooling step, ii), it leads to an initial temperature decrease, also designated as the initial slope of the temperature decrease with time, ΔT/δt, which is lower than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec, or °C/sec per gram of magnetosome or body part or °C/sec per cm³ of magnetosome or body part, iii), it is such that the minimum temperature reached during a cooling step remains below -273, -150, -100, -75, -50, -30, -10, 0, 5, 10, 25, 30, 37, 39, 41, 45, 50, 100, 10³, 10⁵, 10⁹, or 10¹¹ °C, iv), it leads to a temperature decrease, which is lower than 10⁻⁵, 10⁻³, 10⁻¹, 1, 2, 5, or 10, 10³, 10⁵, or 10⁹ °C above the temperature decrease reached by the non-application of the acoustic wave in the absence of the magnetosomes, v), it leads to an initial temperature decrease, ΔT/δt, which is lower than 10⁻⁴⁰, 10⁻²⁰, 10⁻¹⁰, 10⁻⁷, 10⁻⁵, 10⁻³, 10⁻¹, 1, 2, 5, 10, 10², 10³, or 10⁵ °C/sec, or °C/sec per gram of magnetosome or body part or °C/sec per cm³ of magnetosome or body part, above the initial temperature decrease reached by application of the acoustic wave without the magnetosomes. The invention also relates to the non-application of an acoustic wave on magnetosomes or the application of an acoustic wave of low intensity, energy, power, or frequency on magnetosomes that leads to enhanced dissociation of the compound from the magnetosomes, preferentially during the non-dissociation step. In this case, the non-application of an acoustic wave on magnetosomes or the application of an acoustic wave of low intensity, energy, power, frequency on magnetosomes can lead to a percentage of dissociation of the compound, preferentially occurring during t₁, which is larger in the presence than in the absence of magnetosomes or is larger during t₁ than during t₂ or t₃. In some cases, the dissociation of the compound from the magnetosomes occurring during the non-dissociation step is characterized by a percentage of dissociation that is lower than 95, 90, 80, 70, 50, 30, 25, 10, 5, 2, 1, 0.1, or 0.01 %.

In another embodiment of the invention, the acoustic wave power, intensity, energy, frequency, or time of application, as well as the magnetosomes concentration, are set at specific values chosen to reach a desired temperature during the cooling step. In some cases, the desired temperature is above or below -273, -150, -100, -75, -50, -30, -10, 0, 5, 10, 25, 30, 37, 39, 41, 45, 50, 100, 10³, 10⁵, 10⁹, or 10¹¹ °C. In some other cases, the desired temperature is below -273, -150, -100, -75, -50, -30, -10, 0, 5, 10, 25, 30, 37, 39, 41, 45, 50, 100, 10³, 10⁵, 10⁹, or 10¹¹ °C. In sill some other cases, to reach the desired temperature, it is possible to decrease, preferentially between time t₁ and time t₂ or t₃, the acoustic wave power, intensity, energy, frequency, or time of application, preferentially by a factor of at least 1.00001, 1.0001, 1.001, 1.01, 1.1, 1.2, 1.5, 2, 5, 10, 10³, 10⁵, 10¹⁰, 10²⁰, or 10⁴⁰. In still some other cases, to reach the desired temperature, it is also possible to decrease the magnetosome concentration, preferentially between before and after magnetosome administration, preferentially between inside and outside of the body part. In some cases, the magnetosome concentration is decreased by a factor of more than 1.00001, 1.0001, 1.001, 1.01, 1.1, 1.2, 1.5, 2, 5, 10, 10³, 10⁵, 10¹⁰, 10²⁰, or 10⁴⁰. In some other cases, the magnetosome concentration is decreased by a factor of less than 1.00001, 1.0001, 1.001, 1.01, 1.1, 1.2, 1.5, 2, 5, 10, 10³, 10⁵, 10¹⁰, 10²⁰, or 10⁴⁰.

In one embodiment of the invention, the temperature decrease preferentially occurs in the magnetosome region, or occurs in more than 10⁹, 10⁵, 10³, 90, 70, 50, 20, 10, or 1% of the magnetosome region, and preferentially does not occur outside of this region.

In another embodiment of the invention, the acoustic wave power, intensity, energy, frequency, or time of application, as well as the magnetosomes concentration, are set at specific values chosen to reach a desired percentage of dissociated compound during the non-dissociation step. In some cases, this percentage of dissociation is larger than 0.001, 0.01, 0.1, 1, 2, 5, 10, 25, 50, 75, 80, 90, or 99%. In still some other cases, this percentage of dissociation is lower than 0.001, 0.01, 0.1, 1, 2, 5, 10, 25, 50, 75, 80, 90, or 99%. In some cases, to reach the desired percentage of dissociation, it is possible to decrease, preferentially between time t₁ and time t₂ or t₃, the acoustic wave power, intensity, energy, frequency, or time of application, preferentially by a factor of at least 1.00001, 1.0001, 1.001, 1.01, 1.1, 1.2, 1.5, 2, 5, 10, 10³, 10⁵, 10¹⁰, 10²⁰, or 10⁴⁰. In some cases, to reach the desired percentage of dissociated compound, it is also possible to decrease the magnetosome concentration, preferentially between before and after magnetosome administration, preferentially between inside and outside of the body part. In some cases, the magnetosome concentration is decreased by a factor larger than 1.00001, 1.0001, 1.001, 1.01, 1.1, 1.2, 1.5, 2, 5, 10, 10³, 10⁵, 10¹⁰, 10²⁰, or 10⁴⁰. In some other cases, the magnetosome concentration is decreased by a factor lower than 1.00001, 1.0001, 1.001, 1.01, 1.1, 1.2, 1.5, 2, 5, 10, 10³, 10⁵, 10¹⁰, 10²⁰, or 10⁴⁰.

In one embodiment of the invention, the temperature decrease preferentially occurs in the magnetosome region, or occurs in more than 10⁹, 10⁵, 10³, 90, 70, 50, 20, 10, or 1% of the magnetosome region, and preferentially does not occur outside of this region.

In one embodiment of the invention, the different parameters that control the desired temperature reached during the heating or cooling step are the frequency, intensity, power, energy, or time of application of the acoustic wave, or the concentration, organization, distribution, size, or composition of the magnetosomes. In some cases, at least one of these parameters may be adjusted during the heating or cooling step to reach the desired temperature. In some other cases, at least one of these parameters may be fixed during the heating or cooling step to reach the desired temperature.

In one embodiment of the invention, the different parameters that control the desired percentage of dissociated compound reached during the dissociating or non-dissociation step are the frequency, intensity, power, energy, or time of application of the acoustic wave, or the concentration, organization, distribution, size, or composition of the magnetosomes. In some cases, at least one of these parameters may be adjusted during the dissociating or non-dissociation step to reach the desired percentage of dissociated compound. In some other cases, at least one of these parameters may be fixed during the dissociation or non-dissociation step to reach the desired percentage of dissociated compound.

In one embodiment of the invention, the magnetosome region is comprised in more than 10²⁰, 10¹⁰, 10⁵, 10³, 90, 70, 50, 20, 10, or 1% of the acoustic wave volume. In this case, the temperature increase or heating step or dissociation of the compound from the magnetosomes preferentially occurs in more than 10²⁰, 10¹⁰, 10⁵, 10³, 90, 70, 50, 20, 10, or 1% of the acoustic wave volume.

In still another embodiment of the invention, the magnetosome region is comprised in less than 10²⁰, 10¹⁰, 10⁵, 10³, 90, 70, 50, 20, 10, or 1% of the acoustic wave volume. In this case, the temperature increase or heating step or dissociation of the compound from the magnetosomes preferentially occurs in less than 10²⁰, 10¹⁰, 10⁵, 10³, 90, 70, 50, 20, 10, or 1% of the acoustic wave volume.

The invention also relates to at least one heating step taking place over a volume, which is less than 50, 10, or 1% of the acoustic wave volume.

The invention also relates to magnetosomes for use according to the invention, wherein the application of the acoustic wave on magnetosomes induces a temperature increase in the pathological site of more than 1 °C and/or a temperature increase in the healthy site, preferentially surrounding the pathological site, of less than 100 °C.

In one embodiment of the invention, the healthy site surrounds the pathological site when it is located at a distance of less than 1, 10⁻¹, 10⁻³, 10⁻⁶, or 10⁻⁹ m from the pathological site.

In one embodiment of the invention, the temperature increase is preferentially the difference in temperature between the temperatures measured: i), after and before the application of the acoustic wave, ii), during t₁ and t₂, or iii), during t₁ and t₃. In some cases, it can be larger than 0.001, 0.01, 0.1, 1, 10, 10², 10³, or 10⁵ °C. In some other cases, it can be lower than 0.001, 0.01, 0.1, 1, 10, 10², 10³, or 10⁵ °C. The temperature increase can also be more than 0.001, 0.01, 0.1, 1, 10, 10², 10³, or 10⁵ °C more important in the presence than in the absence of the magnetosomes.

The invention also relates to magnetosomes for use according to the invention, wherein the sequential application of the acoustic wave on magnetosomes induces a series of temperature increases of the body part followed by temperature decreases of the body part. Temperature increase preferentially occurs during t₁, while temperature decrease preferentially takes place during t₂ or t₃. The number of temperature increase or temperature decrease is preferentially larger than 1, 5, 10, 10², or 10³.

In one embodiment of the invention, several or more than 1, 5, 10, or 100 sequences, or a series of temperature increases followed by temperature decreases is a treatment session or session.

The invention also relates to magnetosomes for use, wherein the sequential application of the acoustic wave on magnetosomes induces a series of dissociations of the compound from the magnetosomes followed by non-dissociation of the compound from the magnetosomes. Dissociation of the compound preferentially occurs during t₁, while non-dissociation of the compound preferentially takes place during t₂ or t₃. The number of dissociations or non-dissociation is preferentially larger than 1, 5, 10, 10², or 10³.

In one embodiment of the invention, several or more than 1, 5, 10, or 100 sequences, or a series of dissociations followed by non-dissociations is a treatment session or session.

A treatment session can last more or less than 1, 5, 10, 20, 50, 10², 10³, or 10⁵ minute(s). A medical treatment can comprise more than 1, 2, 5, 10, or 10³ treatment session(s), where each treatment session can be separated by a lapse of time of more or less than 1, 5, 10, 10², 10³, 10⁵, 10¹⁰, or 10⁵⁰ minute(s). The lapse of time separating two sessions is preferentially longer than the times t₁, t₂, t₃, t₁+t₂, or t₁+t₃, preferentially by a factor of more or less than 1.001, 1.01, 1.1, 1.2, 1.5, 2, 5, 10, 20, 50, 100, 10³, 10⁵, or 10⁹.

In one embodiment of the invention, the duration of a session or the time separating two sessions is such, preferentially sufficiently long, that a therapeutic mechanism of treatment can occur.

A therapeutic mechanism of treatment such as that of tumor destruction can involve the immune system, an apoptotic mechanism, or a by stander effect. It preferentially enables to destroy pathological cells at a distance from the magnetosome or magnetosome region, which is more or less than 5, 2, 1, 10⁻¹, 10⁻³, 10⁻³, 10⁻⁶, or 10⁻⁹ m. In some cases, such mechanism can be reactivated during each session or sequence by applying or re-applying the acoustic wave and/or deactivated by stopping or re-stopping the application of the acoustic wave.

The invention also relates to magnetosomes for use according to the invention, wherein the application of acoustic waves on the body part leads to a temperature increase of the body part, which is at least 1% larger in the presence than in the absence of the magnetosomes. In some cases, the temperature increase can be more or less than 500, 200, 100, 75, 50, 10, or 1% larger in the presence than in the absence of the magnetosomes.

The invention also relates to magnetosomes for use according to the invention, wherein the temperature increase of the body part or dissociation of the compound from the magnetosome occurs within less than 50% of the body part. In some cases, the temperature increase can occur within more than 10²⁰, 10¹⁰, 10⁵, 10³, 500, 200, 90, 70, 50, 10, or 1% of the body part. In some other cases, the temperature increase can occur within less than 10²⁰, 10¹⁰, 10⁵, 10³, 500, 200, 90, 70, 50, 10, or 1% of the body part.

The invention also relates to magnetosomes for use according to the invention, wherein the temperature increase of the body part or dissociation of the compound from the magnetosome occurs within more than 10²⁰, 10¹⁰, 10⁵, 10³, 500, 200, 90, 70, 50, 10, or 1% of the volume occupied by the magnetosomes in the body part.

In one embodiment of the invention, the application of the acoustic wave on magnetosomes leads to an initial temperature increase (ΔT/δt)_{(M)} with at least one of the following properties: i), (ΔT/δ1)_{(M)} is larger than 10⁻⁷, 10⁻⁵, 10⁻³, 10⁻¹, 1, 10, 10³, or 10⁵ °C/sec, or °C/sec per gram of magnetosome or body part or °C/sec per cm³ of magnetosome or body part, ii), (ΔT/δt)_{(M)} is at least 1.1, 1.2, 1.5, 2, 5, 10, 10³, 10⁵, or 10⁹ more important than (ΔT/δt)_{(WM)}, measured in the absence of the magnetosomes, iii), (ΔT/δt)_{(M)} is lower by a factor of at least 1.1 1.2, 1.5, 2, 5, 10, 10³, 10⁵, or 10⁹ than (ΔT/δt)_{(WM)}. (ΔT/δt)_{(M)} and (ΔT/δt)_{(WM)} can be the temperature increase measured during the initial application of the acoustic wave energy, *i.e.* during less than 10³, 10², 10, 1, 10⁻², 10⁻³, 10⁻⁶, or 10⁻⁹ seconds, preferentially following the beginning of the application of the acoustic wave energy. (ΔT/δt)_{(M)} can represent a temperature variation by more than 1, 5, 10, 50, 75, 10², or 10³ %, during the initial time of application of the acoustic wave designated as tᵢ.

In one embodiment of the invention, the application of the acoustic wave on magnetosomes leads to a saturating temperature (ST) with at least one of the following properties: i), ST is larger than 10⁻⁷, 10⁻⁵, 10⁻³, 10⁻¹, 1, 10, 10³, or 10⁵ °C, ii), ST is more than a factor of 1.2, 1.5, 2, 5, 10, 10³, 10⁵, or 10⁹ more important in the presence than in the absence of the magnetosomes.

In another embodiment of the invention, the application of the acoustic wave on magnetosomes leads to ST with at least one of the following properties: i), ST is lower than 10⁻⁷, 10⁻⁵, 10⁻³, 10⁻¹, 1, 10, 10³, or 10⁵ °C, ii), ST is less than a factor of 1.2, 1.5, 2, 5, 10, 10³, 10⁵, or 10⁹ more important in the presence than in the absence of the magnetosomes.

In one embodiment of the invention, a suitable range of values of ST is between (37±4) °C and (73±4) °C, as deduced from table 2 considering that the initial temperature is the physiological temperature. In some cases, the minimum value of this range, (37±4) °C, can be decreased, for example by more than 1, 5, 10, 20, 50, or 100 °C, preferentially when the temperature of the body part is decreased, for example by using an equipment or a substance such as ice that decreases the temperature of the body part or by decreasing the intensity, power, or frequency of the acoustic wave or by decreasing magnetosome concentration. In some other cases, the maximum value of this range (73±4) °C can be increased, for example by more than 1, 5, 10, 20, 50, or 100 °C, preferentially when the temperature of the body part is increased, for example by using an equipment or a substance that increases the temperature of the body part or by increasing the intensity, power, or frequency of the acoustic wave or by increasing magnetosome concentration.

The saturating temperature is preferentially the maximum temperature that can be reached during the time of application of the acoustic wave or represent a temperature that does not vary by more than 1, 5, 10, 50, 75, 10², or 10³ %, during a time of application of the acoustic wave designated as t_{S}.

In some cases, the saturating temperature can be the desired temperature.

The time tₛ preferentially follows the time tᵢ.

In one embodiment of the invention, the application of the acoustic wave is stopped as soon as the saturating temperature is reached, preferentially to avoid overheating.

In one embodiment of the invention, the ratio tₛ/tᵢ is larger or smaller than 10⁻⁹, 10⁻⁶, 10⁻³, 10⁻¹, 1, 10, 10³, 10⁶, or 10⁹.

In another embodiment of the invention, the heating step lasts tᵢ+tₛ, where tₛ is preferentially smaller than tᵢ, where tᵢ/(tᵢ+tₛ) is preferentially larger than 10⁻⁹, 10⁻⁷, 10⁻⁵, 10⁻⁴, 10⁻³, 10⁻², 10⁻¹, 0.5, 0.75, or 0.9.

The invention also relates to a pharmaceutical composition, comprising at least one magnetosome as defined in the invention, optionally associated with a pharmaceutically acceptable vehicle.

In one embodiment, the vehicle is the compound.

The invention also relates to a pharmaceutical composition, wherein the pharmaceutically acceptable vehicle is the compound.

The invention also relates to a diagnostic composition, comprising at least one magnetosome as defined in this invention.

The invention also relates to a medical device comprising at least one magnetosome as defined in this invention.

The invention also relates to a medical device, comprising at least one magnetosome associated or bound with the compound.

The invention will be further described with the following non-limiting figures and examples.

### Description of the Figures:

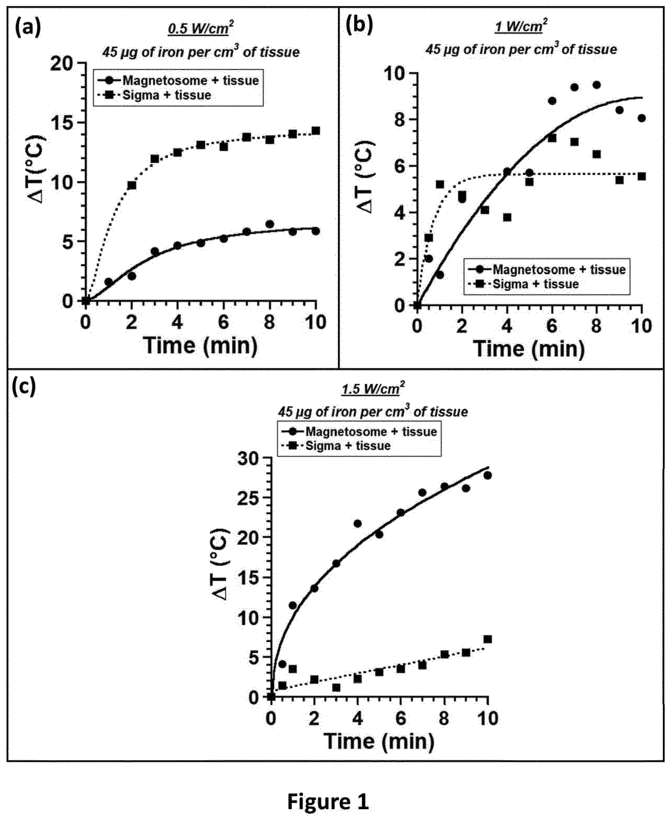

Figure 1: (a), For 210 µg in iron of nanoparticles (magnetosomes or Sigma) inserted in 4.6 cm³ of tissue exposed to ultrasounds of frequency 3 MHz and power 0.5 W/cm², ΔT, designing the temperature difference between the temperature measured for the tissue or body part with the nanoparticles and the temperature measured for the tissue or body part without the nanoparticles, as a function of duration of ultrasound application (time in minutes). (b), same as in (a) for an ultrasound power of 1 W/cm². (c), same as in (a) for an ultrasound power of 1.5 W/cm².

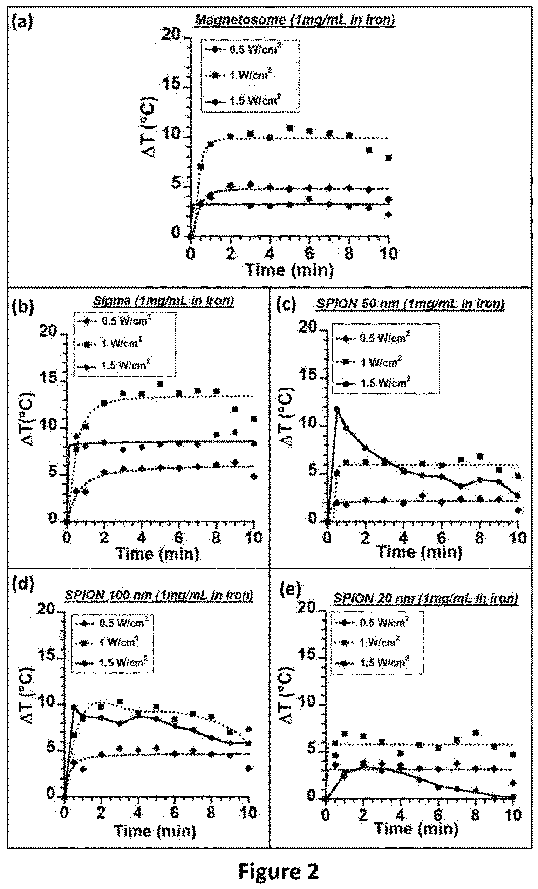

Figure 2: (a), For 100 µg in iron of magnetosomes dispersed in 100 µl of water exposed to ultrasounds of frequency 3 MHz and power 0.5 W/cm², 1 W/cm², or 1.5 W/cm², ΔT, designing the temperature difference between the temperature measured for magnetosomes dispersed in water and the temperature measured for water without magnetosomes, as a function of duration of ultrasound application (time in minutes). (b), For 100 µg in iron of Sigma nanoparticles dispersed in 100 µl of water exposed to ultrasound of frequency 3 MHz and power 0.5 W/cm², 1 W/cm², or 1.5 W/cm², ΔT, designing the temperature difference between the temperature measured for Sigma nanoparticles dispersed in 100 µl of water and the temperature measured for 100 µl of water without Sigma nanoparticles, as a function of duration of ultrasound application (time in minutes). (c), For 100 µg in iron of SPION50 nanoparticles dispersed in 100 µl of water exposed to ultrasound of frequency 3 MHz and power 0.5 W/cm², 1 W/cm², or 1.5 W/cm², ΔT, designing the temperature difference between the temperature measured for SPION50 nanoparticles dispersed in 100 µl of water and the temperature measured for 100 µl of water without SPION50 nanoparticles, as a function of duration of ultrasound application (time in minutes). (d), For 100 µg in iron of SPION100 nanoparticles dispersed in 100 µl of water exposed to ultrasound of frequency 3 MHz and power 0.5 W/cm², 1 W/cm², or 1.5 W/cm², ΔT, designing the temperature difference between the temperature measured for SPION100 nanoparticles dispersed in 100 µl of water and the temperature measured for 100 µl of water without SPION100 nanoparticles, as a function of duration of ultrasound application (time in minute). (e), For 100 µg in iron of SPION20 nanoparticles dispersed in 100 µl of water exposed to ultrasound of frequency 3 MHz and power 0.5 W/cm², 1 W/cm², or 1.5 W/cm², ΔT, designing the temperature difference between the temperature measured for SPION20 nanoparticles dispersed in 100 µl of water and the temperature measured for 100 µl of water without SPION20 nanoparticles, as a function of duration of ultrasound application (time in minute).

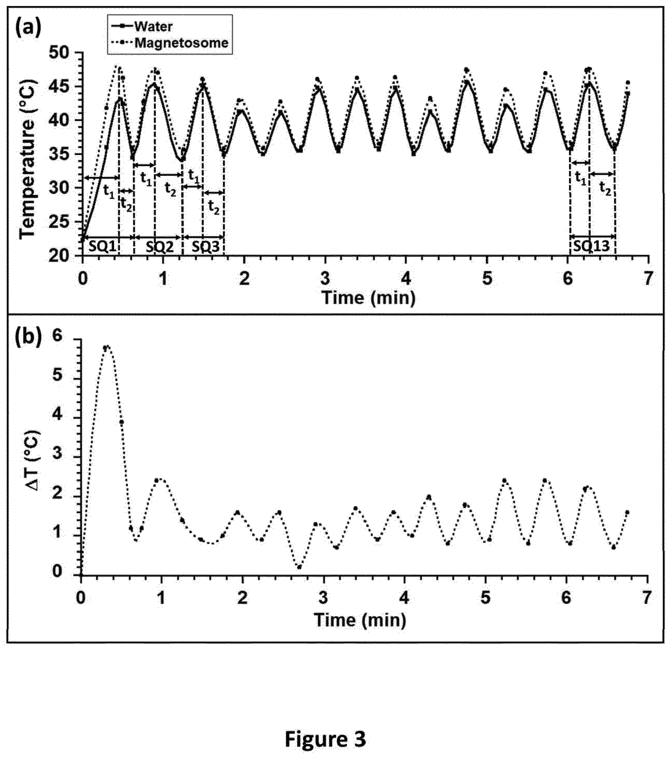

Figure 3: (a), For 800 µg in iron of magnetosomes dispersed in 100 µl of water (magnetosomes) or 100 µl of water without magnetosomes (water) exposed to ultrasound of frequency 3 MHz and power 1.5 W/cm² during heating steps of duration t₁ and no exposed to ultrasound during cooling steps of duration t₂, where the different values of heating and cooling times (t₁ and t₂) during sequences 1 to 13 (SQ1 to SQ13) are indicated in table 3. (b), Difference between the temperature of magnetosomes dispersed in water (Magnetosome in (a)) and the temperature of water without the magnetosomes (Water in (b)) as a function of duration of ultrasound application (time in minutes) during the different sequences.

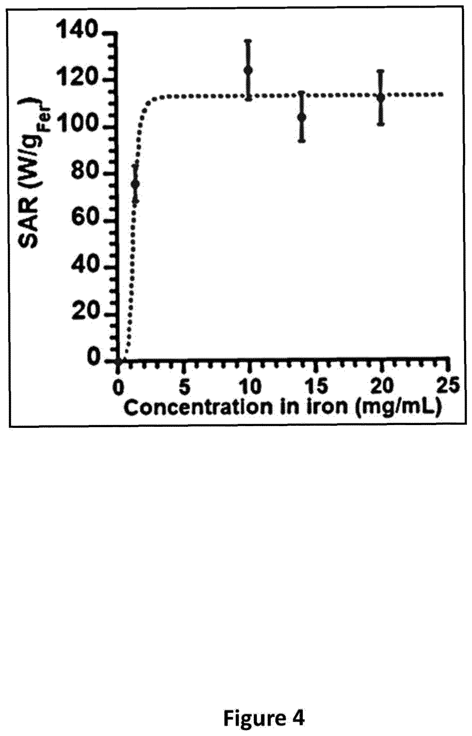

Figure 4: For 100 µl of a suspension of BNF-Starch nanoparticles (ref Micromod: 10-00102) mixed in water, exposed to an alternating magnetic field of average strength 30 mT and frequency 196 kHz, variation of the SAR, expressed in watt per gram of iron comprised in nanoparticles, as a function of the iron concentration comprised in BNF-Starch. BNF-Starch are ferrimagnetic iron oxide nanoparticles of average sizes 18 nm.

### EXAMPLE 1:

### MATERIALS AND METHODS:

Nanoparticles: We used as nanoparticles: i), magnetosomes extracted from magnetotactic bacteria composed of maghemite that were coated with carboxy-methyl-dextran using an adapted and improved protocol described in patent PCT/FR2016/000095 incorporated in reference (example 8), designated as Magnetosome(s) ii), nanoparticles composed of iron oxide of sizes 35 ± 13 nm purchased from Sigma designated as Sigma nanoparticles (Ref: 637106-25G, Lot #MKBK2270V), iii), superparamagnetic nanoparticles composed of iron oxide of 20 nm purchased from Micromod designated as SPION20 (nanomag®-D-spio 20, Ref: 79-02-201), iv), superparamagnetic nanoparticles composed of iron oxide of 50 nm purchased from Micromod designated as SPION50 (synomag-D 50, Ref: 104-000-501), v), superparamagnetic nanoparticles composed of iron oxide of 100 nm purchased from Micromod designated as SPION100 (nanomag®-D-spio 100, Ref: 79-00-102).

Preparation of samples containing nanoparticles inserted in tissue or dispersed in water: For heating experiments in tissues, 10 µl of suspensions containing water alone or 204 µg in iron of nanoparticles (Magnetosome(s) and Sigma or Sigma nanoparticle(s)) were inserted homogenously in 4.5 cm³ of liver tissue leading to a concentration of 45 µg in iron of nanoparticles per cm³ of liver tissue. For heating experiments in aqueous conditions, 100 µl of water alone or 100 µl of water mixed with 100 µg in iron of nanoparticles (Magnetosome, Sigma, SPION20, SPION50, SPION100) were dispersed in a 200 µl Eppendorf.

Heating apparatus generating ultrasound: Samples made of tissues with/without nanoparticles or water with/without nanoparticles were exposed to ultrasound of intensity 0.5, 1, or 1.5 W/cm³, and frequency 3 MHz, during 10 minutes. The intensity corresponds to that red on the apparatus and it is possible that there is a difference between the ultrasound intensity in the body part and the ultrasound intensity that the phyaction 190i indicates. To apply the ultrasound, we used a phyaction 190i ultrasound generator with a transducer of surface area 4 cm². The ultrasound power indicated in the example corresponds to that read on the 190i ultrasound generator and not to an ultrasound power measured with an external probe. We used an ultrasound gel (Winelec, Ref: 1741/WINELEC) located between the transducer and the samples to favor the transmission of the ultrasounds.

Measurement of temperature: We used an infrared camera (EasIRTM-2, Optophase) positioned 13 cm above the transducer to measure the spatial distribution in temperature as a function of time during the experiments. We measured the temperature distribution at the following time points: 0 sec., 30 sec., 1 min., 2 min., 3 min., 4 min., 5 min., 6 min., 7 min., 8 min., 9 min., and 10 min. We only considered the maximum temperature recorded at each time point.

### RESULTS AND DISCUSSION:

### a) Non sequential heating experiment in tissues:

Figure 1 shows ΔT, the difference in temperature between the tissue with the nanoparticles and the tissue without the nanoparticles, measured at each time point, and for an ultrasound power of 0.5 W/cm² (Figure 1(a)), 1 W/cm² (Figure 1(b)), 1.5 W/cm² (Figure 1(c)). At the three different tested powers, ΔT is positive indicating that the temperature increase is more important for the tissue containing nanoparticles than for tissue without the nanoparticles. At the lowest power of 0.5 W/ cm², Sigma nanoparticles produce more heat than Magnetosomes, while at 1.5 W/cm², the opposite behavior is observed with Magnetosomes producing more heat than Sigma nanoparticles.

For the magnetosomes mixed in tissue and exposed to different ultrasound powers of 0.5, 1, or 1.5 W/cm², we have also estimated the values of ΔT_{10mmreal(M)}, which is equal to ΔT_{10min(M)} - ΔT_{10min(W)}, where ΔT_{10min(M)} and ΔT_{10min(W)} are the temperature increases observed after 10 minutes of ultrasound application for the samples containing tissue with the magnetosomes and tissue without the magnetosomes, respectively. We observed that ΔT_{10minreal(M)} increases from 6 °C at 0.5 W/cm² to 28 °C at 1.5 W/cm² (table 1). We also estimated the percentage in temperature rise, Temperature rise _{(M)}, expressed using the formula Temperaturerise_{(M)}=(Δ_{10min(M)}/ΔT_{10min(W)}-1).100. It increases from 37% at 0.5 W/cm² to 100% at 1.5 W/cm² (table 1). We also estimated the value of the specific absorption rate of the magnetosomes inserted in tissue, SAR_{real(M)}, expressed in watt per gram of magnetosomes in iron (W/g_{Fe}), using the formula SAR_{real(M)}=Slope_{real(M)}.Cᵥ/Cₙₐₙₒ, where Slope_{real(M)}=Slope_{(M)}-Slope_{(W)}, with Slope_{(M)} and Slope_{(W)} representing the initial slopes of the temperature variations with time deduced from the plots of Figures 1(a) to 1(c), Cᵥ = 4.2 J.K⁻¹g⁻¹ is the specific heat of water and Cₙₐₙₒ is the nanoparticle concentration in gram of nanoparticles per mL of water. SAR_{real(M)} increases from 5-12 W/g_{Fe} at 0.5-1 W/cm² to 71 W/g_{Fe} at 1.5 W/cm² (table 1). We also estimated the percentage in slope rise, Slope rise _{(M)}, expressed using the formula Slope rise _{(M)} = [(Slope_{(M)}/Slope_{(W)})-1].100. It increases from 9-47% at 0.5-1 W/cm² to 124% at 1.5 W/cm².

For Sigma nanoparticles mixed in tissue and exposed to different ultrasound powers of 0.5, 1, or 1.5 W/cm², we have also estimated the values of ΔT_{10minreal(S)}, which is equal to ΔT_{10min(S)}-ΔT_{10min(W)}, where ΔT_{10min(S)} and ΔT_{10min(W)} are the temperature increases observed after 10 minutes of ultrasound application for the samples containing tissue with the Sigma nanoparticles and tissue without the Sigma nanoparticles, respectively. We observed that ΔT_{10minreal(S)} decreases from 14 °C at 0.5 W/cm² to 6-7 °C at 1-1.5 W/cm² (table 1). We also estimated the percentage in temperature rise, Temperature rise _{(S)}, expressed using the formula Temperature rise _{(S)} = (ΔT_{10min(S)}/ΔT_{10min(W)}-1).100. It decreases from 90% at 0.5 W/cm² to 17-26% at 1-1.5 W/cm² (table 1). We also estimated the value of the specific absorption rate of the Sigma nanoparticles inserted in tissue, SAR_{real(S)}, expressed in watt per gram of Sigma nanoparticles in iron (W/g_{Fe}), using the formula SAR_{real(S)} = Slope_{real(S)}.Cᵥ/Cₙₐₙₒ, where Slope_{real(S)} represent the initial slopes of the temperature variations with time deduced from the plots of Figures 1(a) to 1(c) for sigma nanoparticles, Cᵥ = 4.2 J.K⁻¹g⁻¹ is the specific heat of water and Cₙₐₙₒ is the nanoparticle concentration in gram of Sigma nanoparticles per mL of water. SAR_{real(S)} remains at 16-28 W/g_{Fe} between 0.5 and 1.5 W/cm² (table 1). We also estimated the percentage in slope rise, Slope rise _{(S)}, expressed using the formula Slope rise _{(S)}=(Slope_{(S)}/Slope_{(W)}-1).100. It decreases from 118% at 0.5 W/cm² to 30-36% at 1-1.5 W/cm².

### b) Non-sequential heating experiments in water:

Figures 2(a), 2(b), 2(c), 2(d), and 2(e), show ΔT, the difference between the temperature of the suspension containing the different nanoparticles dispersed in water and temperature of water without the nanoparticles, when the different suspensions are exposed to ultrasounds of 0.5, 1, or 1.5 W/cm² during 10 minutes. Figures 2(a), 2(b), 2(c), 2(d), and 2(e) show ΔT as a function of time for Magnetosomes, Sigma, SPION50, SPION100, and SPION20, respectively. For the different nanoparticles and the three different tested powers, ΔT is positive indicating that the temperature increase is more important for nanoparticles dispersed in water than for water alone.

For the magnetosomes mixed in water and exposed to different ultrasound powers of 0.5, 1, or 1.5 W/cm², we have estimated the values of ΔT_{10minreal(M)}, which is equal to ΔT_{10min(M)}-ΔT_{10min(W)}, where ΔT_{10min(M)} and ΔT_{10min(W)} are the temperature increases observed after 10 minutes of ultrasound application for the samples containing tissue with the magnetosomes and tissue without the magnetosomes, respectively. We observed that ΔT_{10minreal(M)} remains at 3 to 9 °C between 0.5 W/cm² and 1.5 W/cm² (table 2), smaller values of ΔT_{10minreal(M)} than those observed in tissue at 1.5 W/cm² (table 1). We also estimated the percentage in temperature rise, Temperature rise _{(M)}, expressed using the formula Temperature rise _{(M)} = (ΔT_{10min(M)}/ΔT_{10min(W)}-1).100. It decreases from 37-43% at 0.5-1 W/cm² down to 10% at 1.5 W/cm² (table 2) and is also smaller than Temperature rise _{(M)} measured in tissue at 1.5 W/cm² (table 1). We also estimated the value of the specific absorption rate of the magnetosomes dispersed in water, SAR_{real(M)}, expressed in watt per gram of magnetosomes in iron (W/g_{Fe}), using the formula SAR_{real(M)} = Slope_{real(M)}.Cᵥ/Cₙₐₙₒ, where Slope_{real(M)} represents the initial slopes of the temperature variations with time deduced from the plots of Figure 2(a), Cᵥ = 4.2 J.K⁻¹g⁻¹ is the specific heat of water and Cₙₐₙₒ is the Magnetosome concentration in gram of magnetosomes per mL of water. SAR_{real(M)} increases from 294 W/g_{Fe} at 0.5 W/cm² to 424 W/g_{Fe} at 1.5 W/cm² (table 2), higher values than those measured in tissue (table 1). We also estimated the percentage in slope rise, Slope rise _{(M)}, expressed using the formula Slope rise _{(M)} = (Slope_{(M)}/Slope_{(W)}-1).100. It remains at 16-24% at 0.5-1.5 W/cm² (table 2), smaller values than 124% deduced in tissue at 1.5 W/cm² (table 1).

For Sigma nanoparticles dispersed in water and exposed to different ultrasound powers of 0.5, 1, or 1.5 W/cm², we have also estimated the values of ΔT_{10minreal(S)}, which is equal to ΔT_{10min(S)}-ΔT_{10min(W)}, where ΔT_{10min(S)} and ΔT_{10min(W)} are the temperature increases observed after 10 minutes of ultrasound application for the samples containing Sigma nanoparticles dispersed in water and water without the Sigma nanoparticles, respectively. We observed that ΔT_{10minreal(S)} remains at 6-12 °C for ultrasound energies of 0.5-1.5 W/cm² (table 2). We also estimated the percentage in temperature rise, Temperature rise _{(S)}, expressed using the formula Temperature rise _{(S)}=(ΔT_{10min(S)}/ΔT_{10min(W)}-1).100. It remains at 31-60% for powers of 0.5-1.5 W/cm² (table 2). We also estimated the value of the specific absorption rate of the Sigma nanoparticles inserted in tissue, SAR_{real(S)}, expressed in watt per gram of Sigma nanoparticles in iron (W/g_{Fe}), using the formula SAR_{real(S)} = Slope_{real(S)}.Cᵥ/Cₙₐₙₒ, where Slope_{real(S)} represents the initial slopes of the temperature variations with time deduced from the plots of Figure 2(b) for Sigma nanoparticles, Cᵥ = 4.2 J.K⁻¹g⁻¹ is the specific heat capacity of water and Cₙₐₙₒ is the nanoparticle concentration in gram of Sigma nanoparticles in iron per mL of water. SAR_{real(S)} increases from 0 W/g_{Fe} at 0.5 W/cm² to 2686 W/g_{Fe} at 1.5 W/cm² (table 2). We also estimated the percentage in slope rise, Slope rise _{(S)}, expressed using the formula Slope rise _{(S)} = (Slope_{(S)}/Slope_{(W)}-1).100. It increases from 0-8% at 0.5-1 W/cm² to 99% at 1.5 W/cm².

For SPION50, SPION100, and SPION20 nanoparticles dispersed in water and exposed to different ultrasound powers of 0.5, 1, or 1.5 W/cm², we have also estimated the values of ΔT_{10minreal(S50)}, ΔT_{10minreal(S100)}, and ΔT_{10minreal(S20)}, which are equal to ΔT_{10min(S50)}-ΔT_{10min(W)}, ΔT_{10min(S100)}-ΔT_{10min(W)} and ΔT_{10min(S20)} - ΔT_{10min(W)}, respectively. ΔT_{10min(S50)}, ΔT_{10min(S100)}, ΔT_{10min(S20)} and ΔT_{10min(W)} are the temperature increases observed after 10 minutes of ultrasound application for the samples containing SPION50, SPION100, and SPION20 nanoparticles dispersed in water and water without nanoparticles, respectively. We observed that ΔT_{10minreal(S50)}, ΔT_{10minreal(S100)}, and ΔT_{10minreal(S20)}, remain at 0-7 °C for ultrasound energies of 0.5-1.5 W/cm² (table 2). We also estimated the percentage in temperature rise, Temperature rise _{(S)}, expressed using the formula Temperature rise _{(S50)} = (ΔT_{10min(S50)}/ΔT_{10min(W)}-1).100 for SPION50, Temperature rise _{(S100)} = (ΔT_{10min(S100)}/ΔT_{10min(W)}-1).100 for SPION100, Temperature rise _{(S20)} = (ΔT_{10min(S20)}/ΔT_{10min(W)}-1).100 for SPION20. It remains at 1-35% for powers of 0.5-1.5 W/cm² for the different SPION (table 2). We also estimated the value of the specific absorption rate of the SPION50, SPION100, and SPION20 nanoparticles inserted in tissue, SAR_{real(S50)}, SAR_{real(S100)}, SAR_{real(S20)}, expressed in watt per gram of SPION50, SPION100, and SPION20 nanoparticles in iron (W/g_{Fe}). For that, we used the formula SAR_{real(S50)} = Slope_{real(S50)}.Cᵥ/Cₙₐₙₒ, SAR_{real(S100)} = Slope_{real(S100)}.Cᵥ/Cₙₐₙₒ, SAR_{real(S20)} = Slope_{real(S20)}.Cᵥ/Cₙₐₙₒ for SPION50, SPION100, and SPION20 nanoparticles, respectively. Slope_{real(S50)}, Slope_{real(S100)}, Slope_{real(S20)} represent the initial slopes of the temperature variations with time deduced from the plots of Figure 2(c) for SPION50, of Figure 2(d) for SPION100, and of Figure 2(e) for SPION20, where Cᵥ = 4.2 J.K⁻¹g⁻¹ is the specific heat of water and Cₙₐₙₒ is the nanoparticle concentration in gram of SPION50, SPION20, or SPION100 nanoparticles in iron per mL of water. SAR_{real(S20)}, SAR_{real(S50)}, and SAR_{real(S100)} increase from 0-677 W/g_{Fe} at 0.5-1 W/cm² to 787-2795 W/g_{Fe} at 1.5 W/cm² (table 2). We also estimated the percentage in slope rise, Slope rise _{(S20)}, Slope rise _{(S50)}, Slope rise _{(S100)}, expressed using the formula Slope rise _{(S20)}=(Slope_{(S20)}/Slope_{(W)}-1).100 for SPION20, Slope rise _{(S50)}=(Slope_{(S50)}/Slope_{(W)}-1).100 for SPION50 and Slope rise _{(S100)}=(Slope(_{S100)}/Slope_{(W)}-1).100 for SPION100. It remains at 0-104% between 0.5 and 1.5 W/cm².

### c) Sequential heating experiments in water:

Eppendorf containing 500 µg of Magnetosomes dispersed in 100 µl of water were exposed sequentially to ultrasounds. Figure 3(a) shows 13 sequences (SQ1 to SQ13) consisting for each of them in the application of an ultrasound of power 1.5 W/cm² and frequency 3 MHz during time t₁ followed by the non-application of an ultrasound during times t₂. The time t₁ corresponds to the time necessary to reach a desired targeted temperature of 43.5 ± 1.5 °C during the heating step, while the time t₂ corresponds to the time necessary to cool down the sample from 43.5 ± 1.5 °C to 34.5 ± 0.5 °C during the cooling step. The values of t₁ and t₂ are given in table 3 for the different sequences. The average frequency of the sequences, 1/(t₁ₐᵥ+t₂ₐᵥ), where t₁ₐᵥ and t₂ₐᵥ represent the average values of t₁ and t₂ over the 13 sequences, was estimated as 33 mHz. Figure 3(b) shows the variation of ΔT, the difference in temperature between the temperature of the tube containing water with the Magnetosome and the temperature of the tube containing water without the Magnetosome, as a function of time. ΔT is positive indicating that the temperature increase is more important in the tube containing water with magnetosomes than in the tube containing water alone without magnetosomes during all 13 sequences. Furthermore, we are able to repeat the heating and cooling steps due to the presence of Magnetosome a large number of times (13) as seen in Figure 3(b), suggesting that the ultrasound are not damaging the Magnetosomes or are not strongly undermining the heating power of the magnetosomes. The heating steps are more important in magnitude during the two first sequences, which may be attributed to better magnetosome dispersion and lower magnetosome aggregation during the first two sequences than during the other remaining sequences. We also observe that the sequences can be repeated with heating and cooling times that do not vary by more than 53 % between the different sequences (table 3).

### CONCLUSION:

We can draw the following conclusion from this example:
   (i), The values of ΔT, the difference in temperature between the temperature of nanoparticles in tissues or water exposed to ultrasound and the temperature of tissue or water alone exposed to ultrasound, is always positive, indicating that the different nanoparticles (Magnetosome, Sigma, SPION20, SPION50, SPION100) enhance the heating efficacy of ultrasound in the tested conditions (ultrasound frequency = 3 MHz, ultrasound power = 0.5-1.5 W/cm², nanoparticle concentration varied between 60µg/mL and 8 mg/mL and nanoparticles either inserted in tissue or dispersed in water).
   (ii), For water suspensions, lower SAR values observed for magnetosomes than for other nanoparticles (table 2) may be explained by more aggregation for the magnetosomes than for other nanoparticles following application of the ultrasound (as was observed by eyes).
   (iii), in some cases, the SAR may have been underestimated due to the heat produced by the transducer generating the ultrasound that can heat the tissue and interfere with the heat produced by nanoparticles exposed to ultrasound. This may be the reason why some of the SAR values are reported to be 0 W/g for example.
   (iv), When SARᵣₑₐₗ is zero, the value of ΔT_{10min real} is non-zero (table 2), indicating that nanoparticles increase the quantity of heat generated by the acoustic wave but that SARᵣₑₐₗ is underestimated, possibly due to the interference with the heat generated by the transducer.
   (v), Nanoparticle SAR estimated by applying ultrasound in tissue comprising the various nanoparticles reach the largest value for the magnetosomes.
   (vi), Using magnetosomes, we can produce sequences consisting in heating steps (application of ultrasound on magnetosomes) followed by cooling steps (non-application of ultrasound on magnetosomes), with enhanced magnitudes of heating and cooling compared with heating and cooling steps obtained without the magnetosomes (Figures 3(a) and 3(b)).

### EXAMPLE 2:

Figure 4 shows that when 100 µl of a suspension of BNF-Starch nanoparticles mixed in water are exposed to an alternating magnetic field of average strength 30 mT and frequency 196 kHz during 30 minutes, the SAR increases from 4 Watt per gram of iron comprised in nanoparticles for a concentration of 500 µg of iron comprised in nanoparticles per mL up to 114 Watt per gram of iron comprised in nanoparticles for a concentration of 5 mg of iron comprised in nanoparticles per mL. The SAR increases by factor of 29 for an increase in nanoparticle concentration by a factor of 10. Above 5 mg/mL, the SAR saturates at 110 Watt per gram of iron comprised in nanoparticles.

**Table 1.** For 210 µg in iron of nanoparticles Magnetosome inserted in 4.6 cm³ of tissue exposed to ultrasounds of frequency 3 MHz and power 0.5 W/cm², 1 W/cm², 1.5 W/cm², (Slope_{(M)}) designs the slope at the origin of the temperature variation with time of magnetosomes mixed with tissue (Slope_{real(M)}) designing the difference between slope at the origin of the temperature variation with time of magnetomes mixed with tissue (Slope_{(M)}) and the slope at the origin of the temperature variation with time of the tissue without the nanoparticles (Slope_{(w)}). Slope_{realN(M)} is Slope_{real(M)} divided by the magnetosome concentration in gram of iron comprised in magnetosomes per mL. Slope rise (Slope rise (M)) designates the percentage in slope rise estimated using the formula for magnetosomes: Slope rise _{(M)} (%) = ((Slope_{(M)}/Slope_{(W)})-1)*100. The specific absorption rate of magnetosomes (SAR_{(M)}), estimated in watt per gram of magnetosomes is deduced from the values of Slope _{(M)}, using the formula: SAR_{(M)} = Cᵥ.Slope_{(M)}/Cₙₐₙₒ, where Cᵥ = 4.2 J.K⁻¹.g⁻¹ is the specific heat of water and Cₙₐₙₒ is the magnetosome concentration in gram of magnetosomes per cm³ of tissue. The variation in temperature between the initial temperature measured before the application of the ultrasound and the temperature measured after 10 minutes of application of the ultrasound is designated as ΔT_{10min(M)} for magnetosomes. The difference between ΔT_{10min(M)} and ΔT_{10min(W)} is designated as ΔT_{10min,real(M)}. The percentage in temperature rise is estimated for Magnetosomes using the formula: Temperature rise _{(M)} (%) = ((ΔT_{10min(M)}/ΔT_{10min(W)})-1)*100 for Magnetosomes. For 210 µg in iron of Sigma nanoparticles inserted in 4.6 cm³ of tissue exposed to ultrasounds of frequency 3 MHz and power 0.5 W/cm², 1 W/cm², 1.5 W/cm², (Slope_{(S)}) designs the slope at the origin of the temperature variation with time of Sigma nanoparticles mixed with tissue (Slope_{real(S)}) designs the difference between slope at the origin of the temperature variation with time of Sigma nanoparticles mixed with tissue (Slope_{(S)}) and the slope at the origin of the temperature variation with time of the tissue without the nanoparticles (Slope_{(w)}). Slope_{realN(S)} is Slope_{real(S)} divided by the Sigma nanoparticle concentration in gram of iron comprised in Sigma nanoparticles per mL. Slope rise (Slope rise (S)) designates the percentage in slope rise estimated using the formula for magnetosomes: Slope rise _{(S)} (%) = ((Slope_{(S)}/Slope_{(W)})-1)*100. The specific absorption rate of Sigma nanoparticles (SAR(_{S})), estimated in watt per gram of Sigma nanoparticles is deduced from the values of Slope _{(S)}, using the formula: SAR_{(S)} = Cᵥ.Slope_{(S)}/Cₙₐₙₒ, where Cᵥ = 4.2 J.K⁻¹.g⁻¹ is the specific heat of water and Cₙₐₙₒ is the Sigma nanoparticle concentration in gram of Sigma nanoparticles per cm³ of tissue. The variation in temperature between the initial temperature measured before the application of the ultrasound and the temperature measured after 10 minutes of application of the ultrasound is designated as ΔT_{10min(S)} for Sigma nanoparticles. The difference between ΔT_{10min(S)} and ΔT_{10min(W)} is designated as ΔT_{10min,real(S)}. The percentage in temperature rise is estimated for Sigma nanoparticles using the formula: Temperature rise (s) (%) = ((ΔT_{10min(S)}/ΔT_{10min(W)})-1)*100 for Sigma nanoparticles.

**Table 1**

| Heating on tissue | | | | | | | | | | | | | | |
|---|---|---|---|---|---|---|---|---|---|---|---|---|---|---|
| Water | | | | | Magnetosome | | | | | Sigma | | | | |
| | 0.5 W/cm² | 1 W/cm² | 1.5 W/cm² | | | 0.5 W/cm² | 1 W/cm² | 1.5 W/cm² | | | 0.5 W/cm² | 1 W/cm² | 1.5 W/cm² | |
| Slope _{(w)} (°C/sec) | 0.063 | 0.145 | 0.142 | | Slope _{(M)} (°C/sec) | 0.092 | 0.158 | 0.318 | | Slope _{(S)} (°C/sec) | 0.137 | 0.197 | 0.184 | |
| | | | | | Slope_{real (M)} (°C/sec) | 0.029 | 0.013 | 0.177 | | Slope_{real (S)} (°C/sec) | 0.074 | 0.052 | 0.042 | |
| | | | | | Slope_{real N(M)} (mL.°C/sec/g_{Fe}) | 3 | 1 | 17 | | Slope_{real N(S)} (°C/sec/g_{Fe}) | 7 | 5 | 4 | |
| | | | | | Slope rise _{(M)} (%) | 47 | 9 | 124 | | Slope rise _{(S)} (%) | 118 | 36 | 30 | |
| | | | | | SAR _{(M)} (W/g_{Fe}) | 37 | 64 | 128 | | SAR _{(S)} (W/g_{Fe}) | 52 | 75 | 70 | |
| | | | | | SAR_{real (M)} (W/g_{Fe}) | 12 | 5 | 71 | | SAR_{real (S)} (W/g_{Fe}) | 28 | 20 | 16 | |
| ΔT_{10 min (w)} (°C) | 16 | 32 | 28 | | ΔT_{10 min (M)} (°C) | 22 | 40 | 56 | | ΔT_{10 min (S)} (°C) | 30 | 38 | 35 | |
| | | | | | ΔT_{10 min real (M)} (°C) | 6 | 8 | 28 | | ΔT_{10 min real(S)} (°C) | 14 | 6 | 7 | |
| | | | | | Temperature rise _{(M)} (%) | 37 | 25 | 100 | | Temperature rise _{(S)} (%) | 90 | 17 | 26 | |

**Table 2.** For 100 µg in iron of nanoparticles (Magnetosome, Sigma, SPION20, SPION50, SPION100) dispersed in 100 µl of water exposed to ultrasounds of frequency 3 MHz and power 0.5 W/cm², 1 W/cm², 1.5 W/cm², slopes at the origin of the temperature variation with time of the different nanoparticles dispersed in water, designated as Slope _{(M)} for Magnetosome, Slope_{(S)} for Sigma nanoparticles, Slope_{(S20)} for SPION20, Slope_{(S50)} for SPION50, Slope_{(S100)} for SPION100. The difference between the slope at the origin of the temperature variation with time of nanoparticles dispersed in water and the slope at the origin of the temperature variation with time of water without the nanoparticles is designated by Slope_{real(M)} for Magnetosome, Slope_{real(S)} for Sigma nanoparticles, Slope_{real(S20)} for SPION20, slope_{real(S50)} for SPION50, Slope_{real(S100)} for SPION100. Values of slope rise in percentage estimated using the formula: Sloperise_{(M)}=((Slope_{(M)}/Slope_{(W)}-1)*100 for magnetosomes; Sloperise_{(S20)}=((Slope_{(S20)}/Slope_{(W)})-1)*100 for SPION20; Sloperise_{(S50)}=((Slope_{(S50)}/Slope_{(W)}-1)*100 for SPION50; Sloperise_{(S100)}=((Slope_{(S100)}/Slope_{(W)}-1)*100 for SPION100. The specific absorption rate (SAR), measured in watt per gram of nanoparticles, deduced from the values of Slope, using the formula: SAR_{(M)} = Cᵥ.Slope_{(M)}/Cₙₐₙₒ for Magnetosome, SAR_{(S)} = Cᵥ.Slope_{(S)}/Cₙₐₙₒ for Sigma nanoparticles, SAR_{(S20)} = Cᵥ.Slope_{(S20)}/Cₙₐₙₒ for SPION20, SAR_{(S50)} = cᵥ.Slope_{(S50)}/Cₙₐₙₒ for SPION50, and SAR_{(S100)} = Cᵥ.Slope_{(S100)}/Cₙₐₙₒ for SPION100, where Cᵥ = 4.2 J.K⁻¹g⁻¹ is the specific heat of water, Cₙₐₙₒ is the nanoparticle (Magnetosome, Sigma, SPION20, SPION50, or SPION100) concentration in gram of nanoparticles per mL of water. Slope_{(M)}, Slope_{(S)}, Slope_{(S20)}, Slope_{(S50)}, Slope_{(S100)} are the initial slopes of the temperature variation for Magnetosome, Sigma nanoparticles, SPION20, SPION50, and SPION100. The real specific absorption rate (SARᵣₑₐₗ), measured in watt per gram of nanoparticles is deduced from the values of Slopeᵣₑₐₗ, using the formula: SAR_{real(M)}=Cᵥ.Slope_{real(M)}/Cₙₐₙₒ for magnetosomes, SAR_{real(S)}=Cᵥ.Slope_{real(S)}/Cₙₐₙₒ for Sigma nanoparticles, SAR_{real(S20)}=Cᵥ.Slope_{real(S20)}/Cₙₐₙₒ for SPION20, SAR_{real(S50)}=Cᵥ.Slope_{real(S50)}/Cₙₐₙₒ for SPION50, SAR_{real(S100)}=Cᵥ.Slope_{real(S100)}/Cₙₐₙₒ for SPION100, where Cᵥ = 4.2 J.K⁻¹g⁻¹ is the specific heat of water and Cₙₐₙₒ is the nanoparticle (Magnetosome, Sigma, SPION20, SPION50, or SPION100) concentration in gram of nanoparticles per mL of water. Slope_{real(M)}, Slope_{real(S)}, Slope_{real(S20)}, Slope_{real(S50)} Slope_{real(S100)} designate the difference between the initial slope of the temperature variation with time of nanoparticles dispersed in water and the initial slope of the temperature variation with time of water without nanoparticles. For nanoparticles dispersed in water, the variation in temperature between the initial temperature measured before the application of the ultrasound and the temperature measured after 10 minutes of application of the ultrasound is designated as ΔT_{10min(W)} for water alone, ΔT_{10mm(M)} for Magnetosomes, ΔT_{10min(S)} for Sigma nanoparticles, ΔT_{10min(S20)} for SPION20, ΔT_{10min(S50)} for SPION50, ΔT_{10min(S100)} for SPION100. The differences between ΔT_{10min(M)} and ΔT_{10min(w}), ΔT_{10min(S)} and ΔT_{10min(w)}, ΔT_{10min(S20)} and ΔT_{10min(w}), ΔT_{10min(S50)} and ΔT_{10min(w}), ΔT_{10min(S100)} and ΔT_{10mm(w}), are designated as ΔT_{10minreal(M)}, ΔT_{10minreat(S)}, ΔT_{10minreal(S20)}, ΔT_{10minreal(S50)}, ΔT_{10minreal(S100)} for magnetosomes, Sigma nanoparticles, SPION20, SPION50, SPION100, respectively. The percentage in temperature rise, estimated for Sigma using the formula: Temperature rise(%)=((ΔT_{10min(S)}/ΔT_{10min(W)})-1)*100, for Magnetosomes using the formula: Temperature rise(%)=((ΔT_{10min(S)}/ΔT_{10min(W)})-1)*100, for SPION20 using the formula: Temperature rise(%)=((ΔT_{10min(S20)}/ΔT_{10min(W)})-1)*100, for SPION50 using the formula: Temperature rise(%)=((ΔT_{10min(S50)}/ΔT_{10min(W)})-1)*100, for SPION100 using the formula: Temperature rise(%) = ((ΔT_{10min(S100)}/ΔT1_{0min(W)})-1)*100.

**Table 2**

| Heating on aqueous solutions | | | | | | | | | | | | | |
|---|---|---|---|---|---|---|---|---|---|---|---|---|---|
| Water | | | | | Magnetosome | | | | | Sigma | | | |
| | 0.5 W/cm² | 1 W/cm² | 1.5 W/cm² | | | 0.5 W/cm² | 1 W/cm² | 1.5 W/cm² | | | 0.5 W/cm² | 1 W/cm² | 1.5 W/cm² |
| Slope_{(w)} | 0.291 | 0.467 | 0.645 | | Slope_{(M)} (°C/sec) | 0.361 | 0.571 | 0.747 | | Slope_{(S)} (°C/sec) | 0.275 | 0.503 | 1.287 |
| | | | | | Slope_{real (M)} (°C/sec) | 0.070 | 0.104 | 0.101 | | Slope_{real (S)} (°C/sec) | 0 | 0.036 | 0.642 |
| | | | | | Slope_{realN(M)} (mL.°C/sec/g_{Fe}) | 70 | 104 | 101 | | Slope_{real N (S)} (mL.°C/sec/g_{Fe}) | 0 | 36 | 642 |
| | | | | | Slope rise_{(M)} (%) | 24 | 22 | 16 | | Slope rise _{(S)} (%) | 0 | 8 | 99 |
| | | | | | SAR_{(M)} (W/g_{Fe}) | 1511.3 | 2389.5 | 3124.0 | | SAR_{(S)} (W/g_{Fe}) | 1150.1 | 2104.7 | 5385.6 |
| | | | | | SAR_{real (M)} (W/g_{Fe}) | 294.5 | 437.0 | 424.3 | | SAR_{real (S)} (W/g_{Fe}) | 0 | 152.1 | 2685.9 |
| ΔT_{10 min (w)}(°C) | 13 | 20 | 30 | | ΔT_{10 min (M)}(°C) | 18 | 29 | 33 | | ΔT_{10 mln (S)} (°C) | 19 | 32 | 39 |
| | | | | | ΔT_{10 min real (M)} (°C) | 5 | 9 | 3 | | ΔT_{10 min} real _{(S)} (°C) | 6 | 12 | 9 |
| | | | | | Temperature rise _{(M)} (%) | 37 | 43 | 10 | | Temperature rise _{(S)} (%) | 49 | **60** | 31 |
| | | | | | | | | | | | | | |

| SPION 50 nm | | | | | SPION 100 nm | | | | | SPION 20 nm | | | |
|---|---|---|---|---|---|---|---|---|---|---|---|---|---|
| | 0.5 W/cm² | 1 W/cm² | 1.5 W/cm² | | | 0.5 W/cm² | 1 W/cm² | 1.5 W/cm² | | | W/cm² | W/cm² | 1.5 W/cm² |
| Slope_{(S50)} (°C/sec) | 0.274 | 0.481 | 1.313 | | Slope_{(S100)} (°C/sec) | 0.300 | 0.446 | 0.992 | | Slope_{(S20)} (°C/sec) | 0.453 | 0.439 | 0833 |
| Slope_{real (S50)} (°C/sec) | 0 | 0.015 | 0.668 | | Slope_{real (S100)} (°C/sec) | 0.009 | 0 | 0.346 | | Slope_{real (S20)} (°C/sec) | 0.162 | 0 | 0.188 |
| Slope_{real N (S50)} (mL.°C/sec/g_{Fe}) | 0 | 15 | 668 | | Slope_{real N (S10)} (mL.°C/sec/g_{Fe}) | 9 | 0 | 346 | | Slope_{real N (S20)} (mL. °C/Sec/g_{Fe}) | 162 | 0 | 188 |
| Slope rise_{(S50)} (%) | 0 | 3 | 104 | | Slope rise_{(S100)} (%) | 3 | 0 | 54 | | Slope rise_{(S20)} (%) | 56 | 0 | 29 |
| SAR_{(S50)}(W/gFe) | 1145.5 | 2014.5 | 5495.0 | | SAR_{(S100)} (W/gFe) | 1253.2 | 1864.4 | 4149.1 | | SAR_{(S20)} (W/gFe) | 1894.2 | 1837.7 | 3486.8 |
| SAR_{real (S50)} (W/gFe) | 0 | 62,0 | 2795.3 | | SAR_{real (S100)} (W/gFe) | 36.4 | 0 | 1449,4 | | SAR_{real (S20)} (W/gFe) | 677.4 | 0 | 787.1 |
| ΔT_{10 min (S50)} (°C) | 15 | 26 | 34 | | ΔT_{10 min (S100)} (°C) | 17 | 27 | 36 | | ΔT_{10 min (S20)} (°C) | 16 | 26 | 30 |
| ΔT_{10 min real (S50)} (°C) | 2 | 5 | 4 | | ΔT_{10 min real (S100)} (°C) | 4 | 7 | 6 | | ΔT_{10 min real (S20)} (°C) | 3 | 6 | 0 |
| Temperature rise_{(S50)} (%) | 18 | 27 | 14 | | Temperature rise _{(S10)} (%) | 34 | 35 | 20 | | Temperature rise_{(S20)} (%) | 24 | 28 | 1 |

**Table 3.** For 500 µg of magnetosomes dispersed in 100 µl of water, exposed sequentially to ultrasounds, time t₁ necessary to reach the desired temperature of 43±1.5 °C during the heating step (application of an ultrasound of frequency 3 MHz and power 1.5 W/cm²), time t₂ necessary to reach 34.5±0.5 °C during the cooling step (non-application of ultrasound) during each of the 13 sequences, frequency of each sequence in mHz, 1/t₁+t₂.

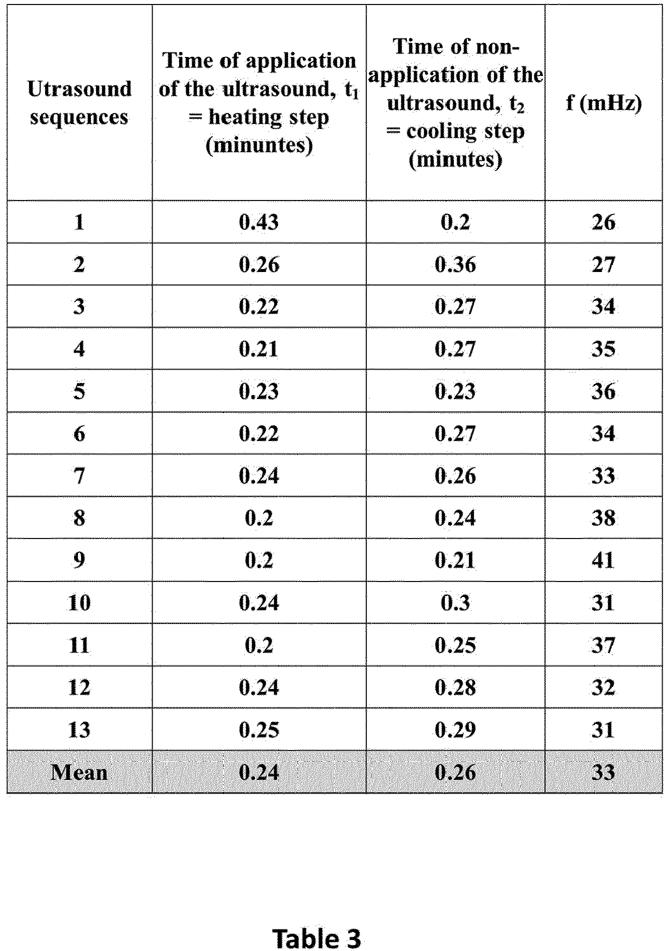

## Claims

1. Magnetosomes for use in an acoustic wave medical treatment of a body part of an individual, wherein the magnetosomes are administered to the body part of an individual and the body part is exposed to at least one acoustics wave.

2. Magnetosomes for use according to claim **1**, wherein the medical treatment is the treatment of a disorder or malfunction of the body part, an infectious disease, an auto-immune disease, a neuropathology, a cancer, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, or a personality disorder.

3. Magnetosomes for use according to claim **1** or **2**, wherein the magnetosomes possess a specific absorption rate, which increases with increasing power of the acoustic wave applied on the magnetosomes at a rate that that increases with decreasing nanoparticle concentrations.

4. Magnetosomes for use according to any one of claims **1** to **3**, wherein the acoustic wave intensity is lower than 1000 W/cm².

5. Magnetosomes for use according to any one of claims **1** to **4**, wherein the acoustic wave frequency is lower than 100 MHz.

6. Magnetosomes for use according to any one of claims **1** to **5**, wherein the acoustic wave is an ultrasound.

7. Magnetosomes for use according to any one of claims **1** to **6**, wherein the acoustic wave is sequentially applied on the magnetosomes.

8. Magnetosomes for use according to anyone of claims **1** to **7**, wherein the acoustic wave has a penetration depth in the body part, which is larger than 1 cm.

9. Magnetosomes for use according to any one of claims **1** to **8**, wherein the concentration of magnetosomes exposed to the acoustic wave is lower than 10 g per cm³ of body part.

10. Magnetosomes for use according to anyone of claims **1** to **9**, wherein the application of the acoustic wave on the magnetosomes induces a temperature increase of the body part, which is larger than 1 °C.

11. Magnetosomes for use according to anyone of claims **1** to **10**, wherein the application of the acoustic wave on the magnetosomes induces a temperature increase of the body part, which is lower than 30 °C.

12. Magnetosomes for use according to anyone of claims **1** to **11**, wherein a compound to be administered to the body part is attached to the magnetosomes.

13. Magnetosomes for use according to claim **12**, wherein the application of the acoustic wave on the magnetosomes induces the dissociation of the compound from the magnetosomes.

14. Magnetosomes for use according to any one of claims **1** to **11**, wherein the sequential application of the acoustic wave on magnetosomes induces a series of temperature increases of the body part followed by temperature decreases of the body part.

15. Magnetosomes for use according to claim **12**, wherein the sequential application of the acoustic wave on magnetosomes induces a series of dissociations of the compound from the magnetosomes followed by non-dissociation of the compound from the magnetosomes.
